# EUROPEAN PATENT APPLICATION

(11) **EP 3 825 408 A1**
(43) Date of publication of application: **26.05.2021**
(21) Application number: 19209940.6
(22) Date of filing: 19.11.2019
(51) Int. Cl.: C12N 15/82, C12N 15/113

(54) **METHODS OF MULTI-SPECIES INSECT PEST CONTROL**

(71) Applicant: FRAUNHOFER-GESELLSCHAFT zur Förderung der angewandten Forschung e.V., 80686 München (DE)
(72) Inventor: Vilcinskas, Andreas, 35463 Fernwald (DE); Skaljac, Marisa, 79713 Bad Saeckingen (DE); Kirfel, Phillipp, 65520 Bad Camberg (DE)
(74) Representative: Roth, Andy Stefan

(57) **Abstract**

The technology provided herein relates to compounds and methods of multi-species insect pest control by incorporating an inhibitor against a histone acetyltransferase (HAT) into the body of an agricultural target pest, and to pest control agents to be used in the method and to transgenic crop, greenhouse and ornamental plants.

## Description

### FIELD OF THE DISCLOSURE

The technology provided herein relates to compounds and methods of multi-species insect pest control by incorporating an inhibitor against a histone acetyltransferase (HAT) into the body of an agricultural target pest, and to pest control agents to be used in the method and to transgenic crop, greenhouse and ornamental plants.

### BACKGROUND

The environment in which humans live is replete with pest infestation. Pests including insects, arachnids, crustaceans, fungi, bacteria, viruses, nematodes, flatworms, roundworms, pinworms, hookworms, tapeworms, trypanosomes, schistosomes, botflies, fleas, ticks, mites, and lice and the like are pervasive in the human environment. For example, insects of the order Hemiptera including aphids are significant pests of crops and garden plants as well as ornamentals.

Aphids are the most common vectors of plant viruses, transmitting nearly 30% of all known virus species. They typically vector so-called non-persistent viruses, such as the highly diverse viruses of the family Potyviridae (Brault et al., 2010). Non-persistent virus infections occur a few minutes after mechanical inoculation via the aphid stylet, which is a mouthpart specialized for the penetration of plant tissue . These viruses are also of great economic importance. All of the devastating aphid species listed above act as vectors for plant viruses (Brault et al., 2010). In addition to the damage caused by direct feeding, virus-infected plants may show a range of symptoms including leaf yellowing and curling, growth abnormalities, and ultimately death.

Annual worldwide crop losses due to aphids and the viral diseases they carry have been valued at hundreds of millions of euros (The International Aphid Genomics, 2010). In Australia alone, the losses caused by aphid feeding and disease transmission amount to €210 million and €420 million per year, respectively. The global cost associated with the management of sharka disease, which is caused by aphid-transmitted Plum pox virus (Potyviridae), exceeded €10 billion between 1976 and 2006. This disease affects many stone fruits and is one of the most widely studied viral diseases.

Intensive plant trade and global warming provide opportunities for the introduction of new aphid species and their associated viruses to formerly unaffected regions. More than 100 aphid species originating from other continents are now well established in Europe. Invasive species of insects and pathogens pose a serious threat to crops. In the United States, crop and forest production losses from aphids can exceed €30 billion per year.

Chemical insecticides have long been used to control pest insects, including aphids. In the 1940s, new synthetic organic insecticides such as dichlorodiphenyltrichloroethane (DDT) and organophosphates led to great improvements in insecticidal efficacy and were subsequently used globally for pest control. Not surprisingly, this resulted in the rapid selection of resistant insect populations and species. As more insecticidal compounds are discovered, the same selection process occurs, encapsulating the concept of an evolutionary arms race between humans and pest insects (Sparks and Nauen, 2015). Despite the widespread use of insecticides, 18-20% of global crops are still lost to damage and disease caused by insect pests.

The number of insecticides on the market is declining due to tightening regulatory controls in the EU and elsewhere, favoring the evolution of resistance against the smaller number of permitted compounds. More than 1000 insecticidal compounds were approved by the European Commission in 2001, but this had fallen to 250 in 2009. In addition, the European Crop Protection Association reported 70 new active compounds in the development pipeline in 2000, whereas there were only 28 in 2012 (Barzman et al., 2015). The best way to delay the emergence of resistance is to switch between compounds with different modes of action, and to implement integrated pest management (IPM) strategies.

IPM is an effective solution to the challenges associated with pest control which aims to suppress insect populations below the threshold of economic significance. In other words, IPM is broad approach that involves multiple strategies in concert or in rotation (e.g., natural predators and parasites, pest-resistant varieties of plants, cultural practices, biological controls, various physical techniques, and chemical insecticides) for efficient pest control). IPM was proposed in the 1950s by scientists facing outbreaks of insect pests due to the eradication of natural enemies and the emergence of resistance to broad-spectrum insecticides (Barzman et al., 2015).

If the regulatory system allows, IPM can involve the use of genetically modified (GM) pest-resistant crops. This includes well-known GM corn, cotton and soybean varieties producing insecticidal Cry endotoxins from *Bacillus thuringiensis.* These so-called Bt crops are primarily developed to prevent infestation by moths (Lepidoptera) and beetles (Coleoptera) by discouraging feeding. Bt crops have been broadly accepted due to their economic and environmental benefits, but they too have led to the emergence of resistant pest populations, particularly during the deployment of first-generation Bt varieties expressing only one type of Bt toxin.

Although IPM can significantly reduce selection pressure against insect pests, the use of chemical insecticides still remains the most widely-deployed control strategy in practice (Sparks and Nauen, 2015, Guedes et al., 2016). Aphids can be controlled by several groups of chemicals, including carbamates, organophosphates, pyrethroids, neonicotinoids and pymetrozine (http://www.irac-online.org/documents/sucking-pests-moa-poster/). Due to the long-term and frequent use of these insecticides, aphids have evolved multiple forms of resistance against most of them, making some species particularly difficult to control - especially the green peach aphid *Myzus persicae* and the cotton aphid *Aphis gossypii* (Sparks and Nauen, 2015). These extremely polyphagous species are among the top 12 pest insects ranked by resistance, with *M. persicae* resistant to 75 insecticidal compounds and *A. gossypii* resistant to 48 (Sparks and Nauen, 2015). Remarkably, *M. persicae* has evolved at least seven genetically independent resistance mechanisms (reviewed in Bass, Puinean et al., 2014):
1) Overproduction of carboxylesterases associated with resistance to organophosphate and carbamate insecticides;
2) Mutation of the acetylcholinesterase enzyme associated with insensitivity to dimethyl carbamate insecticides;
3) Mutation of the voltage-gated sodium channel associated with resistance to pyrethroid insecticides;
4) Duplication and mutation of the GABA receptor subunit gene associated with resistance to cyclodiene insecticides;
5) Overexpression of CYP6CY3, a cytochrome P450 associated with resistance to nicotine and neonicotinoid insecticides;
6) Reduced penetration of insecticides through the cuticle associated with resistance to neonicotinoid insecticides;
7) Mutation of the nicotinic acetylcholine receptor (nAChR) associated with resistance to neonicotinoid insecticides.

As well increasing selection pressure and favoring the survival of resistant mutants, the overuse of insecticides is controversial because it has many undesirable effects on the environment (e.g., contamination of soil and groundwater), human health, and non-target beneficial organisms such as bees and other pollinators (Barzman et al., 2015). More than 80% of soil samples collected across 11 EU Member States in 2015 contained one or more pesticide residues, including insecticides, fungicides and herbicides. Pesticides not only accumulate in soil but also in the human body, causing chronic effects particularly in farmers that are routinely exposed to high levels of these harmful substances (Damalas and Koutroubas, 2016). Some of the insecticides commonly used for pest control can suppress the human immune system, cause hormone disruption, affect intelligence, induce reproductive abnormalities and trigger cancer (https://www.who.int/ceh/capacity/Pesticides.pdf). The correlation between the global decline of pollinators (bees, beetles, hoverflies, butterflies and mosquitoes) and the intensive use of insecticides has been reviewed.

The harmful effects of insecticides, the spread of insecticide resistance and the continued restriction of available compounds on the market, therefore, call for alternative and more sustainable pest control measures.

To reduce the amount of insecticides, new biotechnical approaches are developed in accordance with IPM (Integrated pest management to control pests in agriculture. One of these approaches is the use of RNA interference (RNAi). With regard to aphids, RNAi-mediated gene silencing was achieved in a number of publications by injection of dsRNA or siRNAs into the hemolymph (Mutti NS et al., 2006; or artificial feeding of dsRNA (.

The very first proof of concept for transgenic plants that deliver highly specific dsRNA to their aphid hosts was conducted by Pitino M et al. (2011). The authors selected rack1 (gut located) and c002 (salivary gland located) as two different gene targets for the green peach aphid *M. persicae.* Two different plants, *Nicotiana benthamiana* and *Arabidopsis thaliana,* were transformed for each target and a silencing effect in aphids of up to 60% was observed on respective GM plant species. As a consequence of gene silencing the authors described for both genes a reduced fecundity. Surprisingly, silencing of C002 did not influence survival as previously observed for *in vitro* experiments with the pea aphid *Acyrthosiphon pisum* (Mutti NS et al., 2006). The authors suggest that this discrepancy is related to different species.

Because most plants are infested by more than one pest species, an approach is needed whose efficiency does not differ between different species.

Therefore, the availability of improved pest control methods for numerousness pest species would be highly advantageous.

### SUMMARY OF THE DISCLOSURE

The present disclosure pertains to multi-species pest control methods comprising incorporating an inhibitor against a histone acetyltransferase (HAT), in particular against the p300/CREB-binding protein into the body of an agricultural target pest expressing the HAT, a protein, which is present in a wide range of Hemiptera species.

The present disclosure pertains in particular to methods of RNAi mediated silencing of the CREB-binding protein p300 and/or its paralog CBP (together named as p300/CREB-binding protein or p300/CBP) for control of plant-sucking insects of the order Hemiptera, in particular of the groups Sternorryhncha in agriculture.

Therefore, the technology disclosed herein includes novel aphid control strategies achieved by incorporating an inhibitor of p300/CREB-binding protein into the body of a target agricultural pest, in particular into the body of aphids like *Acyrthosiphon pisum.*

In a first aspect, embodiments of the disclosure provide novel pest control methods comprising incorporating an inhibitor against a HAT into the body of an agricultural target pest expressing a HAT, wherein the target pest is an insect belonging to the order Hemiptera and the uptake by the target pest of said inhibitor reduces at least the reproduction and/or survival rate and/or inhibits the development of said target pest.

In a second aspect, embodiments of this disclosure relate to isolated polynucleotides, which
(i) is defined as operably linked to a heterologous promoter; or
(ii) is defined as comprised of a plant transformation vector;
wherein the polynucleotide is selected from the group consisting of:
a) a polynucleotide comprising a nucleic acid sequence of SEQ ID NO. 2;
b) a polynucleotide that hybridizes to a nucleic acid sequence of SEQ ID NO. 2 under stringent conditions;
c) a polynucleotide of at least 70, at least 80, at least 85, at least 90 percent sequence identity, to the nucleic acid sequence of SEQ ID NO. 2;
d) a polynucleotide that is derived from SEQ ID NO. 1;
e) a fragment of at least 16 contiguous nucleotides of a nucleic acid sequence of SEQ ID NO. 1; and
f) a complement of the sequence of (a), (b), (c), (d) or (e),
wherein incorporating of a double stranded ribonucleotide sequence comprising at least one strand that is complementary to said polynucleotide or said fragment into the body of a Hemiptera pest reduces at least the reproduction and/or survival of said pest.

In a third aspect, embodiments of this disclosure relate to double stranded ribonucleotide sequences produced from the expression of a polynucleotide according to the present disclosure, wherein the uptake of said ribonucleotide sequences by a Hemiptera crop plant pest reduces at least the reproduction and/or the survival rate of said pest.

In a fourth aspect, embodiments of this disclosure provide vectors or expression systems comprising a nucleic acid molecule according to the second aspect and to cells transformed, transduced or transfected with said vector.

In a fifth aspect, some embodiments of this disclosure relate to plants transformed with a polynucleotide according to the present disclosure, or seed thereof comprising said polynucleotide.

Further, some embodiments pertain to commodity products produced from a plant according to the fifth aspect, wherein said commodity product comprises a detectable amount of a polynucleotide according to the second aspect or a ribonucleotide expressed therefrom.

In a sixth aspect, some embodiments provide methods for controlling Hemiptera pest infestation comprising providing in the diet of a Hemiptera pest an agent comprising a first polynucleotide sequence that functions upon uptake by the pest to inhibit a biological function within said pest, wherein said polynucleotide sequence exhibits from about 95 to about 100 percent nucleotide sequence identity along at least from about 16 to about 25 contiguous nucleotides to a HAT coding sequence, in particular to a p300/CREB-binding protein coding sequence derived from said pest and is hybridized to a second polynucleotide sequence that is complementary to said first polynucleotide sequence, and wherein said coding sequence derived from said pest comprise a sequence selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11 and SEQ ID NO:12 or a complement thereof.

Further, in a seventh aspect, embodiments of the present disclosure pertains to methods for controlling a Hemiptera pest infestation comprising providing in the diet of a Hemiptera pest, in particular of aphids, a polypeptide according to the present disclosure, wherein the polynucleotide is expressed to produce a double stranded ribonucleic acid, wherein said double stranded ribonucleotide acid and/or a RNAi inducing compound derived from said double stranded ribonucleotide acid functions upon ingestion by the pest to inhibit the expression of a HAT encoding target sequence within said pest and results at least in reduced survival rate and/or reproduction on said diet relative to a diet lacking the plant cell.

Further, in an eight aspect, embodiments of the present disclosure pertain to a method for improving the yield of a crop produced from a crop plant subjected to insect pest infestation, said method comprising the steps of,
a) introducing a polynucleotide according to the present disclosure into said crop plant,
b) cultivating the crop plant to allow the expression of said polynucleotide, wherein expression of the polynucleotide reduces at least the reproduction and/or survival rate of said pest and loss of yield due to pest infestation.

In a further aspect, the present disclosure relates to a transgenic plant comprising a gene coding an inhibitor against a HAT, in particular to a p300/CREB-binding protein of a Hemiptera target pest, in particular to an aphid target pest.

In a further aspect, the present disclosure relates to an isolated double stranded ribonucleotide (dsRNA) sequence having a survival and/or reproduction and/or development inhibitory effect on a Hemiptera pest and consisting of an nucleic acid sequence between 250 to 600 nucleic acids in length, in particular between 300 and 550 nucleic acids in length, in particular between 300 and 520 nucleic acids in length, wherein the dsRNA sequence comprises an nucleic acid sequence derived from SEQ ID NO. 1 or homologs thereof, wherein said homologs have a sequence identity of at least 80 %, in particular of at least 85%, in particular of at least 90%, even more particular of at least 95%, or 98% to SEQ ID NO: 1, and wherein the derived dsRNA is coded by a continuous part of SEQ ID NO. 1 or said homologs thereof, and having a nucleic acid sequence between 300 to 520 nucleic acids in length and wherein the Hemiptera pest belonging to the suborder Sternorrhyncha, in particular belonging to the family of aphids like *Acyrthosiphon pisum.*

In a further aspect, the present disclosure relates to a pest control method comprising incorporating an inhibitor against a histone acetyltransferase (HAT) into the body of an agricultural target pest expressing HAT, wherein the inhibitor is a compound selected from the group consisting of the following a) and (b):
(a) a RNAi inducing compound targeted at the nucleic acid coding HAT or parts thereof;
(b) a nucleic acid construct intracellularly producing a RNAi inducing compound targeted at the nucleic acid coding HAT or parts thereof; and wherein the target pest is an insect belonging to a pest belonging to the family of aphids, in particular, *Acyrthosiphon pisum*; and the ingestion by the target pest of said inhibitor reduces at least the reproduction and/or survival rate of said pest; wherein the RNAi inducing compound is a compound selected from the group consisting of short interfering nucleic acids, siNA, short interfering RNA (siRNA), microRNA (miRNA), short hairpin RNAs (shRNA) and precursors thereof, in particular double-stranded RNA (dsRNA), which are processed in the cell to the actual RNAi inducing compound, wherein in particular the HAT is p300 and/or its paralog CREB-binding protein (p300/CBP).

In a further aspect, the present disclosure relates to an isolated polynucleotide, which
i. is defined as operably linked to a heterologous promoter; or
ii. is defined as comprised on a plant transformation vector; wherein the polynucleotide comprises the nucleic acid sequence of SEQ ID NO:2 or homologs thereof, wherein said homologs have a sequence identity of at least 80 %, in particular of at least 85%, in particular of at least 90%, and more particular of at least 95%, 98% to SEQ ID NO: 2 and reduces at least the reproduction and/or survival of said target pest;
wherein ingestion by a Hemiptera crop, greenhouse and/or ornamental plant pest of a double stranded ribonucleotide sequence comprising at least one strand that is complementary to said polynucleotide reduces at least the reproduction and/or survival of said pest.

In a further aspect, the present disclosure relates to a method of inhibiting expression of the p300/CBP gene in *Acyrthosiphon pisum* wherein a partially or fully stabilized double-stranded RNA (dsRNA) nucleotide molecule derived from SEQ ID NO: 2 is introduced into a nutritional composition on that A. *pisum* relies on as a food source and making the nutritional composition available to the *A. pisum* for feeding.

Further aspects pertains to a double-stranded ribonucleic acid (dsRNA) for inhibiting the expression of a gene encoding a HAT in a cell, wherein said dsRNA comprises a first strand comprising a sequence with at least 95% sequence identity to a portion of at least 18 consecutive nucleotides of SEQ ID NO: 1 and a second strand complementary to the first strand.

Further aspects pertains to a method for controlling an aphid, the method comprising: constructing a double stranded ribonucleic acid (dsRNA) wherein one strand of the dsRNA comprises a sequence with at least 95% sequence identity to a portion of at least 18 consecutive nucleotides of SEQ ID NO: 1 and a second strand complementary to the first strand, that is complementary to an aphid gene that encodes a HAT, dissolving the dsRNA to form a solution, and contacting an effective amount of said solution to an aphid species, wherein said solution is ingested by said aphid species and RNA interference is induced, resulting in reduced survival and/or reproduction of said aphid.

Before the disclosure is described in detail, it is to be understood that this disclosure is not limited to the particular component parts of the process steps of the methods described. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. It is moreover to be understood that, in case parameter ranges are given which are delimited by numeric values, the ranges are deemed to include these limitation values.

### BRIEF DESCRIPTION OF THE DRAWINGS

Figure 1 shows a cDNA as a part of the *A. pisum* p300/CBP codingsequence (SEQ ID NO. 3), indicating the annealing position of RNAi primers (bold) and the section used to generate the dsRNA construct (underlined, SEQ ID NO. 2).
Figure 2 shows a *A. pisum* p300/CBP cDNA sequence with the start and stop codons, open reading frame (ORF), and the RNAi target site. The positions of the sequence fragments are also shown.
Figure 3 is an alignment of the RNAi target sequence of *A. pisum* p300/CBP with orthologs from *Myzus persicae* (97% identical), *Aphis gossypii* (93% identical), *Diuraphis noxia* (96% identical), *Melanaphis sacchari* (93% identical), *Rhopalosiphum maidis* (92% identical) and *Sipha flava* (88% identical). There is a high probability that the p300/CBP dsRNA construct will, therefore, target the corresponding mRNA in other aphid species (see Table 5). The sequences were aligned using Geneious v10.2.4 and the positions of single nucleotide polymorphisms are highlighted.
Figure 4 comprises diagrams showing life-history parameters following the RNAi-mediated silencing of p300/CBP. Survival (A), start of reproduction (B), total number of offspring (C), average number of premature offspring (D).
Figure 5 shows the influence of RNAi-mediated p300/CBP silencing on the structure and content of A. *pisum* ovaries 10 days post-injection. Ovaries were dissected from aphids injected with GFP control dsRNA (A) or p300/CBP dsRNA (B, C). The silencing of p300/CBP resulted in the accumulation of a greater number of late-stage embryos (B, C), and the ovary tissue was severely weakened and prone to rupture (C).
Figure 6 is a diagram showing the distribution of early-stage (stage 17 and lower) and late-stage (stage 18 and later) embryos among the total number of embryos counted in dissected ovaries. Ovaries of p300/CBP dsRNA treated aphids contain significantly less early-stage embryos (p < 0.0001) and significantly more late-stage embryos (p < 0.0001) than the GFP control group.
Figure 7 is a diagram showing the survival of the F1 generation of *A. pisum* following the injection of their mothers with p300/CBP dsRNA compared to a control group whose mothers were injected with GFP dsRNA. Aphid survival was monitored for 2 weeks. The groups were compared using the Kaplan-Meier log-rank test. The survival of aphids in the p300/CBP dsRNA treatment group was significantly reduced compared to the control group (p < 0.05).

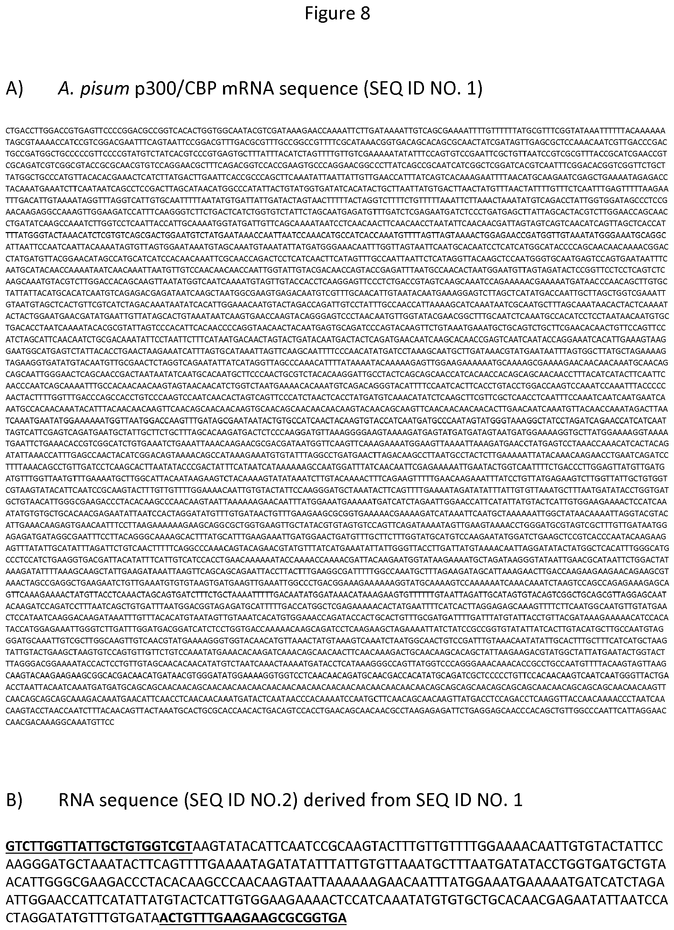

Figure 8 shows A) *A. pisum* p300/CBP mRNA coding cDNA sequence (SEQ ID NO.1) and B) a cDNA sequence (SEQ ID NO.2) derived from SEQ ID NO. 1, exemplarily used to generate a dsRNA construct for *A. pisum* pest control *in vitro* and *in planta.* The annealing position of RNAi primers are highlighted (bold letters).

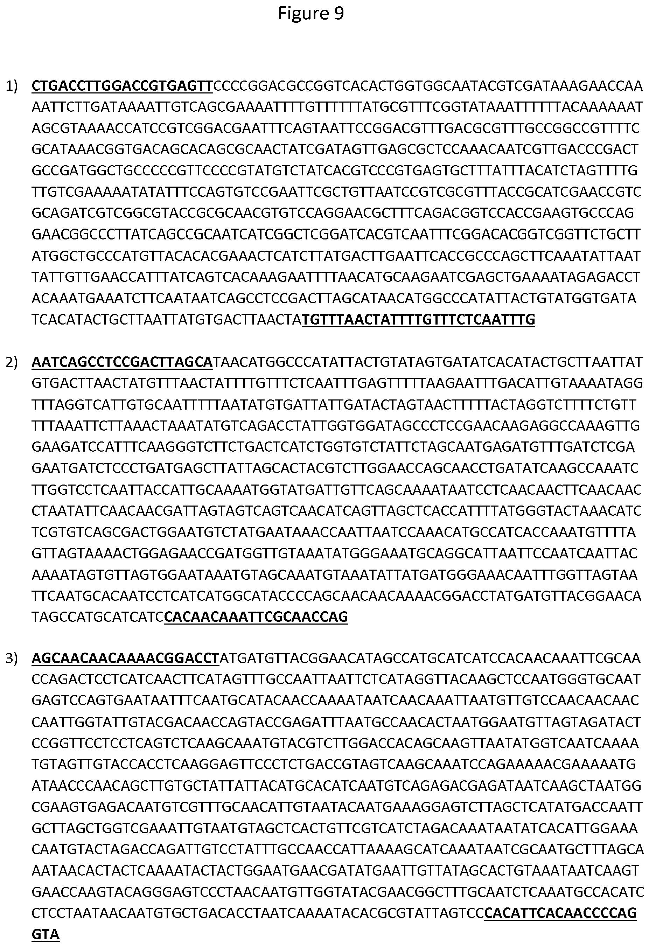

Figure 9 shows Fragment 1 to 3 of *A. pisum* p300/CBP mRNA coding cDNA, with primer positions underlined and indicated in bold (see Table 3).

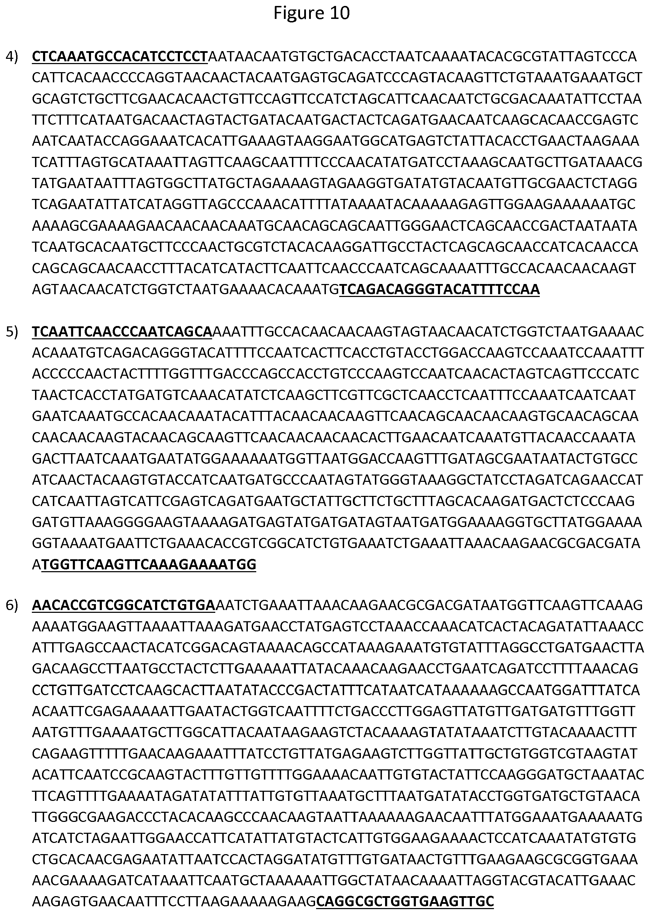

Figure 10 shows Fragment 4 to 6 of *A. pisum* p300/CBP mRNA coding cDNA, with primer positions underlined and indicated in bold (see Table 3).

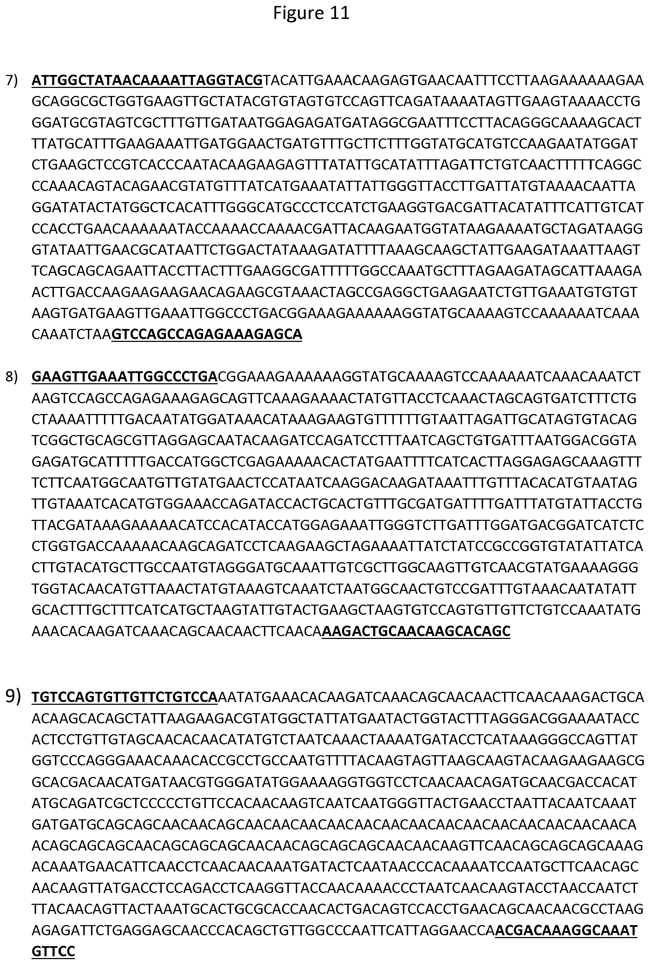

Figure 11 shows Fragment 7 to 9 of *A. pisum* p300/CBP mRNA coding cDNA, with primer positions underlined and indicated in bold (see Table 3).

### DETAILED DESCRIPTION OF THE DISCLOSURE

Disclosed herein are novel pest control methods comprising the incorporation of an inhibitor against a histone acetyltransferase (HAT) into the body of an agricultural target pest, in particular against insect pests belonging to the order Hemiptera like aphids, and to pest control agents to be used in the method.

Therefore, the present disclosure relates to methods of inhibiting the longevity/survival, development and/or reproduction of a target pest expressing a histone acetyltransferase (HAT), whereby the method comprises contacting said target pest with an inhibitor against said HAT, wherein said inhibitor inhibits the HAT expression and/or binds to a RNA and/or protein product of a gene coding the HAT.

In particular, the present disclosure pertains to pest control methods comprising incorporating an inhibitor against a histone acetyltransferase (HAT) into the body of an agricultural target pest expressing a HAT, wherein the target pest is an insect belonging to the order Hemiptera and incorporating of said inhibitor reduces/inhibits at least the reproduction (rate) and/or survival (rate) of said target pest, in particular the reduces/inhibits at least the reproduction (rate) and/or survival (rate) and/or inhibits the development of said target pest, preferably reduces/inhibits at least the reproduction (rate) and survival (rate) and inhibits the development of said target pest.

Furthermore, the present disclosure provides methods and compositions for genetic control of pest infestations. For example, the present disclosure provides recombinant DNA technologies to post-transcriptionally repress or inhibit expression/translation of a HAT coding sequence in the cell of a pest to provide a pest-protective effect by feeding to the pest one or more double stranded RNA (dsRNA) and/or small interfering ribonucleic acid (siRNA) molecules transcribed from all or a portion of a target coding sequence, thereby controlling the infestation. Therefore, the present disclosure relates to sequence-specific inhibition of expression of HAT coding sequences using double stranded RNA (dsRNA), including small interfering RNA (siRNA), to achieve the intended levels of pest control.

Surprisingly, the inventors found that inhibiting HAT, in particular against the p300/CREB-binding proteins is a universally applicable form of a multi-species target pest control like for a control of target pests belonging to the order Hemiptera, in particular belonging to the suborder Sternorrhyncha, and in particular belonging to the family of aphids like *Acyrthosiphon pisum.*

In particular, the inventors identify the HAT-encoding genes as target genes in several pests, which are for example suitable for reverse genetics of RNAi-mediated gene silencing. For example, the generation of transgenic plants expressing dsRNA targeting the HAT encoding nucleic acid in insects could be an efficient and environmentally sustainable approach to reduce chemical insecticides in agriculture. The inventor further identified that silencing of HATs in the insects, in particular of silencing p300/CREB-binding proteins in aphids, for example, induced by application of specific double stranded RNA, prevents in particular aphid growth, development, survival and/or reproduction.

In particular, the incorporation of an inhibitor according to the present disclosure in a method according to the present disclosure negatively influences survival (survival rate), reproduction and/or development of aphids.

Furthermore, the inventors show that a HAT dsRNA construct, in particular a p300/CBP dsRNA construct is likely to target multiple polyphagous aphid species. This could lead to a considerable reduction in the use of chemical insecticides, also limiting their associated costs and negative impact on the environment.

Therefore, transgenic or non-transgenic RNAi approaches targeting e.g. p300/CBP could be combined with chemical insecticides to delay or even prevent the evolution of resistance against chemical insecticides in the field.

Isolated and substantially purified nucleic acid molecules including but not limited to non-naturally occurring nucleotide sequences and recombinant DNA constructs for transcribing dsRNA molecules of the present disclosure are provided that suppress or inhibit the expression of the target coding sequence for the histone acetyltransferase (HAT) in the pest when introduced thereto. Transgenic plants that (a) contain nucleotide sequences encoding the isolated and substantially purified nucleic acid molecules and the non-naturally occurring recombinant DNA constructs for transcribing the dsRNA molecules for controlling plant pest infestations, and (b) display resistance and/or enhanced tolerance to the insect infestations, are also provided. Compositions containing the dsRNA nucleotide sequences of the present disclosure for use in topical applications onto plants or onto animals or into the environment of an animal to achieve the elimination or reduction of pest infestation are also described.

Surprisingly, the inventors found that inhibiting HAT, in particular the p300/CBP is a universally applicable form of pest control, in particular of aphid pest control. For example, the generation of transgenic plants expressing dsRNA targeted the HAT in specific insect pests is an efficient and environmentally sustainable approach to reduce the impact of insect pests on agriculture.

### Histone acetyltransferases (HATs)

In eukaryotes, chromatin modifications such as acetylation, methylation, phosphorylation, glycosylation, ubiquitination and SUMOylation play an important role in the epigenetic regulation of gene expression (Yi, 2017). One of the first discovered modifications was the acetylation of lysine residues in the N-terminal tail of the histone core proteins of the nucleosome (Bannister and Kouzarides, 2011, Burggren, 2017). As well as on histones, acetylation sites have been found on many non-histone proteins in all cellular compartments (Ali et al., 2018, Narita et al., 2019). Acetylation is therefore a major post-translational modification involved in the regulation of many biological processes including gene expression, the cell cycle, development, and aging (Ali et al., 2018). Acetylation affects the function of proteins by controlling their stability, enzymatic activity, subcellular localization, cross-talk with other chromatin modifications, and interactions with other proteins or nucleic acids (Narita et al., 2019). Here we refer to acetylation as a modification of histone proteins unless otherwise stated.

The acetylation status of histones is regulated by the opposing activity of two types of enzymes (Ali et al., 2018): histone acetyltransferases (HATs; also known as lysine acetyltransferases or KATs) and histone deacetylases (HDACs; also known as lysine deacetylases or KDACs) (Ali et al., 2018, Bannister and Kouzarides, 2011). HATs catalyze the transfer of acetyl groups to the lysine side chain of histone proteins, whereas HDACs remove these groups (Bannister and Kouzarides, 2011). Lysine acetylation is known to neutralize the positive charge on histone tails (Patel et al., 2011). This weakens the interactions between histones and DNA, thereby facilitating gene expression (Mukherjee et al., 2012). In contrast, the deacetylation of histones by HDACs makes DNA less accessible to transcription factors and this usually leads to the suppression of gene expression (Bannister and Kouzarides, 2011). A fine balance between HATs and HDACs maintains normal physiological and developmental processes (Haberland et al., 2009). Any disruption to this balance (naturally or triggered by the use of inhibitors) can cause severe changes in behavior, development, health and other life-history traits (Bassett and Barnett, 2014, Damjanovski et al., 2004, Schneider et al., 2013).

There is a remarkably diverse panel of HDACs and HATs in many organisms (Verdin and Ott, 2015). Eleven groups of HDACs (HDAC1-HDAC11) have been discovered, and these can be assigned to three subfamilies: class I or Rpd3-like proteins (Rpd3/HDAC1, HDAC2, HDAC3 and HDAC8); class II or Hda1-like proteins (HDAC4-HDAC7, HDAC9 and HDAC10); and class IV or Hos3-like proteins (HDAC11) (Ali et al., 2018). The seven members of the sirtuin family (Sir1-Sir7), which suppress gene expression at telomeres, rDNA clusters and other genomic regions (Dang, 2014, Imai and Guarente, 2016), were initially assigned as class III HDACs, but are now considered as an entirely separate group of deacetylases (Ali et al., 2018).

Similarly, HATs are assigned to three major groups: the GCN5-related N acetyltransferases (GNAT); the p300/CREB-binding proteins (p300/CBP); and the MOZ/Ybf2/Sas2/Tip60 (MYST) family (Ali et al., 2018). Other acetyltransferases exist which are not included in these three groups (Ali et al., 2018). Generally, HATs and HDACs (including sirtuins) are highly conserved from yeasts to humans (Crump et al., 2011).

In higher eukaryotes, p300 and its paralog CBP are closely related HAT enzymes that regulate the expression of genes controlling basic processes such as cellular proliferation and homeostasis (Dancy and Cole, 2015, Zucconi et al., 2019). In the literature, p300 is also known as EP300 or KAT3B, whereas CBP is known as CREBBP or KAT3A. Due to high sequence similarity between p300 and CBP, the two enzymes are collectively described as p300/CBP (Dancy and Cole, 2015). They are critical components of multiple signaling pathways that modulate protein functions and gene expression in response to a variety of signals from inside the cell and outside, including calcium levels, hypoxia, Notch and NFκB signaling, and many others (Dancy and Cole, 2015). The complex interactions with p300/CBP are mediated by multiple protein-binding domains that interact with more than 400 different protein ligands, leading to the acetylation of ∼100 protein substrates (Dancy and Cole, 2015).

Evolutionary studies have shown that p300/CBP is restricted to metazoans, suggesting that its function may be essential for the growth and development of multicellular organisms by mediating cell-to-cell signaling and controlling morphogenesis (Bordoli et al., 2001, Wang et al., 2008, Dancy and Cole, 2015). Specific chemical inhibitors of p300/CBP have shown that the acetylation activity of this enzyme controls cell migration and invasion, maintenance of the differentiated state, neurodegeneration, learning and memory (Dancy and Cole, 2015). Furthermore, the dysregulation of p300/CBP has been linked with various human diseases, including solid tumors.

Some insects (including *D. melanogaster, T. castaneum* and the moth *Galleria mellonella*) are well-established as models for the investigation of epigenetic mechanisms, including histone acetylation and deacetylation (Bingsohn et al., 2016, Gegner et al., 2019, Mukherjee et al., 2012, Vilcinskas, 2016b). The loss of p300/CBP gene function causes lethal developmental disruption in D. melanogaster. Flies producing eggs that do not express p300/CBP yield offspring with severe developmental defects, including the absence of the head, thorax, and cuticular structures. However, the genes encoding HATs and HDACs, and the effect of epigenetic modifications on the life history traits of aphids, are largely unknown.

Surprisingly the inventors found that the inhibition of a HAT like p300/CBP by RNAi induces a severe decline in life-history traits and can therefore be used as a strategy for the management of *Hemiptera* pest, in particular of a plurality of different aphid pests.

The results according to the present disclosure indicate that a nucleotide sequence, either DNA or RNA coding for a HAT like p300/CBP can be used to construct plant cells resistant to infestation by the pest. The pest host, for example, can be transformed to contain one or more of HAT encoding nucleotide sequences. The nucleotide sequence transformed into the pest host or symbiont may encode one or more RNAs that form into a dsRNA sequence in the cells or biological fluids within the transformed host or symbiont, thus making the dsRNA available in the diet of the pest if/when the pest feeds upon the transgenic host or symbiont, resulting in the suppression of expression/translation of HAT in the cells of the pest and ultimately the death, stunting, or other inhibition of the pest.

Post-transcriptional gene silencing may be used to downregulate the expression of the HAT coding gene. The gene silencing can be achieved e.g. by antisense molecules or molecules that mediate RNA interference.

Antisense polynucleotides are designed to specifically bind to RNA, resulting in the formation of RNA-DNA or RNA-RNA hybrids, with an arrest of reverse transcription or messenger RNA translation. Many forms of antisense have been developed and can be broadly categorized into enzyme-dependent antisense or steric blocking antisense. Enzyme-dependent antisense includes forms dependent on RNase H activity to degrade target mRNA, including single-stranded DNA, RNA, and phosphorothioate antisense. Antisense polynucleotides are typically generated within the cell by expression from antisense constructs that contain the antisense strand as the transcribed strand. Antisense polynucleotides will bind and/or interfere with the translation of the corresponding mRNA. Antisense RNA or antisense oligodeoxynucleotides (antisense ODNs) can both be used and may also be prepared in vitro synthetically or by means of recombinant DNA techniques. In order to avoid their digestion by DNAse, ODNs and antisense RNAs may be chemically modified. Trans-cleaving catalytic RNAs (ribozymes) are RNA molecules possessing endoribonuclease activity. Ribozymes are specifically designed for a particular target, and the target message must contain a specific nucleotide sequence.

They are engineered to cleave any RNA species site-specifically in the background of cellular RNA. The cleavage event renders the mRNA unstable and prevents protein expression.

In other advantageous embodiments, the used methods for reducing HAT expression on a post-transcriptional level are based on RNA interference (RNAi). Methods for downregulating genes by RNAi are well known to the skilled person and thus, do not need any detailed description here. Examples of RNAi inducing compounds that can be used to knockdown the expression of the HAT encoding gene include but are not limited to short interfering nucleic acids (siNA), short interfering RNA (siRNA), microRNA (miRNA), short hairpin RNAs (shRNA) as well as precursors thereof which are processed in the cell to the actual RNAi inducing compound. According to one embodiment, a siRNA is used for silencing. The siRNA may be provided as double-stranded molecule having 3' overhangs on each strand. Blunt ended molecules may also be used. Said siRNA may comprise desoxy- as well as ribonucleotides and furthermore, may comprise modified nucleotides. Several embodiments and variations of siRNA compounds are known in the prior art and can be used to reduce expression of the HAT gene. In order to efficiently induce silencing, the siRNA used as RNAi inducing compound is substantially complementary to a portion of the target gene transcript for inhibiting the expression of said gene by RNA interference.

The present disclosure relates generally to genetic control of infestations with insect pests belonging to the order Hemiptera. More particularly, the present disclosure includes methods for delivery of pest control agents to an aphid pest. Such pest control agents cause, directly or indirectly, an impairment in the ability of the pest to maintain itself, grow or otherwise infest a target pest. The present disclosure provides methods for employing stabilized dsRNA molecules in the diet of the pest as a means for suppression of the targeted genes encoding HAT, in particular p300/CBP in the pest.

In accomplishing the foregoing, the present disclosure provides methods of inhibiting expression of a HAT encoding target gene in an insect pest, in particular in an insect belonging to the order Hemiptera including insects belonging to the suborder Sternorrhyncha, in particular in aphids like *Acyrthosiphon pisum,* resulting in the cessation of survival, growth, development and/or reproduction of the pest.

The method comprises in one embodiment introducing partial or fully stabilized double-stranded RNA (dsRNA) nucleotide molecules into a nutritional composition that the pest relies on as a food source, and making the nutritional composition available to the pest for feeding. Ingestion of the nutritional composition containing the double stranded or siRNA molecules results in the uptake of the molecules by the cells of the pest, resulting in the inhibition of expression of at least one target gene in the cells of the pest. Inhibition of the target gene exerts a deleterious effect upon the pest.

In certain embodiments, dsRNA molecules provided by the disclosure comprise nucleotide sequences complementary to a nucleic acid sequence comprised in SEQ ID NO:1, the inhibition of which in a pest organism results in the reduction or removal of HAT, in particular of p300/CBP. The nucleotide sequence selected may exhibit from about 80% to at least about 100% sequence identity to 16 to 25 contiguous nucleotides of SEQ ID NO:1, including the complement thereof. Such inhibition can be described as specific in that a nucleotide sequence from a portion of the HAT encoding target gene is chosen from which the inhibitory dsRNA or siRNA is transcribed. The method is effective in inhibiting the expression of the HAT target gene and can be used to inhibit many different types of pests, in particular many types of aphids. In a particular embodiment, the nucleotide sequence is coded by SEQ ID NO:2.

In advantageous embodiments, the nucleic acid sequences identified as having a pest protective effect may be readily expressed as dsRNA molecules through the creation of appropriate expression constructs. For example, such sequences can be expressed as a hairpin and stem and loop structure by taking a first segment corresponding to SEQ ID NO:1 or a fragment thereof, linking this sequence to a second segment spacer region that is not homologous or complementary to the first segment, and linking this to a third segment that transcribes an RNA, wherein at least a portion of the third segment is substantially complementary to the first segment. Such a construct forms a stem and loop structure by hybridization of the first segment with the third segment and a loop structure forms comprising the second segment (WO94/01550, WO98/05770, US 2002/0048814A1, and US 2003/0018993 A1).

The present invention finds application in any area where it is desirable to inhibit viability, growth, development or reproduction of a pest, or to decrease pathogenicity or infectivity of a pest.

Administering or exposing a double stranded ribonucleic acid molecule to a pest results in one or more of the following attributes: reduction in feeding by the pest, reduction in viability of the pest, death of the pest, inhibition of differentiation and development of the pest, absence of or reduced capacity for sexual reproduction by the pest, muscle formation, juvenile hormone formation, juvenile hormone regulation, ion regulation and transport, maintenance of cell membrane potential, amino acid biosynthesis, amino acid degradation, sperm formation, pheromone synthesis, pheromone sensing, antennae formation, wing formation, leg formation, development and differentiation, egg formation, larval maturation, digestive enzyme formation, haemolymph synthesis, haemolymph maintenance, neurotransmission, cell division, energy metabolism, respiration, apoptosis, and any component of a eukaryotic cells' cytoskeletal structure, such as, for example, actins and tubulins. Any one or any combination of these attributes can result in an effective inhibition of pest infestation.

### A. Definitions

As used in the present disclosure, "cell", "cell line", and "cell culture" can be used interchangeably and all such designations include progeny. Thus, the words "transformants" or "transformed cells" include the primary subject cell and cultures derived therefrom without regard for the number of transfers. It is also understood that all progeny may not be precisely identical in DNA content, due to deliberate or inadvertent mutations. Mutant progeny that have the same functionality as screened for in the originally transformed cell are included.

As used herein, the phrase "coding sequence", "encoding sequence", "structural nucleotide sequence" or "structural nucleic acid molecule" refers to a nucleotide sequence that is translated into a polypeptide, usually via mRNA, when placed under the control of appropriate regulatory sequences. The boundaries of the coding sequence are determined by a translation start codon at the 5'-terminus and a translation stop codon at the 3'-terminus. A coding sequence can include, but is not limited to, genomic DNA, cDNA, EST and recombinant nucleotide sequences.

The term "complementary" as used herein refers to a relationship between two nucleic acid sequences. One nucleic acid sequence is complementary to a second nucleic acid sequence if it is capable of forming a duplex with the second nucleic acid, wherein each residue of the duplex forms a guanosine-cytidine (G-C) or adenosine-thymidine (A-T) base pair or an equivalent base pair. Equivalent base pairs can include nucleoside or nucleotide analogues other than guanosine, cytidine, adenosine, or thymidine.

The term "derivative" as used herein, refers to a nucleic acid molecule that has similar binding characteristics to the HAT target nucleic acid sequence as a nucleic acid molecule according to one of the claimed sequences.

As used herein, the term "derived from" refers to a specified nucleotide sequence that may be obtained from a particular specified nucleic acid sequence. Therefore, as used herein the term "nucleic acid sequence derived from" refers to polynucleotides comprising the nucleic acid sequence of polynucleotide. Therefore, the term refers in particular to a continuous part of the full-length nucleic acid sequence with or without mutations, which is separate from and not in the context of the full-length nucleic acid sequence. The term "derived from" include e.g polynucleotides derived from SEQ ID NO. 1 and/or short nucleotides derived from SEQ ID NO.1, in particular from SEQ ID NO.2, SEQ ID NO.3 or SEQ ID NO. 9, or from variants thereof, wherein said variants are at least 80%, at least 85 %, in particular of at least 90%, in particular of at least 95%, and more in particular of at least 98% identical to the nucleic acid sequence of SEQ ID NO. 1, SEQ ID NO.2, SEQ ID NO. 3 or SEQ ID NO.9, In particular, advantageous embodiments of nucleic acid sequences derived from SEQ ID NO. 1 are SEQ ID NO. 2, SEQ ID NO. 3, SEQ ID NO. 4, SEQ ID NO. 5, SEQ ID NO. 6 SEQ ID NO. 7, SEQ ID NO. 8, SEQ ID NO. 9, SEQ ID NO. 10 SEQ ID NO. 11 and SEQ ID NO. 12, or fragments, analogs, derivatives or elongations thereof having at least 85%, at least 90%, at least 95 or at least 99 % identity to SEQ ID NO. 2, SEQ ID NO. 3, SEQ ID NO. 4, SEQ ID NO. 5, SEQ ID NO. 6 SEQ ID NO. 7, SEQ ID NO. 8, SEQ ID NO. 9, SEQ ID NO. 10 SEQ ID NO. 11 or SEQ ID NO. 12, wherein the nucleic acid sequences derived from SEQ ID NO. 1 or the coded dsRNA or siRNA must reduce the reproduction and/or survival of the target pest. The term "derived from" in particular pertains to dsRNA coded by SEQ ID NO.1 or parts thereof like SEQ ID NO. 2.

In an advantageous embodiment, the inhibitor against HAT, in particular against p300/CBP is a dsRNA derived from SEQ ID NO. 1, in particular from SEQ ID NO. 9 and reduces the reproduction and/or survival of the target pest, wherein the target pest is an insect belonging to the order Hemiptera, in particular belonging to the suborder Sternorrhyncha, in particular belonging to the family of aphids like *Acyrthosiphon pisum.*

The term "expression clone" refers to DNA sequences containing a desired coding sequence and control sequences in operable linkage, so that hosts transformed with these sequences are capable of producing the encoded proteins. The term "expression system" refers to a host transformed with an expression clone. To effect transformation, the expression clone may be included on a vector; however, the relevant DNA may also be integrated into the host chromosome.

The term "gene" refers to a DNA sequence that comprises control and coding sequences necessary for the production of a recoverable bioactive polypeptide or precursor.

The term "homologue of the nucleic acid molecule" refers to a nucleic acid molecule the sequence of which has one or more nucleotides added, deleted, substituted or otherwise chemically modified in comparison to a nucleic acid molecule according to one of the claimed sequences, provided always that the homologue retains substantially the same inhibitory effect on HAT, in particular to p300 and/or its paralog CREB-binding protein.

The term "isolated" describes any molecule separated from its natural source.

As used herein, the term "nucleic acid" refers to a single or double-stranded polymer of deoxyribonucleotide or ribonucleotide bases read from the 5' to the 3' end. The "nucleic acid" may also optionally contain non-naturally occurring or altered nucleotide bases that permit correct read through by a polymerase and do not reduce expression of a polypeptide encoded by that nucleic acid.

The term "nucleotide sequence" or "nucleic acid sequence" refers to both the sense and antisense strands of a nucleic acid as either individual single strands or in the duplex. The term "ribonucleic acid" (RNA) is inclusive of RNAi (inhibitory RNA), dsRNA (double stranded RNA), siRNA (small interfering RNA), mRNA (messenger RNA), miRNA (micro-RNA), tRNA (transfer RNA, whether charged or discharged with a corresponding acylated amino acid), and cRNA (complementary RNA) and the term "deoxyribonucleic acid" (DNA) is inclusive of cDNA and genomic DNA and DNA-RNA hybrids. The words "nucleic acid segment", "nucleotide sequence segment", or more generally "segment" will be understood by those in the art as a functional term that includes both genomic sequences, ribosomal RNA sequences, transfer RNA sequences, messenger RNA sequences, operon sequences and smaller engineered nucleotide sequences that express or may be adapted to express, proteins, polypeptides or peptides.

Provided according to the disclosure are nucleotide sequences, the expression of which results in an RNA sequence which is substantially homologous to an RNA molecule of a targeted gene encoding HAT in an insect that comprises an RNA sequence encoded by a nucleotide sequence within the genome of the insect. Thus, after ingestion of the stabilized RNA sequence down- regulation of the nucleotide sequence of the target gene in the cells of the insect may be obtained resulting in a deleterious effect on the maintenance, viability, proliferation, reproduction and infestation of the insect.

As used herein, the term "homologous" or "homologs", with reference to a nucleic acid sequence, includes a nucleotide sequence that hybridizes under stringent conditions to one of the coding sequences of SEQ ID NO:1, SEQ ID NO. 2, SEQ ID NO. 3 or SEQ ID NO. 9, or the complements thereof. Sequences that hybridize for example under stringent conditions to SEQ ID NO:1, or the complements thereof, are those that allow an antiparallel alignment to take place between the two sequences, and the two sequences are then able, under stringent conditions, to form hydrogen bonds with corresponding bases on the opposite strand to form a duplex molecule that is sufficiently stable under the stringent conditions to be detectable using methods well known in the art. Substantially homologous sequences have preferably from about 70% to about 80% sequence identity, or more preferably from about 80% to about 85% sequence identity, or most preferable from about 90% to about 95% sequence identity, to about 99% sequence identity, to the referent nucleotide sequences of SEQ ID NO:1, SEQ ID NO. 2, SEQ ID NO. 3 or SEQ ID NO. 9, or to the sequence of SEQ ID NO:2 as set forth in the sequence listing, or the complements thereof.

As used herein, the term "insect control agent", or "gene suppression agent" refers to a particular RNA molecule comprising a first RNA segment and a second RNA segment, wherein the complementarity between the first and the second RNA segments results in the ability of the two segments to hybridize in vivo and in vitro to form a double stranded molecule. It may generally be preferable to include a third RNA segment linking and stabilizing the first and second sequences such that the entire structure forms into a stem and loop structure, or even more tightly hybridizing structures may form into a stem-loop knotted structure. Alternatively, a symmetrical hairpin could be formed without a third segment in which there is no designed loop, but for steric reasons a hairpin would create its own loop when the stem is long enough to stabilize itself. The first and the second RNA segments will generally be within the length of the RNA molecule and are substantially inverted repeats of each other and linked together by the third RNA segment. The first and the second segments correspond invariably and not respectively to a sense and an antisense sequence with respect to the target RNA transcribed from the target gene in the target insect pest that is suppressed by the uptake of the dsRNA molecule. The insect control agent can also be a substantially purified (or isolated) nucleic acid molecule and more specifically nucleic acid molecules or nucleic acid fragment molecules thereof from a genomic DNA (gDNA) or cDNA library. Alternatively, the fragments may comprise smaller oligonucleotides having from about 15 to about 250 nucleotide residues, and more preferably, about 15 to about 30 nucleotide residues.

As used herein, the phrase "inhibition of gene expression" or "inhibiting expression of a target gene in the cell of an insect" refers to the absence (or observable decrease) in the level of protein and/or mRNA product from the target gene. Specificity refers to the ability to inhibit the target gene without manifest effects on other genes of the cell and without any effects on any gene within the cell that is producing the dsRNA molecule. The inhibition of gene expression of the target gene in the insect pest may result in novel phenotypic traits in the insect pest.

The term "microorganism" includes prokaryotic and eukaryotic microbial species such as bacteria, fungi and algae. Fungi include yeasts and filamentous fungi, among others. Illustrative prokaryotes, both Gram-negative and Gram-positive, include Enterobacteriaceae, such as Escherichia, Erwinia, Shigella, Salmonella, and Proteus; Bacillaceae; Rhizobiceae, such as Rhizobium; Spirillaceae, such as photobacterium, Zymomonas, Serratia, Aeromonas, Vibrio, Desulfovibrio, Spirillum', Lactobacillaceae; Pseudomoriadaceae, such as Pseudomonas and Acetobacter; Azotobacteraceae, Actinomycetales, and Nitrobacteraceae. Among eukaryotes are fungi, such as Phycomycetes and Ascomycetes, which includes yeast, such as Saccharomyces and Schizosaccharomyces; and Basidiomycetes, such as Rhodotorula, Aureobasidium, Sporobolomyces, and the like.

The term "operably linked", as used in reference to a regulatory sequence and a structural nucleotide sequence, means that the regulatory sequence causes regulated expression of the linked structural nucleotide sequence. "Regulatory sequences" or "control elements" refer to nucleotide sequences located upstream (5' noncoding sequences), within, or downstream (3' non-translated sequences) of a structural nucleotide sequence, and which influence the timing and level or amount of transcription, RNA processing or stability, or translation of the associated structural nucleotide sequence. Regulatory sequences may include promoters, translation leader sequences, introns, enhancers, stem-loop structures, repressor binding sequences, and polyadenylation recognition sequences and the like.

In the present description, "pest control" refers to the removal or the reduction of harm of pests. The concept of "pest control" include reducing of the target pest, killing of pests (extermination), pest proliferation inhibition, pest development inhibition, pest growth inhibition, repelling of pests (repellence), reducing of the survival rate of the target pest and the removal or the reduction of harm of pests (for example, inhibition of ingestion capacity of agricultural pests).

The term "plant" includes the plant body, plant organs (for example, leaves, petals, stem, root, rhizome, and seeds), plant tissues (for example, epidermis, phloem, parenchyma, xylem, and vascular bundle), and plant cells. In addition, the term "plant cell" includes seed suspension cultures, embryos, meristematic tissue regions, callus tissues, cells derived from leaves and roots, and gametophytes (embryos and pollens) and their precursors. When plant culture cells are transformed, an organ or individual is regenerated from the transformed cells by a known tissue culture method. These operations are readily performed by those skilled in the art. An example is described below. Firstly, the transformed plant cells are cultured in a sterilized callus forming medium (containing a carbon source, saccharides, vitamins, inorganics, and phytohormones such as auxin and cytokinin), thereby forming a dedifferentiated calluse which indefinitely proliferates (callus induction). The formed callus is transferred to a new medium containing a plant growth regulator such as auxin, and further proliferated thereon (subcultivation). When the callus induction is carried out on a solid medium such as agar and subcultivation is carried out in a liquid medium, the respective cultures are efficiently achieved. Secondly, the callus proliferated by subcultivation was cultured under appropriate conditions, thereby inducing redifferentiation of the organ (inductive redifferentiation), and regenerating the plant body. The inductive redifferentiation is achieved by appropriately adjusting the type and amount of the various components of the medium, including plant growth regulators such as auxin and cytokinin, and the carbon source, and the light and temperature. The inductive redifferentiation forms adventitious embryos, adventitious roots, adventitious buds, adventitious foliage, and others, and they are grown into a complete plant body. The plant before being a complete plant body may be stored in the form of, for example, capsulated artificial seeds, dry embryos, lyophilized cells, or tissues.

The term "plasmid", "vector system", "vector" or "expression vector" means a construct capable of *in vivo* or *in vitro* expression. In the context of the present disclosure, these constructs may be used to introduce genes encoding enzymes into host cells.

The term "p300/CBP" is used herein for p300 and its paralog CBP that are closely related HAT enzymes that regulate the expression of genes controlling basic processes such as cellular proliferation and homeostasis (Dancy and Cole, 2015, Zucconi et al., 2019). In the literature, p300 is also known as EP300 or KAT3B, whereas CBP is known as CREBBP or KAT3A. Due to the high sequence similarity between p300 and CBP, the two enzymes are collectively described as p300/CBP (Dancy and Cole, 2015). They are critical components of multiple signaling pathways that modulate protein functions and gene expression in response to a variety of signals from inside the cell and outside, including calcium levels, hypoxia, Notch and NFκB signaling, and many others (Dancy and Cole, 2015). The complex interactions with p300/CBP are mediated by multiple protein-binding domains that interact with more than 400 different protein ligands, leading to the acetylation of ∼100 protein substrates (Dancy and Cole, 2015).

An inhibitor of p300/CBP could inhibit p300 and/or CBP alone or both of them, as long as the reproduction and/or survival of said target pest is given. In advantageous embodiments, the preferred HAT according to the present disclosure is p300/CBP.

The term "polynucleotide" corresponds to any genetic material of any length and any sequence, comprising single-stranded and double-stranded DNA and RNA molecules, including regulatory elements, structural genes, groups of genes, plasmids, whole genomes and fragments thereof.

The term "recombinant DNA" or "recombinant nucleotide sequence" refers to DNA that contains a genetically engineered modification through manipulation via mutagenesis, restriction enzymes, and the like.

The term "stringent conditions" relates to conditions under which a probe will hybridize to its target subsequence, but to no other sequences. Stringent conditions are sequence-dependent and will be different in different circumstances. Longer sequences hybridize specifically at higher temperatures. Generally, stringent conditions are selected to be about 5° C lower than the thermal melting point (Tm) for the specific sequence at a defined ionic strength and pH. The Tm is the temperature (under defined ionic strength, pH and nucleic acid concentration) at which 50% of the probes complementary to the target sequence hybridize to the target sequence at equilibrium. (As the target sequences are generally present in excess, at Tm, 50% of the probes are occupied at equilibrium). Typically, stringent conditions will be those in which the salt concentration is less than about 1.0 M Na ion, typically about 0.01 to 1.0 M Na ion (or other salts) at pH 7.0 to 8.3 and the temperature is at least about 30° C for short probes (e.g. 10 to 50 nucleotides) and at least about 60° C for longer probes. Stringent conditions may also be achieved with the addition of destabilizing agents, such as formamide and the like.

As used herein, the term "sequence identity", "sequence similarity" or "homology" is used to describe sequence relationships between two or more nucleotide sequences. The percentage of "sequence identity" between two sequences is determined by comparing two optimally aligned sequences over a comparison window, wherein the portion of the sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison, and multiplying the result by 100 to yield the percentage of sequence identity. A sequence that is identical at every position in comparison to a reference sequence is said to be identical to the reference sequence and vice-versa. A first nucleotide sequence when observed in the 5' to 3' direction is said to be a "complement" of, or complementary to, a second or reference nucleotide sequence observed in the 3' to 5' direction if the first nucleotide sequence exhibits complete complementarity with the second or reference sequence. As used herein, nucleic acid sequence molecules are said to exhibit "complete complementarity" when every nucleotide of one of the sequences read 5' to 3' is complementary to every nucleotide of the other sequence when read 3' to 5'. A nucleotide sequence that is complementary to a reference nucleotide sequence will exhibit a sequence identical to the reverse complement sequence of the reference nucleotide sequence. These terms and descriptions are well defined in the art and are easily understood by those of ordinary skill in the art.

By "synergistic" it is meant to include the synergistic effects of the combination on the pesticidal activity (or efficacy) of the combination of the transgenic event and the pesticide. However, it is not intended that such synergistic effects be limited to the pesticidal activity, but that they should also include such unexpected advantages as increased scope of activity, advantageous activity profile as related to type and amount of damage reduction, decreased cost of pesticide and application, decreased pesticide distribution in the environment, decreased pesticide exposure of personnel who produce, handle and plant corn seeds, and other advantages known to those skilled in the art.

The term "variant of the nucleic acid molecule" refers herein to a nucleic acid molecule which is substantially similar in structure and biological activity to a nucleic acid molecule according to one of the claimed sequences.

The "pest" refers to the pest subjected to pest control or the pest controlled by the present disclosure. The pest may be two or more pests and are not particularly limited. In general, pests are broadly divided into agricultural pests, sanitary pests, and unpleasant pests. "Agricultural pests" refer to the pests that attack crops (including garden crops and crops during storage). "Sanitary pests" refer to the pests that attack the sanitary environment of human. In addition, "unpleasant pests" refer to the pests that attack the mood of human by their appearance or motion. The present disclosure is also applicable to the pests that attack the assets of human (for example, termite and bristletail) and livestock (for example, mosquito and parasite).

Therefore, as used herein, the term "target pest" refers to insects, arachnids, crustaceans, fungi, bacteria, viruses, nematodes, flatworms, roundworms, pinworms, hookworms, tapeworms, trypanosomes, schistosomes, botflies, fleas, ticks, mites, and lice and the like that are pervasive in the human environment and that may ingest or contact one or more cells, tissues, or fluids produced by a pest host or symbiont transformed to express or coated with a double stranded gene suppression agent or that may ingest plant material containing the gene suppression agent.

As used herein, a "pest resistance" trait is a characteristic of a transgenic plant, transgenic animal, transgenic host or transgenic symbiont that causes the plant, animal, host, or symbiont to be resistant to attack from a pest that typically is capable of inflicting damage or loss to the plant, animal, host or symbiont. Such pest resistance can arise from a natural mutation or more typically from incorporation of recombinant DNA that confers pest resistance. To impart insect resistance to a transgenic plant a recombinant DNA can, for example, be transcribed into a RNA molecule that forms a dsRNA molecule within the tissues or fluids of the recombinant plant. The dsRNA molecule is comprised in part of a segment of RNA that is identical to a corresponding RNA segment encoded from a DNA sequence within an insect pest that prefers to feed on the recombinant plant. Expression of the gene within the target insect pest is suppressed by the dsRNA, and the suppression of expression of the gene in the target insect pest results in the plant being insect resistant. Fire et al. (U.S. Patent No. 6,506,599) generically described inhibition of pest infestation, providing specifics only about several nucleotide sequences that were effective for inhibition of gene function in the nematode species *Caenorhabditis elegans.* Similarly, Plaetinck et al. (US 2003/0061626) describe the use of dsRNA for inhibiting gene function in a variety of nematode pests. Mesa et al. (US 2003/0150017) describe using dsDNA sequences to transform host cells to express corresponding dsRNA sequences that are substantially identical to target sequences in specific pathogens, and particularly describe constructing recombinant plants expressing such dsRNA sequences for ingestion by various plant pests, facilitating down-regulation of a gene in the genome of the pest and improving the resistance of the plant to the pest infestation.

The advantageous effect that incorporating of the HAT inhibitor reduces at least the reproduction and/or survival of said target pest, in particular the reproduction and/or survival and/or the inhibition of the development of the Hemiptera target pest could be measured by monitoring the following parameters: aphid survival, age of first reproduction, total number of offspring, average number of premature (dead) offspring, number of premature offspring per day, and number of viviparous (normal) offspring per day (Skaljac et al., 2018).

The intent is to reduce the number of pests to a level where the harm they are causing is acceptable. Once a pest's presence is detected and the decision is made that control is necessary, suppression and prevention often are joint goals. The right combination of control measures can often suppress the pests already present and prevent them from building up again to a level where they are causing unacceptable harm.

### B. Target Pests

The present disclosure pertains to pest control methods comprising incorporating an inhibitor against a histone acetyltransferase (HAT) into the body of an agricultural target pest expressing a HAT, wherein the target pest is an insect belonging to the order Hemiptera and the uptake by the target pest of said inhibitor reduce at least the reproduction and/or survival (or survival rate) of said target pest. In particular, the mRNA encoding the HAT is coded by a nucleic acid sequence comprising the sequence set forth in SEQ ID NO: 1, or homologs thereof, wherein said homologs may have a sequence identity of at least 80 %, in particular of at least 85%, in particular of at least 90%, in particular of at least 95, 96%, 97%, 98%, 99 % to SEQ ID NO: 1 and the coded dsRNA reduces the reproduction and/or survival of said target pest. In an advantageous example, said homologs of SEQ ID NO: 1 are parts of sequences that encode a functional HAT, in particular a functional p300/CBP in the target pest or SEQ ID NO: 1 with one or more mutation. In particular, the incorporation/uptake of said inhibitor against a histone acetyltransferase (HAT), in particular of a dsRNA derived from SEQ ID. NO.1 results in the inhibition of the reproduction (or reproduction rate), the survival (or survival rate) and of the development of the Hemiptera target pest. In particular, the HAT inhibitor is delivered by spraying on the target pest and/or the infested plant.

In advantageous embodiments of the present disclosure, the target pests are insects belonging to the insect order Hemiptera also known as the true bugs. Many Hemipteran insects are important agricultural pests because they cause direct feeding damage to their host plants and/or transmit plant disease agents including viruses and bacteria. Microscopic and behavioral studies on different Hemiptera species showed that their exuviae (molted skins) normally had either fully or partially extended stylets in a feeding-like position. In most cases these stylets were still partially embedded in their host plants after ecdysis, which indicated that plant-feeding hemipteran nymphs use their stylets to anchor themselves to host plants during molting.

Examples of the target pests belonging to Hemiptera include insects of the suborder Sternorrhyncha including aphids. In particular, examples of the target pests belonging to Hemiptera include *Nilaparvata lugens, Sogatella furcifera, Laodelphax stratella, Nephotettix cincticeps, Recilia dorsalis, Stenotus rubrovittatus, Trigonotylus caelestialium, Leptocorisa chinensis, Nezara antennata, Nezara viridula, Lagynotomus elongatus, Scotinophara lurida, Eysarcoris annamita, Eysarcoris lewisi, Eysarcoris ventralis, Togo hemipterus Scott, Cletus punctiger, Piezodorus hybneri, Halyomorpha halys, Dolycoris baccarum, Neotoxoptera formosana, Rhopalosiphum padi, Rhopalosiphum maidis, Acyrthosiphon pisum* and *Aphis glycines.*

In advantageous embodiments, the target pests are belonging to the genera of aphids, in particular *Acyrthosiphon pisum.*

### C. HAT Inhibitor

According to the pest control methods of the present disclosure, an inhibitor against HAT is incorporated into the body of the target pest. The term "incorporated" includes active and/or passive uptake of the inhibitor into the body of the target pest. The term "HAT inhibitor" is used as the generic name of the substances inhibiting HAT, in particular the p300/CBP. The HAT inhibitor may be of any type as long as it has inhibitory against the expression, the transcription and/or the translation of HAT and/or has inhibitory activity against HAT, in particular against the activity of p300/CBP. For example, an HAT Inhibitor according to the present disclosure may prevent translation of a particular mRNA, thereby inhibiting the biological activity of the HAT encoded by the mRNA or other biological activity of the pest.

Examples of the HAT inhibitor include a nucleic acid that inhibits the expression of the HAT gene, and a substance that specifically binds to HAT (for example, an antibody or a low molecular weight compound). The former one is further described below. The substance that specifically binds to HAT may be obtained or prepared using binding assay targeted at HAT. An antibody that specifically binds to HAT may be prepared using, for example, an immunological method, a phage display method, or a ribosome display method.

According to one aspect of the present disclosure, a compound selected from the group consisting of the following (a) to (d) is used as the HAT inhibitor:
(a) a RNAi inducing compound targeted a nucleic acid coding HAT or parts thereof;
(b) a nucleic acid construct intracellularly producing a RNAi inducing compound targeted a nucleic acid coding HAT or parts thereof;
(c) an antisense nucleic acid targeted at the transcript product of a gene coding HAT of the target pest; and
(d) a ribozyme targeted at the transcript product of a gene coding HAT of the target pest.

The (a) and (b) are the compounds used for the inhibition of expression by so-called RNA interference (RNAi). In other words, when the compound (a) or (b) is used, the expression of HAT is inhibited by RNAi, whereby pest control effect is achieved. In this manner, the use of RNAi allows specific control of the target pest, and facilitates rapid achievement of pest control effect. Furthermore, owing to its properties, the possibility of occurrence of resistant strains is likely extremely low. In addition, RNAi does not modify plant genes, and thus will not genetically influence them.

The "RNAi" refers to the inhibition of expression and/or blocking of the translation of the target gene by the introduction of an RNA composed of a sequence homologous to that of the target gene (specifically homologue to the mRNA corresponding to the target gene) into the target cell. For the inhibition of expression using the RNAi method in pests such as insects, generally, a double strand RNA (dsRNA) composed of a sequence corresponding a part of the target gene (the gene coding HAT, in particular p300/CBP of the target pest). Two or more dsRNAs may be used for one target gene.

The RNAi targeted at the gene of a mammal cell uses a short dsRNA (siRNA) of about 16 to 25 nucleotides. When the RNAi is targeted at the gene of a pest such as an insect, a long dsRNA of more than several hundreds of nucleotides is preferred because owing to its effectiveness. The length of the dsRNA used for RNAi is, for example, 30 nucleotides or more, and preferably 200 nucleotides or more (for example, from 200 to 500 nucleotides). The use of a dsRNA is preferred for inducing effective inhibition of expression, but the use of a single strand RNA will also be contemplated. The dsRNA used herein is not necessarily composed of two molecules of sense and antisense strands, and, for example, may have a structure wherein the sense and antisense strands composing the dsRNA are connected via a hairpin loop. A dsRNA composed of a modified RNA may be used. Examples of the modification include phosphorothioation, and the use of a modified base (for example, fluorescence-labeled base). In advantageous embodiments, the RNAi inducing compound is a compound selected from the group consisting of short interfering nucleic acids, siNA, short interfering RNA (siRNA), microRNA (miRNA), short hairpin RNAs (shRNA) and precursors thereof which are processed in the cell to the actual RNAi inducing compound. In a preferred embodiment, the precursor is double-stranded RNA (dsRNA). An example of a dsRNA used in the pest control method according to the present disclosure is a dsRNA coded by the sequence set forth in SEQ ID NO: 2, or homologs thereof, wherein said homologs have a sequence identity of at least 80 %, in particular of at least 85%, in particular of at least 90% to SEQ ID NO: 2 and the dsRNA reduce the reproduction and/or survival of a target pest..

An RNAi specific to the target gene can be also produced by intracellular expression of a dsRNA targeted at the target gene. The nucleic acid construct (b) is used as such a means.

The dsRNA used in the RNAi method may be prepared by chemical synthesis, or *in vitro* or *in vivo* using an appropriate expression vector. The method using an expression vector is particularly effective for the preparation of a relatively long dsRNA. The design of dsRNA normally includes the sequence (continuous sequence) specific to the target nucleic acid. Programs and algorithms for selecting an appropriate target sequence have been developed.

RNAi-mediated aphid control has been extensively reviewed and the following articles can be consulted for additional information (Gong et al., 2014, Coleman et al., 2014, Mao and Zeng, 2014, Jaubert-Possamai et al., 2007, Will and Vilcinskas, 2013, Mao and Zeng, 2012, Yu et al., 2016), wherein all articles are hereby incorporated by reference to the present diclosure.

**Table 1. Examples of RNAi technology as an effective tool for aphid control.**

| **Delivery methods of dsRNA or siRNA** | **Aphid species** | **Target and gene** | **Delivered volume and concentration/length of dsRNA or siRNA (bp)** | **Life-history trait negatively affected by RNAi** | **Reference** |
|---|---|---|---|---|---|
| Injection | *Acyrthosiphon pisum* | Salivary gland transcript (*Coo2*) | 5 nl (10 µg/µl)/660 bp | Survival | Mutti et al. (2006) |
| Injection | *Acyrthosiphon pisum* | Structural sheath protein *(HAT)* | 15 nl (50 ng)/491 bp | Reproduction | Will and Vilcinskas (2015); patent 20160135466 |
| Transgenic plant | *Sitobion avenae* | Structural sheath protein *(HAT)* | 491 bp | Development, reproduction, and survival | Abdellatef et al. (2015) |
| Injection | *Acyrthosiphon pisum* | Heat shock protein 83 (*HSP83*) | 6.9 nl (~50 ng)/530 bp | Survival, reproduction, development | Will et al. (2017) |
| Injection; oral | *Acyrthosiphon pisum* | V-type ATPase subunit E *(VTE)* | Injections: 7.5-30 ng/277 bp; Oral: 250 ng/µl and 500 ng/µl/277 bp | Injection: survival; Oral: development, reproduction | Cao et al. (2018) |
| Injection; plant-mediated (soaking) | *Acyrthosiphon pisum* | Chitin synthase (CHS) | Injections: ∼600 ng/364 bp Plant-mediated: 1 ml (1200 ng/µl)/364 bp | Injection: survival, development; Plant-mediated: development | Ye et al. (2019) |
| Oral | *Myzus persicae* | Voltage-gated sodium channels (*VGSC*) | 750 ng/µl/289 bp | Survival, reproduction | Tariq et al. (2019) |

Patents related to the application of RNAi for the control of hemipteran insects are listed in Table 2, wherein all patents and patent application are hereby incorporated by reference to the present diclosure.

**Table 2. Selected RNAi invention disclosures covering hemipteran insects.**

| **Patent number** | **Publication date** | **Topic** | **Institution** | **Source** |
|---|---|---|---|---|
| WO2016191357 | 01.12.2016 | Thread nucleic acid molecules that confer resistance to hemipteran pests | Dow AgroSciences LLC | World Intellectual Property Organization (WIPO) |
| WO2016060913 | 21.04.2016 | COPI coatomer beta subunit nucleic acid molecules that confer resistance to coleopteran and hemipteran pests | Dow AgroSciences LLC | WIPO |
| WO2016060914 | 21.04.2016 | COPI coatomer delta subunit nucleic acid molecules that confer resistance to coleopteran and hemipteran pests | Dow AgroSciences LLC | WIPO |
| WO2016060911 | 21.04.2016 | OPI coatomer gamma subunit nucleic acid molecules that confer resistance to coleopteran and hemipteran pests | Dow AgroSciences LLC | WIPO |
| WO2015171648 | 12.11.2015 | Sec23 nucleic acid molecules that confer resistance to coleopteran and hemipteran pests | Dow AgroSciences LLC; The Board of Regents of the University of Nebraska | WIPO |
| WO2016057822 | 14.04.2016 | Gho/sec24b2 and sec24b1 nucleic acid molecules to control coleopteran and hemipteran pests | Dow AgroSciences LLC; The Board of Regents of the University of Nebraska | WIPO |
| WO2016100490 | 23.06.2016 | Parental RNAi suppression of *hunchback* gene to control hemipteran pests | Dow AgroSciences LLC; The Board of Regents of the University of Nebraska | WIPO |
| WO2016100507 | 23.06.2016 | Parental RNAi suppression of chromatin remodeling genes to control hemipteran pests | Dow AgroSciences LLC; The Board of Regents of the University of Nebraska | WIPO |
| WO2015095774A1 | 25.06.2015 | Ras opposite (ROP) and related nucleic acid molecules that confer resistance to coleopteran and/or hemipteran pests | Dow AgroSciences LLC ; Fraunhofer IME, Giessen | Google Patents |
| WO2016196247 A1 | 08.12.2016 | Spt5 nucleic acid molecules to control insect pests | Dow Agrosciences LLC; Fraunhofer IME, Giessen | Google Patents |
| WO2016149178A1 | 22.09.2016 | RNA polymerase ii215 nucleic acid molecules to control insect pests | Dow AgroSciences LLC; Fraunhofer IME, Giessen | Google Patents |
| US20160355841A1 | 08.12.2016 | RNA polymerase ii33 nucleic acid molecules to control insect pests | Dow AgroSciences LLC; Fraunhofer IME, Giessen | Google Patents |
| WO2014159829 | 02.10.2014 | Compositions and methods for insecticidal control of stinkbugs | Du Pont de Nemours & Co | WIPO |
| WO2009091864 | 23.07.2009 | Compositions and methods for the suppression of target polynucleotides from Lygus | Pioneer Hi-Bred International, Dupont de Nemours & Co | WIPO |
| EP2810952A1 | 10.12.2014 | Novel pest control methods | Fraunhofer IME, Giessen; Justus-Liebig-University of Giessen, Giessen | European Patent Office |

As mentioned above, advantageous embodiments of the present disclosure pertain to the use of RNA interference to silence the expression and/or the translation of HAT, in particular of p300/CBP to control a target pest, in particular to control aphids.

The above described (c) is a compound used for the inhibition of expression by an antisense method. The inhibition of expression using an antisense method is generally carried out using an antisense construct that produces a RNA complementary to the portion specific to the mRNA corresponding to the target gene upon transcription. The antisense construct (also referred to as antisense nucleic acid) is, for example, introduced into the target cell in the form of an expression plasmid. The antisense construct may be an oligonucleotide probe that hybridizes with the DNA sequence or corresponding mRNA sequence of the target gene (these sequences may be collectively referred to as "target nucleic acid") upon introduction into the target cell, and inhibits their expression. The oligonucleotide probe is preferably resistant to endogenous nucleases such as exonuclease and/or endonuclease. When a DNA molecule is used as an antisense nucleic acid, the DNA molecule is preferably an oligodeoxyribonucleotide derived from the region containing the translation initiation site of the mRNA corresponding to the target gene (for example, the region from -10 to +10).

The complementation between the antisense nucleic acid and target nucleic acid is preferably precise, but some mismatch may occur. The hybridization capacity of the antisense nucleic acid for the target nucleic acid generally depends on the degree of complementation between the nucleic acids and the length of the antisense nucleic acid. In principle, the longer the antisense nucleic acid, the more stable double strand (or triplex) is formed between the antisense and target nucleic acids, even if many mismatches occur. Those skilled in the art can examine the degree of acceptable mismatch using a standard method.

The antisense nucleic acid may be DNA, RNA, or a chimera mixture thereof, or a derivative or modified product thereof. The antisense nucleic acid may be single or double strand. The stability and hybridization capacity of the antisense nucleic acid is improved by the modification of the base, sugar, or phosphoric acid backbone. The antisense nucleic acid may be synthesized by an ordinary method using, for example, a commercially available automatic DNA synthesizing apparatus (for example, manufactured by Applied Biosystems). The preparation of the modified nucleic acid and derivatives may refer to, for example, Stein et al. (1988), Nucl. Acids Res. 16:3209 or Sarin et al., (1988), Proc. Natl. Acad. Sci. U.S.A. 85:7448-7451.

In order to improve the action of the antisense nucleic acid in the target cell, a promoter (for example, actin promoter or ie1 promoter) that strongly acts in the target cell may be used. More specifically, when a construct containing the antisense nucleic acid under control of the promoter is introduced into the target cell, a sufficient amount of antisense nucleic acid is transcribed.

According to one aspect of the present disclosure, the inhibition of expression by ribozyme is used (when the compound (d) is used). The mRNA corresponding to the target gene may be destroyed using a ribozyme that cleaves the mRNA at the site-specific recognition sequence, but preferably a hammerhead ribozyme is used. The method for constructing the hammerhead ribozyme may be referred to, for example, Haseloff and Gerlach, 1988, Nature, 334:585-591.

In the same manner as in the antisense method, for example, for the purpose of improving stability and target performance, the ribozyme construction may use a modified oligonucleotide. In order to produce an effective amount of ribozyme within the target cell, it is preferred that a nucleic acid construct including DNA coding the ribozyme be used under the control of a promoter which strongly acts in insect cells (for example, an actin promoter or an ie1 promoter).

### D. Nucleic Acid Compositions and Constructs

The present disclosure provides recombinant DNA constructs for use in achieving a stable transformation of particular host or symbiont pest targets. Transformed host or symbiont pest targets may express pesticidally effective levels of preferred dsRNA or siRNA molecules from the recombinant DNA constructs, and provide the molecules in the diet of the pest. Pairs of isolated and purified nucleotide sequences may be provided from cDNA library and/or genomic library information. The pairs of nucleotide sequences may be derived from any preferred hemipteran pest for use as thermal amplification primers to generate DNA templates for the preparation of dsRNA and siRNA molecules of the present disclosure.

Provided according to the present disclosure are nucleotide sequences, the expression of which results in an RNA sequence that is substantially homologous to an RNA molecule of a targeted gene in an insect that comprises an RNA sequence encoded by a nucleotide sequence within the genome of the insect. Thus, after incorporation (uptake) of the stabilized RNA sequence down-regulation of the nucleotide sequence of the target gene in the cells of the insect may be obtained resulting in a deleterious effect on the maintenance, viability, proliferation, reproduction and infestation of the insect.

Examples of isolated polynucleotide suitable as a pest control agent against a target pest are the following (A) to (d):
a) a polynucleotide comprising a nucleic acid sequence of SEQ ID NO:2;
b) a polynucleotide that hybridizes to a nucleic acid sequence of SEQ ID NO:2 under stringent conditions;
c) a polynucleotide of at least 70, at least 80, at least 85, at least 90 percent sequence identity, to a nucleic acid sequence of SEQ ID NO:2;
d) a fragment of at least 16 contiguous nucleotides of a nucleic acid sequence of SEQ ID NO:2; and
e) a complement of the sequence of (a), (b), (c) or (d),
wherein the uptake by a Hemiptera crop plant pest of a double stranded ribonucleotide sequence comprising at least one strand that is complementary to said polynucleotide or said fragment reduces at least the reproduction and/or survival of a target pest. In particular reduces/inhibits the reproduction, the survival and/or the development of a Hemiptera target pest.

Further advantageous embodiments of isolated polynucleotide suitable as a pest control agent against a target pest are isolated double stranded ribonucleotides (dsRNA) sequence having a survival and/or reproduction inhibitory effect on a Hemiptera pest and consisting of a nucleic acid sequence between 250 to 600 nucleic acids in length, in particular between 300 and 550 nucleic acids in length, in particular between 300 and 520 nucleic acids in length, wherein the dsRNA sequence comprises an nucleic acid sequence derived from SEQ ID NO. 1 or homologs thereof, wherein said homologs have a sequence identity of at least 80 %, in particular of at least 85%, in particular of at least 90% to SEQ ID NO: 1, and wherein the derived dsRNA is coded by a continuous part of SEQ ID NO. 1 or said homologs thereof, and having a nucleic acid sequence between 300 to 520 nucleic acids in length and wherein the *Hemiptera* pest belonging to the suborder Sternorrhyncha, in particular belonging to the family of aphids like *Acyrthosiphon pisum.*

Further provided by the disclosure is a fragment or concatemer of a nucleic acid sequence of SEQ ID NO:1 or SEQ ID NO. 9. The fragment may be defined as causing the death, inhibition, stunting, or development of a pest when expressed as a dsRNA or siRNA and provided to the pest. The fragment may, for example, comprise at least about 16, 17, 18, 19, 21, 23, 25, 40, 60, 80, 100, 125 or more contiguous nucleotides of the sequence set force in SEQ ID NO:1 or SEQ ID NO. 9, or a complement thereof. One beneficial DNA segment for use in the present disclosure is at least from about 19 to about 23, or about 23 to about 100 nucleotides up to about 2000 nucleotides or more in length. Particularly useful will be dsRNA sequences including about 23 to about 300 nucleotides homologous to a pest target sequence. The disclosure also provides a ribonucleic acid expressed from any of such sequences including a dsRNA. A sequence selected for use in expression of a gene suppression agent can be constructed from a single sequence derived from one or more target pests and intended for use in expression of an RNA that functions in the suppression of a single gene or gene family in the one or more target pests, or that the DNA sequence can be constructed as a chimera from a plurality of DNA sequences.

In further embodiments, the disclosure pertains to recombinant DNA constructs comprising a nucleic acid molecule encoding a dsRNA molecule described herein. The dsRNA may be formed by transcription of one strand of the dsRNA molecule from a nucleotide sequence which is at least from about 80% to about 100% identical to a nucleotide sequence comprising SEQ ID NO:1. Such recombinant DNA constructs may be defined as producing dsRNA molecules capable of inhibiting the expression of endogenous target gene(s) in a pest cell upon ingestion. The construct may comprise a nucleotide sequence of the plant operably linked to a promoter sequence that functions in the host cell. Such a promoter may be tissue-specific and may, for example, be specific to a tissue type which is the subject of pest attack.

Nucleic acid constructs in accordance with the disclosure may comprise at least one non-naturally occurring nucleotide sequence that can be transcribed into a single stranded RNA capable of forming a dsRNA molecule *in vivo* through hybridization. Such dsRNA sequences self-assemble and can be provided in the diet of a hemipteran pest to achieve the desired inhibition.

A recombinant DNA construct may comprise two different non-naturally occurring sequences which, when expressed *in vivo* as dsRNA sequences and provided in the diet of a hemipteran pest, inhibit the expression of at least two different target genes in the cell of the hemipteran pest. In certain embodiments, at least 3, 4, 5, 6, 8 or 10 or more different dsRNAs are produced in a cell or plant comprising the cell that has a pest-inhibitory effect. The dsRNAs may be expressed from multiple constructs introduced in different transformation events or could be introduced on a single nucleic acid molecule. The dsRNAs may be expressed using a single promoter or multiple promoters. In one embodiments of the disclosure, single dsRNAs are produced that comprise nucleic acids homologous to multiple loci within a pest. In still yet another aspect, the disclosure provides a recombinant host cell having in its genome at least one recombinant DNA sequence that is transcribed to produce at least one dsRNA molecule that functions when ingested by a hemipteran pest to inhibit the expression of a target gene in the pest. The dsRNA molecule may be encoded by any of the nucleic acids described herein and as set forth in the sequence listing. The present disclosure also provides a transformed plant cell having in its genome at least one recombinant DNA sequence described herein. Transgenic plants comprising such a transformed plant cell are also provided, including progeny plants of any generation, seeds, and plant products, each comprising the recombinant DNA.

The present disclosure provides DNA sequences capable of being expressed as a RNA in a cell or microorganism to inhibit target gene expression in a cell, tissue or organ of an insect. The sequences comprise a DNA molecule coding for one or more different nucleotide sequences, wherein each of the different nucleotide sequences comprises a sense nucleotide sequence and an antisense nucleotide sequence connected by a spacer sequence coding for a dsRNA molecule of the present disclosure. The spacer sequence constitutes part of the sense nucleotide sequence or the antisense nucleotide sequence and forms within the dsRNA molecule between the sense and antisense sequences. The sense nucleotide sequence or the antisense nucleotide sequence is substantially identical to the nucleotide sequence of the target gene or a derivative thereof or a complementary sequence thereto. The dsDNA molecule may be placed operably under the control of a promoter sequence that functions in the cell, tissue or organ of the host expressing the dsDNA to produce dsRNA molecules. In one embodiment, the DNA sequence may be derived from a nucleotide sequence of SEQ ID NO:1.

As mentioned above, the present disclosure also provides a DNA sequence for expression in a cell of a plant that, upon expression of the DNA to RNA and ingestion by a target pest achieves suppression of a target gene in a cell, tissue or organ of an insect pest. The dsRNA at least comprises one or multiple structural gene sequences, wherein each of the structural gene sequences comprises a sense nucleotide sequence and an antisense nucleotide sequence connected by a spacer sequence that forms a loop within the complementary and antisense sequences. The sense nucleotide sequence or the antisense nucleotide sequence is substantially identical to the nucleotide sequence of the target gene, a derivative thereof, or sequence complementary thereto. The one or more structural gene sequences is placed operably under the control of one or more promoter sequences, at least one of which is operable in the cell, tissue or organ of a prokaryotic or eukaryotic organism, particularly a plant.

A gene sequence or fragment for pest control according to the present disclosure may be cloned between two tissue-specific promoters, such as two root-specific promoters which are operable in a transgenic plant cell and therein expressed to produce dsRNA in the transgenic plant cell. The dsRNA molecules contained in plant tissues are ingested by an insect so that the intended suppression of the target gene expression is achieved.

A nucleotide sequence provided by the present disclosure may comprise an inverted repeat separated by a "spacer sequence." The spacer sequence may be a region comprising any sequence of nucleotides that facilitates secondary structure formation between each repeat, where this is required. In one embodiment of the present disclosure, the spacer sequence is part of the sense or antisense coding sequence for mRNA. The spacer sequence may alternatively comprise any combination of nucleotides or homologs thereof that are capable of being linked covalently to a nucleic acid molecule. The spacer sequence may comprise a sequence of nucleotides of at least about 10-100 nucleotides in length, or alternatively at least about 100-200 nucleotides in length, at least 200-400 about nucleotides in length, or at least about 400-500 nucleotides in length.

The nucleic acid molecules or fragment of the nucleic acid molecules or other nucleic acid molecules in the sequence listing are capable of specifically hybridizing to other nucleic acid molecules under certain circumstances. As used herein, two nucleic acid molecules are said to be capable of specifically hybridizing to one another if the two molecules are capable of forming an anti-parallel, double-stranded nucleic acid structure. A nucleic acid molecule is said to be the complement of another nucleic acid molecule if they exhibit complete complementarity. Two molecules are said to be "minimally complementary" if they can hybridize to one another with sufficient stability to permit them to remain annealed to one another under at least conventional "low-stringency" conditions. Similarly, the molecules are said to be complementary if they can hybridize to one another with sufficient stability to permit them to remain annealed to one another under conventional "high-stringency" conditions. Conventional stringency conditions are described by Sambrook, et al. (1989), and by Haymes et al. (1985).

Departures from complete complementarity are therefore permissible, as long as such departures do not completely preclude the capacity of the molecules to form a double stranded structure. Thus, in order for a nucleic acid molecule or a fragment of the nucleic acid molecule to serve as a primer or probe it needs only be sufficiently complementary in sequence to be able to form a stable double-stranded structure under the particular solvent and salt concentrations employed.

Appropriate stringency conditions which promote DNA hybridization are, for example, 6.0 x sodium chloride/sodium citrate (SSC) at about 45 °C, followed by a wash of 2.0 x SSC at 50 °C, are known to those skilled in the art or can be found in Current Protocols in Molecular Biology (1989). For example, the salt concentration in the wash step can be selected from a low stringency of about 2.0 x SSC at 50 °C to a high stringency of about 0.2 x SSC at 50 °C. In addition, the temperature in the wash step can be increased from low stringency conditions at room temperature, about 22°C, to high stringency conditions at about 65 °C. Both temperature and salt may be varied, or either the temperature or the salt concentration may be held constant while the other variable is changed. A nucleic acid for use in the present disclosure may specifically hybridize to one or more of nucleic acid molecules from aphids, in particular from *Acyrthosiphon pisum* or complements thereof under such conditions. Preferably, a nucleic acid for use in the present disclosure will exhibit at least from about 85%, or at least from about 90%, or at least from about 95%, or at least from about 98% or even about 100% sequence identity with a nucleic acid molecule of SEQ ID NO:1.

Nucleic acids of the present disclosure may also be synthesized, either completely or in part, especially where it is desirable to provide plant-preferred sequences, by methods known in the art. Thus, all or a portion of the nucleic acids of the present disclosure may be synthesized using codons preferred by a selected host. Species-preferred codons may be determined, for example, from the codons used most frequently in the proteins expressed in a particular host species. Other modifications of the nucleotide sequences may result in mutants having slightly altered activity. dsRNA or siRNA nucleotide sequences comprise double strands of polymerized ribonucleotide and may include modifications to either the phosphate-sugar backbone or the nucleoside. Modifications in RNA structure may be tailored to allow specific genetic inhibition. In one embodiment, the dsRNA molecules may be modified through an enzymatic process so that siRNA molecules may be generated. The siRNA can efficiently mediate the down-regulation effect for some target genes in some insects. This enzymatic process may be accomplished by utilizing an RNAse III enzyme or a DICER enzyme, present in the cells of an insect, a vertebrate animal, a fungus or a plant in the eukaryotic RNAi pathway (Elbashir et al, 2002; Hamilton and Baulcombe, 1999). This process may also utilize a recombinant DICER or RNAse III introduced into the cells of a target insect through recombinant DNA techniques that are readily known to the skilled in the art. Both the DICER enzyme and RNAse III, being naturally occurring in an insect or being made through recombinant DNA techniques, cleave larger dsRNA strands into smaller oligonucleotides. The DICER enzymes specifically cut the dsRNA molecules into siRNA pieces each of which is about 19-25 nucleotides in length while the RNAse III enzymes normally cleave the dsRNA molecules into 12-15 base-pair siRNA. The siRNA molecules produced by the either of the enzymes have 2 to 3 nucleotide 3' overhangs, and 5' phosphate and 3' hydroxyl termini. The siRNA molecules generated by RNAse III enzyme are the same as those produced by Dicer enzymes in the eukaryotic RNAi pathway and are hence then targeted and degraded by an inherent cellular RNA-degrading mechanism after they are subsequently unwound, separated into single-stranded RNA and hybridize with the RNA sequences transcribed by the target gene. This process results in the effective degradation or removal of the RNA sequence encoded by the nucleotide sequence of the target gene in the insect. The outcome is the silencing of a particularly targeted nucleotide sequence within the insect. Detailed descriptions of enzymatic processes can be found in Harmon (2002).

In some embodiments, the present disclosure pertains to double stranded ribonucleotide sequences produced from the expression of a polynucleotide according to the present disclosure, wherein the ingestion of said ribonucleotide sequence or fragments thereof as RNAi inducing compounds by a Hemiptera crop plant pest reduces survival and/or reproduction of said pest. In an advantageous embodiment, said double stranded ribonucleotide sequence comprises a nucleic acid sequence of SEQ ID NO:2, or homologs thereof, wherein said homologs have a sequence identity of at least 80 %, in particular of at least 85%, in particular of at least 90%, in particular of at least 95% to SEQ ID NO: 2.

### E. Incorporation of HAT Inhibitor

The manner for the incorporation of the HAT inhibitor is not particularly limited and may be selected according to the target pest. When the target pest is a pest that attacks a plant, for example, the agent (pesticide) containing the HAT inhibitor is in advance retained in the plant, which is to be attacked by the target pest, through application, spraying, or atomization. As a result of this, when the target pest ingests the plant, the HAT inhibitor is incorporated into the body of the target pest. The incorporation of the HAT inhibitor may be done by topical application, uptake through respiratory system etc.

The term "incorporating" or "incorporating an inhibitor" includes any method by which a pest may uptake or come in contact with an inhibitor, e.g a dsRNA, wherein e.g. the dsRNA comprises annealed complementary strands, one of which has a nucleotide sequence which is complementary to at least part of the nucleotide sequence of a pest target gene to be down-regulated. A pest may be exposed to the inhibitor, in particular a dsRNA by direct uptake (e.g. by feeding), which does not require expression of dsRNA within the pest. Alternatively, a pest may come into direct contact with a composition comprising the dsRNA. For example, a pest may come into contact with a surface or material treated with a composition comprising a dsRNA. A dsRNA may be expressed by a prokaryotic (for instance, but not limited to, a bacterial) or eukaryotic (for instance, but not limited to, a yeast) host cell or host organism like a microorganism. In particular, the target pest may be contacted with the HAT inhibitor by topical application, uptake through respiratory system etc..

In particular, the HAT inhibitor may be a coating or a powder that can be applied to a substrate as a means for protecting the substrate from infestation by an insect and thereby preventing pest-induced damage to the substrate or material. Thus, in one embodiment, the composition is in the form of a coating on a suitable surface which adheres to, and is eventually ingested by an insect which comes into contact with the coating. Such a composition can be used to protect any substrate or material that is susceptible to infestation by or damage caused by a pest, for example foodstuffs and other perishable materials, and substrates such as wood.

For example, the HAT inhibitor may be comprised in a liquid that is brushed or sprayed on the target pest. In particular, the inhibitor is incorporated by delivering the inhibitor via ingestion, application, spraying and/or atomization of the inhibitor on the target pest.

In one embodiment of the method according to the present disclosure the HAT-Inhibitor, in particular p300/CBP is introduced into the target pest by contacting the target pest with the inhibitor.

On the other hand, when a feed (feed agent) containing the HAT inhibitor is placed at the site of occurrence or in the route of entry of the target pest, the target pest ingests the feed, and thus the HAT inhibitor is incorporated into the body of the target pest. In addition, when the plant to be attacked is modified by the introduction of a gene coding the HAT inhibitor, the HAT inhibitor is incorporated into the body of the target pest when the pest ingests the transgenic plant. The transgenic plant used in this method may be a plant subjected to gene modification so as to express: (A) a siRNA targeted at a gene coding the HAT of the target pest; (B) an antisense nucleic acid targeted at the transcript product of a gene coding the HAT of the target pest; or (C) a ribozyme targeted at the transcript product of a gene coding the HAT of the target pest.

Therefore, in some embodiments, the pest control method according to the present disclosure comprise making a plant, which is to be attacked by the target pest, possess an agent containing the inhibitor by application, spraying, or atomization in advance, and incorporating the inhibitor into the body of the target pest by ingestion of the plant.

However, in some advantageous embodiments, the pest control method according to the present disclosure comprises incorporating the inhibitor into the body of the target pest by ingestion of a transgenic plant containing a gene encoding the inhibitor.

Therefore, in order to circumvent challenges associated with the regulation of GM crops, non-transgenic RNAi strategies have been evaluated for pest control with encouraging results (Joga et al., 2016). These strategies include:
1) dsRNA uptake by plant roots following soil irrigation (Joga et al., 2016);
2) Trunk delivery by plant cuttings or injection into the stems of woody plants (Joga et al., 2016, Cagliari et al., 2018);
3) RNAi-based seed treatment (Cagliari et al., 2018);
4) dsRNA sprays that can be applied directly as a bioinsecticide (Cagliari et al., 2018).

In comparison to chemical insecticides, dsRNA sprays offer an environmentally friendly alternative given the short half-life of nucleic acids in the environment (Zotti et al., 2018). Pests such as the Colorado potato beetle (*Leptinotarsa decemlineata*) and diaprepes root weevil (*Diaprepes abbreviatus*) can be controlled by the foliar application of dsRNA.

Several companies have investigated cost-efficient methods for the production of dsRNA (Cagliari et al., 2018) and the cost to produce 1 g of dsRNA (100-800 bp) has dropped from $12,500 USD in 2008 to less than $60 USD in 2018 (http://www.agrorna.com/sub_05.html). However, naked dsRNA (as sold, for example, by the company agroRNA) cannot be applied in the field because it is degraded by RNases, UV light and other environmental factors. It must therefore be formulated as a ready-to-spray product (Cagliari et al., 2018). This formulation not only has to protect the dsRNA but must also facilitate its transfer across the cell membrane to reach the target tissue and ensure its interaction with the components of RNAi machinery (Heidebrecht Jr, 2017). The reduction in target mRNA levels triggered by RNAi must also be sufficient to achieve the required phenotype (Heidebrecht Jr, 2017).

The following technologies have been evaluated as formulations that promote the transfer of dsRNA into target tissues (Zhou et al., 2013, Mitter et al., 2017):
1) Nanoparticles in which dsRNA is encapsulated by lipids (liposomes) or cyclodextrin;
2) dsRNA complexes with chitosan;
3) dsRNA loaded on layered double hydroxide clay (BioClay) nanosheets;
4) Dendrimer dsRNA nanoparticles;
5) Complexes or chimeras in which siRNA is associated with antibodies, aptamers or peptides.

The company RNAagri (https://www.rnagri.com/) has developed a non-transgenic technology called Apse RNA Containers that allow the production of encapsulated ready-to-spray dsRNA with costs near $1 USD per 1 g (Cagliari et al., 2018). The technology is based on plasmids engineered to produce naturally occurring proteins such as capsids, which are co-expressed with the plasmid carrying the dsRNA, the latter including a packing site that promotes the self-assembly of dsRNA nanoparticles, thus protecting the dsRNA from the environment (Cagliari et al., 2018).

### E. Vectors and Host Cell Transformation

As mentioned above, the present disclosure contemplates the transformation of a nucleotide sequence of the present disclosure into a plant to achieve pest inhibitory levels of expression of one or more dsRNA molecules. A transformation vector can be readily prepared using methods available in the art. The transformation vector comprises one or more nucleotide sequences that is/are capable of being transcribed to an RNA molecule and that is/are substantially homologous and/or complementary to one or more nucleotide sequences encoded by the genome of the insect, such that upon uptake of the RNA there is down-regulation of expression of at least one of the respective nucleotide sequences of the genome of the insect.

The transformation vector may be termed a dsDNA construct and may also be defined as a recombinant molecule, an insect control agent, a genetic molecule or a chimeric genetic construct. A chimeric genetic construct of the present disclosure may comprise, for example, nucleotide sequences encoding one or more antisense transcripts, one or more sense transcripts, one or more of each of the aforementioned, wherein all or part of a transcript therefrom is homologous to all or part of an RNA molecule comprising an RNA sequence encoded by a nucleotide sequence within the genome of an insect.

In one embodiment, the disclosure transformation vector comprises an isolated and purified DNA molecule comprising a promoter operatively linked to one or more nucleotide sequences of the present disclosure. The nucleotide sequence is for example SEQ ID NO:1, SEQ ID NO:2, SEQ ID NO:9 or parts thereof. The nucleotide sequence includes a segment coding all or part of a RNA present within a targeted pest RNA transcript and may comprise inverted repeats of all or a part of a targeted pest RNA. The DNA molecule comprising the expression vector may also contain a functional intron sequence positioned either upstream of the coding sequence or even within the coding sequence, and may also contain a five prime (5') untranslated leader sequence (i.e., a UTR or 5'-UTR) positioned between the promoter and the point of translation initiation.

A plant transformation vector may contain sequences from more than one gene, thus allowing the production of more than one dsRNA for inhibiting expression of two or more genes in cells of a target pest. One skilled in the art will readily appreciate that segments of DNA whose sequence corresponds to that present in different genes can be combined into a single composite DNA segment for expression in a transgenic plant. Alternatively, a plasmid of the present disclosure already containing at least one DNA segment can be modified by the sequential insertion of additional DNA segments between the enhancer and promoter and terminator sequences. In the insect control agent of the present disclosure designed for the inhibition of multiple genes, the genes to be inhibited can be obtained from the same insect species in order to enhance the effectiveness of the insect control agent. In certain embodiments, the genes can be derived from different insects in order to broaden the range of insects against which the agent is effective. When multiple genes are targeted for suppression or a combination of expression and suppression, a polycistronic DNA element can be fabricated as illustrated and disclosed in Fillatti, Application Publication No. US 2004-0029283.

Promoters that function in different plant species are also well known in the art. Promoters useful for expression of polypeptides in plants include those that are inducible, viral, synthetic, or constitutive as described in Odell et al. (1985), and/or promoters that are temporally regulated, spatially regulated, and spatio-temporally regulated. Preferred promoters include the enhanced CaMV35S promoters, the SUC2 promoter and the FMV35S promoter. For the purpose of the present disclosure, e.g., for optimum control of species that feed on the phloem via their stylet, it may be preferable to achieve the highest levels of expression of these genes within the phloems of plants. Therefore, in an advantageous embodiment the promoter is active in the phloem of a crop plant like the CaMV 35S promoter (Yang and Christou, 1990) and the SUC2 promoter (Truermit and Sauer, 1995). dsRNA expression control by the CaMV 35S promoter was used by Pitino et al. (2011) that demonstrated host induced gene silencing (HIGS) in aphids.

The phloem located expression of target-specific dsRNA or siRNA in genetically modified plants that target HAT allows most likely the reduction of infestation of crop plants by aphids and other plant-sucking insects of the groups Sternorryhncha under the critical economic threshold, which is declared aim of IPS. In this context varying length of dsRNA and siRNA are possible that cover different regions of HAT mRNA.

A recombinant DNA vector or construct of the present disclosure will typically comprise a selectable marker that confers a selectable phenotype on plant cells. Selectable markers may also be used to select for plants or plant cells that contain the exogenous nucleic acids encoding polypeptides or proteins of the present disclosure. The marker may encode biocide resistance, antibiotic resistance (e.g., kanamycin, G418 bleomycin, hygromycin, etc.), or herbicide resistance (e.g., glyphosate, etc.). Examples of selectable markers include, but are not limited to, a neo gene which codes for kanamycin resistance and can be selected for using kanamycin, G418, etc., a bar gene which codes for bialaphos resistance; a mutant EPSP synthase gene which encodes glyphosate resistance; a nitrilase gene which confers resistance to bromoxynil; a mutant acetolactate synthase gene (ALS) which confers imidazolinone or sulfonylurea resistance; and a methotrexate resistant DHFR gene. Examples of such selectable markers are illustrated in U.S. Patents 5,550,318; 5,633,435; 5,780,708 and 6,118,047.

A recombinant vector or construct of the present disclosure may also include a screenable marker. Screenable markers may be used to monitor expression. Exemplary screenable markers include a [beta]-glucuronidase or uidA gene (GUS) which encodes an enzyme for which various chromogenic substrates are known (Jefferson, 1987; Jefferson et al, 1987); an R-locus gene, which encodes a product that regulates the production of anthocyanin pigments (red color) in plant tissues (Dellaporta et al, 1988); a [beta]-lactamase gene (Sutcliffe et al, 1978), a gene which encodes an enzyme for which various chromogenic substrates are known (e.g., PADAC, a chromogenic cephalosporin); a luciferase gene (Ow et al, 1986) a xylE gene (Zukowsky et al, 1983) which encodes a catechol dioxygenase that can convert chromogenic catechols; an [alpha]-amylase gene (Bcatu et al, 1990); a tyrosinase gene (Katz et al, 1983) which encodes an enzyme capable of oxidizing tyrosine to DOPA and dopaquinone which in turn condenses to melanin.

In some advantageous embodiments, the isolated polynucleotides according to the present disclosure are operably linked to a heterologous promoter and/or are defined as comprised of a plant transformation vector.

Preferred plant transformation vectors include those derived from a Ti plasmid of *Agrobacterium tumefaciens* (e.g. U.S. Patent Nos. 4,536,475, 4,693,977, 4,886,937, 5,501,967 and EP 0 122 791). *Agrobacterium rhizogenes* plasmids (or "Ri") are also useful and known in the art. Other preferred plant transformation vectors include those disclosed, e.g., by Herrera-Estrella (1983); Bevan (1983), Klee (1985) and EP 0 120 516. In an advantageous embodiment, the vector is a binary vector.

In general, it is preferred to introduce a functional recombinant DNA at a non-specific location in a plant genome. In special cases it may be useful to insert a recombinant DNA construct by site-specific integration. Several site-specific recombination systems exist which are known to function implants include cre-lox as disclosed in U.S. Patent 4,959,317 and FLP-FRT as disclosed in U.S. Patent 5,527,695.

Suitable methods for transformation of host cells for use with the current plant are believed to include virtually any method by which DNA can be introduced into a cell, such as by direct delivery of DNA such as by PEG-mediated transformation of protoplasts (Omirulleh et al, 1993), by desiccation/inhibition-mediated DNA uptake (Potrykus et al, 1985), by electroporation (U.S. Patent No. 5,384,253), by agitation with silicon carbide fibers (Kaeppler et al, 1990; U.S. Patent No. 5,302,523; and U.S. Patent No. 5,464,765), by Agrobacterium-mediated transformation (U.S. Patent No. 5,591,616 and U.S. Patent No. 5,563,055) and by acceleration of DNA coated particles (U.S. Patent No. 5,550,318; U.S. Patent No. 5,538,877; and U.S. Patent No. 5,538,880), etc. Through the application of techniques such as these, the cells of virtually any species may be stably transformed. In the case of multicellular species, the transgenic cells may be regenerated into transgenic organisms. Methods for the creation of transgenic plants and expression of heterologous nucleic acids in plants in particular are known and may be used with the nucleic acids provided herein to prepare transgenic plants that exhibit reduced susceptibility to feeding by a target pest organism such as aphids. Plant transformation vectors can be prepared, for example, by inserting the dsRNA producing nucleic acids disclosed herein into plant transformation vectors and introducing these into plants. One known vector system has been derived by modifying the natural gene transfer system of *Agrobacterium tumefaciens.* The natural system comprises large Ti (tumor-inducing)-plasmids containing a large segment, known as T-DNA, which is transferred to transformed plants. Another segment of the Ti plasmid, the vir region, is responsible for T-DNA transfer. The T-DNA region is bordered by terminal repeats, the modified binary vectors the tumor-inducing genes have been deleted and the functions of the vir region are utilized to transfer foreign DNA bordered by the T-DNA border sequences. The T-region may also contain a selectable marker for efficient recovery of transgenic plants and cells, and a multiple cloning site for inserting sequences for transfer such as a dsRNA encoding nucleic acid.

A transgenic plant formed using *Agrobacterium* transformation methods typically contains a single simple recombinant DNA sequence inserted into one chromosome and is referred to as a transgenic event. Such transgenic plants can be referred to as being heterozygous for the inserted exogenous sequence. A homozygous transgenic plant can be obtained by sexually mating (selfmating) an independent segregant transgenic plant to produce Fl seed. One-fourth of the Fl seed produced will be homozygous with respect to the transgene. Germinating Fl seed results in plants that can be tested for heterozygosity or homozygosity, typically using a SNP assay or a thermal amplification assay that allows for the distinction between heterozygotes and homozygotes.

The methods and compositions of the present disclosure may be applied to any monocot and dicot plant, depending on the hemipteran pest control desired. Specifically, the plants are intended to include, without limitation, alfalfa, aneth, apple, apricot, artichoke, arugula, asparagus, avocado, banana, barley, beans, beet, blackberry, blueberry, broccoli, brussel sprouts, cabbage, canola, cantaloupe, carrot, cassava, cauliflower, celery, cherry, cilantro, citrus, Clementine, coffee, corn, cotton, cucumber, Douglas fir, eggplant, endive, escarole, eucalyptus, fennel, figs, gourd, grape, grapefruit, honey dew, jicama, kiwifruit, lettuce, leeks, lemon, lime, Loblolly pine, mango, melon, mushroom, nut, oat, okra, onion, orange, an ornamental plant, papaya, parsley, pea, peach, peanut, pear, pepper, persimmon, pine, pineapple, plantain, plum, pomegranate, poplar, potato, pumpkin, quince, radiata pine, radicchio, radish, raspberry, rice, rye, sorghum, Southern pine, soybean, spinach, squash, strawberry, sugarbeet, sugarcane, sunflower, sweet potato, sweetgum, tangerine, tea, tobacco, tomato, turf, a vine, watermelon, wheat, yams, and zucchini plants. Thus, a plant transformed with a recombinant DNA sequence of SEQ ID NO:1, SEQ ID NO:2 or SEQ ID NO:9, or concatemer, fragment, or complement thereof, that is transcribed to produce at least one dsRNA molecule that functions when ingested by a hemipteran pest to inhibit the expression of a target gene in the pest is also provided by the plant. In particular embodiments, the recombinant DNA sequence is SEQ ID NO:2, or fragments, complements, or concatemers thereof.

However, the polynucleotide according to the present disclosure may be transformed, transduced or transfected via a recombinant DNA vector also in a prokaryotic cell or eukaryotic cell, for example for production of an agent (pesticide) containing the HAT inhibitor.

A recombinant DNA vector may, for example, be a linear or a closed circular plasmid. The vector system may be a single vector or plasmid or two or more vectors or plasmids that together contain the total DNA to be introduced into the genome of the bacterial host. In addition, a bacterial vector may be an expression vector. The nucleic acid molecules according to the present disclosure can, for example, be suitably inserted into a vector under the control of a suitable promoter that functions in one or more microbial hosts to drive expression of a linked coding sequence or other DNA sequence. Many vectors are available for this purpose, and the selection of the appropriate vector will depend mainly on the size of the nucleic acid to be inserted into the vector and the particular host cell to be transformed with the vector. Each vector contains various components depending on its function (amplification of DNA or expression of DNA) and the particular host cell with which it is compatible. The vector components for bacterial transformation generally include but are not limited to, one or more of the following: a signal sequence, an origin of replication, one or more selectable marker genes, and an inducible promoter allowing the expression of exogenous DNA.

### F. Target Gene Suppression

The present disclosure provides, as an example, a transformed host or symbiont pest target organism, transformed plant cells and transformed plants and their progeny. The transformed plant cells and transformed plants may be engineered to express one or more of the dsRNA or siRNA sequences described herein to provide a pest-protective effect. As used herein the words "gene suppression" are intended to refer to any of the well-known methods for reducing the levels of gene transcription to mRNA and/or subsequent translation of the mRNA.

Gene suppression is also intended to mean the reduction of protein expression from a gene or a coding sequence including posttranscriptional gene suppression and transcriptional suppression. Posttranscriptional gene suppression is mediated by the homology between of all or a part of an mRNA transcribed from a gene or coding sequence targeted for suppression and the corresponding double stranded RNA used for suppression and refers to the substantial and measurable reduction of the amount of available mRNA available in the cell for binding by ribosomes. The transcribed RNA can be in the sense orientation to effect what is called co-suppression, in the anti-sense orientation to effect what is called anti-sense suppression, or in both orientations producing a dsRNA to effect what is called RNA interference (RNAi).

Transcriptional suppression is mediated by the presence in the cell of a dsRNA gene suppression agent exhibiting substantial sequence identity to a promoter DNA sequence or the complement thereof to effect what is referred to as promoter trans suppression. Gene suppression may be effective against a native plant gene associated with a trait, e.g., to provide plants with reduced levels of a protein encoded by the native gene or with enhanced or reduced levels of an affected metabolite. Gene suppression can also be effective against target genes in plant pests that may ingest or contact plant material containing gene suppression agents, specifically designed to inhibit or suppress the expression of one or more homologous or complementary sequences in the cells of the pest. Post-transcriptional gene suppression by anti-sense or sense oriented RNA to regulate gene expression in plant cells is disclosed in U.S. Pat. Nos. 5,107,065, 5,759,829, 5,283,184, and 5,231,020. The use of dsRNA to suppress genes in plants is disclosed in WO 99/53050, WO 99/49029, U.S. Patent Application Publication No. 2003/0175965, and 2003/0061626, U.S. Patent Application No.10/465,800, and U.S. Patent Nos. 6,506,559, and 6,326,193.

A beneficial method of post transcriptional gene suppression in plants employs both sense-oriented and anti-sense-oriented, transcribed RNA which is stabilized, e.g., as a hairpin and stem and loop structure. A preferred DNA construct for effecting post transcriptional gene suppression is one in which a first segment encodes an RNA exhibiting an anti-sense orientation exhibiting substantial identity to a segment of a gene targeted for suppression, which is linked to a second segment in sense orientation encoding an RNA exhibiting substantial complementarity to the first segment. Such a construct forms a stem and loop structure by hybridization of the first segment with the second segment and a loop structure from the nucleotide sequences linking the two segments (see WO94/01550, WO98/05770, US 2002/0048814, and US 2003/0018993).

According to one embodiment of the present disclosure, there is provided a nucleotide sequence, for which *in vitro* expression results in transcription of a stabilized RNA sequence that is substantially homologous to an RNA molecule of a targeted gene in an insect that comprises an RNA sequence encoded by a nucleotide sequence within the genome of the insect. Thus, after the insect uptake (incorporation) of the stabilized RNA sequence incorporated in a diet or sprayed on a plant surface, a down-regulation of the nucleotide sequence corresponding to the target gene in the cells of a target insect is affected.

Inhibition of the HAT target gene using the stabilized dsRNA technology of the present disclosure is sequence-specific in that nucleotide sequences corresponding to the duplex region of the RNA are targeted for genetic inhibition. RNA containing nucleotide sequences identical to a portion of the target gene is preferred for inhibition. RNA sequences with insertions, deletions, and single point mutations relative to the target sequence have also been found to be effective for inhibition. In performance of the present disclosure, it is preferred that the inhibitory dsRNA and the portion of the target gene share at least from about 80% sequence identity, or from about 90% sequence identity, or from about 95% sequence identity, or from about 99% sequence identity, or even about 100% sequence identity. Alternatively, the duplex region of the RNA may be defined functionally as a nucleotide sequence that is capable of hybridizing with a portion of the target gene transcript. The length of the identical nucleotide sequences may be at least about 25, 50, 100, 200, 300, 400, 500 or at least about 1000 bases. Normally, a sequence of greater than 20-100 nucleotides should be used, though a sequence of greater than about 200-300 nucleotides would be preferred, and a sequence of greater than about 500-1000 nucleotides would be especially preferred depending on the size of the target gene. The plant has the advantage of being able to tolerate sequence variations that might be expected due to genetic mutation, strain polymorphism, or evolutionary divergence. The introduced nucleic acid molecule may not need to be absolute homology, may not need to be full length, relative to either the primary transcription product or fully processed mRNA of the target gene. Therefore, those skilled in the art need to realize that, as disclosed herein, 100% sequence identity between the RNA and the target gene is not required to practice the present disclosure. Inhibition of target gene expression may be quantified by measuring either the endogenous target RNA or the protein produced by translation of the target RNA and the consequences of inhibition can be confirmed by examination of the outward properties of the cell or organism. Techniques for quantifying RNA and proteins are well known to one of ordinary skill in the art. Multiple selectable markers are available that confer resistance to ampicillin, bleomycin, chloramphenicol, gentamycin, hygromycin, kanamycin, lincomycin, methotrexate, phosphinothricin, puromycin, spectinomycin, rifampicin, and tetracycline, and the like. In certain embodiments, gene expression is inhibited by at least 10%, preferably by at least 33%, more preferably by at least 50%, and yet more preferably by at least 80%. In particularly preferred embodiments of the plant gene expression and/or the translation is inhibited by at least 80%, more preferably by at least 90%, more preferably by at least 95%, or by at least 99% within cells in the insect so a significant inhibition takes place. Significant inhibition is intended to refer to sufficient inhibition that results in a detectable phenotype (e.g., cessation of larval growth, paralysis or mortality, etc.) or a detectable decrease in RNA and/or protein corresponding to the target gene being inhibited. Although in certain embodiments of the disclosure inhibition occurs in substantially all cells of the insect, in other preferred embodiments inhibition occurs in only a subset of cells expressing the gene. For example, if the gene to be inhibited plays an essential role in cells in the insect alimentary tract, inhibition of the gene within these cells is sufficient to exert a deleterious effect on the insect. dsRNA molecules may be synthesized either *in vivo* or *in vitro.* The dsRNA may be formed by a single self-complementary RNA strand or from two complementary RNA strands. Endogenous RNA polymerase of the cell may mediate transcription *in vivo,* or cloned RNA polymerase can be used for transcription *in vivo* or *in vitro.* Inhibition may be targeted by specific transcription in an organ, tissue, or cell type; stimulation of an environmental condition (e.g., infection, stress, temperature, chemical inducers); and/or engineering transcription at a developmental stage or age. The RNA strands may or may not be polyadenylated; the RNA strands may or may not be capable of being translated into a polypeptide by a cell's translational apparatus.

A RNA, dsRNA, siRNA, or miRNA of the present disclosure may be produced chemically or enzymatically by one skilled in the art through manual or automated reactions or in vivo in another organism. RNA may also be produced by partial or total organic synthesis; any modified ribonucleotide can be introduced by *in vitro* enzymatic or organic synthesis. The RNA may be synthesized by a cellular RNA polymerase or a bacteriophage RNA polymerase (e.g., T3, T7, SP6). The use and production of an expression construct are known in the art (see, for example, WO 97/32016; U.S. Pat. No's. 5,593, 874, 5,698,425, 5,712,135, 5,789,214, and 5,804,693). If synthesized chemically or by *in vitro* enzymatic synthesis, the RNA may be purified prior to introduction into the cell. For example, RNA can be purified from a mixture by extraction with a solvent or resin, precipitation, electrophoresis, chromatography, or a combination thereof. Alternatively, the RNA may be used with no or a minimum of purification to avoid losses due to sample processing. The RNA may be dried for storage or dissolved in an aqueous solution. The solution may contain buffers or salts to promote annealing, and/or stabilization of the duplex strands.

For transcription from a transgene *in vivo* or an expression construct, a regulatory region (e.g., promoter, enhancer, silencer, and polyadenylation) may be used to transcribe the RNA strand (or strands). Therefore, in one embodiment, the nucleotide sequences for use in producing RNA molecules may be operably linked to one or more promoter sequences functional in a microorganism, a fungus or a plant host cell. Ideally, the nucleotide sequences are placed under the control of an endogenous promoter, normally resident in the host genome. The nucleotide sequence of the present disclosure, under the control of an operably linked promoter sequence, may further be flanked by additional sequences that advantageously affect its transcription and/or the stability of a resulting transcript. Such sequences are generally located upstream of the operably linked promoter and/or downstream of the 3' end of the expression construct and may occur both upstream of the promoter and downstream of the 3' end of the expression construct, although such an upstream sequence only is also contemplated.

The present disclosure provides for inhibiting gene expression of one or multiple target genes in a target pest using stabilized dsRNA methods. The plant is particularly useful in the modulation of eukaryotic gene expression, in particular the modulation of expression of genes present in pests that exhibit a digestive system pH level that is from about 4.5 to about 9.5, more preferably from about 5.0 to about 8.0, and even more preferably from about 6.5 to about 7.5. For plant pests with a digestive system that exhibits pH levels outside of these ranges, delivery methods may be desired for uses that do not require the ingestion of dsRNA molecules.

The modulatory effect of dsRNA is applicable to a variety of genes expressed in the pests including, for example, endogenous genes responsible for cellular metabolism or cellular transformation, including house-keeping genes, transcription factors and other genes which encode polypeptides involved in cellular metabolism.

The present disclosure provides in part a delivery system for the delivery of the insect control agents to insects through their exposure to a diet containing the insect control agents of the present disclosure. In accordance with one of the embodiments, the stabilized dsRNA or siRNA molecules may be incorporated in the insect diet or may be overlaid on the top of the diet for consumption by an insect. The present disclosure also provides in part a delivery system for the delivery of the insect control agents to insects through their exposure to a microorganism or host such as a plant containing the insect control agents of the present disclosure by ingestion of the microorganism or the host cells or the contents of the cells. In accordance with another embodiment, the present disclosure involves generating a transgenic plant cell or a plant that contains a recombinant DNA construct transcribing the stabilized dsRNA molecules of the present disclosure. As used herein, the phrase "generating a transgenic plant cell or a plant" includes methods of employing the recombinant DNA technologies readily available in the art (e.g., by Sambrook, et al, 1989) to construct a plant transformation vector transcribing the stabilized dsRNA molecules of the present disclosure, to transform the plant cell or the plant and to generate the transgenic plant cell or the transgenic plant that contain the transcribed, stabilized dsRNA molecules.

In still another embodiment, non-pathogenic, attenuated strains of microorganisms may be used as a carrier for the insect control agents and, in this perspective, the microorganisms carrying such agents are also referred to as insect control agents. The microorganisms may be engineered to express a nucleotide sequence of a target gene to produce RNA molecules comprising RNA sequences homologous or complementary to RNA sequences typically found within the cells of an insect. Exposure of the insects to the microorganisms results in an ingestion of the microorganisms and down-regulation of expression of target genes mediated directly or indirectly by the RNA molecules or fragments or derivatives thereof.

The present disclosure alternatively provides exposure of an insect to the insect control agents of the present disclosure incorporated in a spray mixer and applied to the surface of a host, such as a host of the present disclosure. In an exemplary embodiment, ingestion of the insect control agents by an insect delivers the insect control agents to the gut of the insect and subsequently to the cells within the body of the insect. In another embodiment, infection of the insect by the insect control agents through other means such as by injection or other physical methods also permits the delivery of the insect control agents. In yet another embodiment, the RNA molecules themselves are encapsulated in a synthetic matrix such as a polymer and applied to the surface of a host such as a plant. Ingestion of the host cells by an insect permits the delivery of the insect control agents to the insect and results in down-regulation of a target gene in the host.

It is envisioned that the compositions of the present disclosure can be incorporated within the seeds of a plant species either as a product of expression from a recombinant gene incorporated into a genome of the plant cells, or incorporated into a coating or seed treatment that is applied to the seed before planting. The plant cell containing a recombinant gene is considered herein to be a transgenic event. It is believed that a pesticidal seed treatment can provide significant advantages when combined with a transgenic event that provides protection from hemipteran pest infestation that is within the preferred effectiveness range against a target pest. In addition, it is believed that there are situations that are well known to those having skill in the art, where it is advantageous to have such transgenic events within the preferred range of effectiveness.

The present disclosure provides in part a delivery system for the delivery of insect control agents to insects. The stabilized dsRNA or siRNA molecules of the present disclosure may be directly introduced into the cells of an insect or introduced into an extracellular cavity, interstitial space, lymph system, digestive system, into the circulation of the insect through oral ingestion or other means that one skilled in the art may employ. Methods for oral introduction may include direct mixing of RNA with food of the insect, as well as engineered approaches in which a species that is used as food is engineered to express the dsRNA or siRNA, then fed to the insect to be affected. In one embodiment, for example, the dsRNA or siRNA molecules may be incorporated into, or overlaid on the top of, the insect's diet. In another embodiment, the RNA may be sprayed onto a plant surface. In still another embodiment, the dsRNA or siRNA may be expressed by microorganisms and the microorganisms may be applied onto a plant surface or introduced into root, stem by a physical means such as an injection. In still another embodiment, a plant may be genetically engineered to express the dsRNA or siRNA in an amount sufficient to kill the insects known to infect the plant.

Specifically, in practicing the present disclosure in aphids, the stabilized dsRNA or siRNA may be introduced in the midgut inside the insect and achieve the desired inhibition of the targeted genes. The dsRNA or siRNA molecules may be incorporated into a diet or be overlaid on the diet as discussed above and may be ingested by the insects. In any event, the dsRNAs of the present disclosure are provided in the diet of the target pest. The target pest of the present disclosure will exhibit a digestive tract pH from about 4.5 to about 9.5, or from about 5 to about 8.5, or from about 6 to about 8, or from about 6.5 to about 7.7, or about 7.0. The digestive tract of a target pest is defined herein as the location within the pest that food that is ingested by the target pest is exposed to an environment that is favorable for the uptake of the dsRNA molecules of the present disclosure without suffering a pH so extreme that the hydrogen bonding between the double-strands of the dsRNA is caused to dissociate and form single stranded molecules.

It is also anticipated that dsRNAs produced by chemical or enzymatic synthesis may be formulated in a manner consistent with common agricultural practices and used as spray-on products for controlling insect infestations. The formulations may include the appropriate stickers and wetters required for efficient foliar coverage as well as UV protectants to protect dsRNAs from UV damage. Such additives are commonly used in the bio-insecticide industry and are well known to those skilled in the art. Such applications could be combined with other spray-on insecticide applications, biologically based or not, to enhance plant protection from insect feeding damage.

The present inventors contemplate that bacterial strain producing insecticidal proteins may be used to produce dsRNAs for insect control purposes. These strains may exhibit improved insect control properties. A variety of different bacterial hosts may be used to produce insect control dsRNAs. Exemplary bacteria may include *E. coli, B. thuringiensis, Pseudomonas sp., Photorhabdus sp., Xenorhabdus sp., Serratia entomophila* and related *Serratia sp., B. sphaericus, B. cereus, B. laterosporus, B. popilliae, Clostridium bifermentans* and other *Clostridium* species, or other spore-forming gram-positive bacteria. In certain embodiments, bacteria may be engineered for control of pests such as mosquitoes.

The present plant also relates to recombinant DNA constructs for expression in a microorganism. Exogenous nucleic acids from which a RNA of interest is transcribed can be introduced into a microbial host cell, such as a bacterial cell or a fungal cell, using methods known in the art.

The nucleotide sequences of the present disclosure may be introduced into a wide variety of prokaryotic and eukaryotic microorganism hosts to produce the stabilized dsRNA or siRNA molecules.

### F. Transgenic Plants

Another aspect of the present disclosure relates to a transgenic plant and seeds. A gene coding an inhibitor against the HAT of the target pest has been introduced into the transgenic plant of the present disclosure. Typically, the transgenic plant of the present disclosure has been subjected to gene modification so as to express: (A) a dsRNA molecule, wherein the dsRNA may be modified in the plant through an enzymatic process so that siRNA molecules may be generated targeting a transcript product of a gene coding the HAT of the target pest; (B) an antisense nucleic acid targeted at the transcript product of a gene coding the HAT of the target pest; or (C) a ribozyme targeted at the transcript product of a gene coding the HAT of the target pest.

As mentioned above, the present disclosure provides seeds and plants having one or more transgenic events. Combinations of events are referred to as "stacked" transgenic events. These stacked transgenic events can be events that are directed at the same target pest, or they can be directed at different target pests. In one embodiment, a seed having the ability to express a nucleic acid provided herein also has the ability to express at least one other insecticidal agent, including, but not limited to, an RNA molecule the sequence of which is derived from the sequence of an RNA expressed in a target pest and that forms a double stranded RNA structure upon expressing in the seed or cells of a plant grown from the seed, wherein the ingestion of one or more cells or the cell content of the plant by the target pest results in the suppression of expression of the RNA in the cells of the target pest. In further embodiments, a seed having the ability to express a dsRNA the sequence of which is derived from a target pest also has a transgenic event that provides herbicide tolerance. One beneficial example of a herbicide tolerance gene provides resistance to glyphosate, N- (phosphonomethyl) glycine, including the isopropylamine salt form of such herbicide.

In the present method, combination of expression of an insecticidal amount of a dsRNA within the cells of a transgenic seed or plant grown from the seed coupled with treatment of the seed or plant with certain chemical or protein pesticides may be used to provide unexpected synergistic advantages, including unexpectedly superior efficacy for protection against damage to the resulting transgenic plant by the target pest. In particular embodiments, treatment of a transgenic seed that is capable of expressing certain constructs that form dsRNA molecules, the sequence of which are derived from one or more sequences expressed in aphids. In addition, it is believed that such combinations are also effective to protect the emergent plants against predation by other pests. The seeds of the present disclosure may also be used to decrease the cost of pesticide use because less pesticide can be used to obtain a required amount of protection than when such methods are not used. Moreover, because less pesticide is used and because it is applied prior to planting and without a separate field application, it is believed that the subject method is therefore safer to the operator and to the environment, and is potentially less expensive than conventional methods.

Pesticides and insecticides that are useful in compositions in combination with the methods and compositions of the present disclosure, including as seed treatments and coatings, as well as methods for using such compositions, can be found, for example, in U.S. Patent 6,551,962, the entirety of which is incorporated herein by reference.

It is anticipated that the combination of certain stabilized dsRNA constructs with one or more insect control protein genes will result in synergies that enhance the insect control phenotype of a transgenic plant. Insect bioassays employing artificial diet- or whole plant tissue can be used to define dose-responses for larval mortality or growth inhibition using both dsRNAs and insect control proteins. One skilled in the art can test mixtures of dsRNA molecules and insect control proteins in bioassay to identify combinations of actives that are synergistic and desirable for deployment in insect-protected plants (Tabashnik, 1992). Synergy in killing insect pests has been reported between different insect control proteins (for review, see Schnepf et al., 1998). It is anticipated that synergies will exist between certain dsRNAs and between certain dsRNAs and certain insect control proteins.

The disclosure also relates to commodity products containing one or more of the sequences of the present disclosure, and produced from a recombinant plant or seed containing one or more of the nucleotide sequences of the present disclosure are specifically contemplated as embodiments of the present disclosure. A commodity product containing one or more of the sequences of the present disclosure is intended to include, but not be limited to, meals, oils, crushed or whole grains or seeds of a plant, or any food product comprising any meal, oil, or crushed or whole grain of a recombinant plant or seed containing one or more of the sequences of the present disclosure. The detection of one or more of the sequences of the present disclosure in one or more commodity or commodity products contemplated herein is de facto evidence that the commodity or commodity product is composed of a transgenic plant designed to express one or more of the nucleotide.

### H. Obtaining Nucleic Acids

Some embodiments pertain to isolated and purified nucleotide sequences as HAT inhibitors that may be used as insect control agents.

Therefore, the present disclosure provides a method for obtaining a nucleic acid comprising a nucleotide sequence for producing a dsRNA or siRNA. In one embodiment, such a method for obtaining a nucleic acid fragment comprises a nucleotide sequence for producing a substantial portion of a dsRNA or siRNA comprises: (a) synthesizing first and a second oligonucleotide primers corresponding to a portion of one of the nucleotide sequences from a targeted insect; and (b) amplifying a cDNA or gDNA template in a cloning vector using the first and second oligonucleotide primers of step (a) wherein the amplified nucleic acid molecule transcribes a substantial portion of a dsRNA or siRNA of the present invention. The preferred target genes of the present disclosure are genes encoding HAT. For the purpose of the present invention, the dsRNA or siRNA molecules may be obtained from a HAT encoding DNA or RNA by polymerase chain (PCR) amplification of a target HAT gene sequences.

Nucleic acid molecules and fragments thereof from aphids or other Hemiptera pest species may be employed to obtain other nucleic acid molecules from other species for use in the present disclosure to produce desired dsRNA and siRNA molecules. Such nucleic acid molecules include the nucleic acid molecules that encode the complete coding sequence of a protein and promoters and flanking sequences of such molecules. In addition, such nucleic acid molecules include nucleic acid molecules that encode for gene family members. Such molecules can be readily obtained by using the above-described nucleic acid molecules or fragments thereof to screen, for instance, cDNA or gDNA libraries. Methods for forming such libraries are well known in the art.

In order to obtain a DNA segment from the corresponding HAT gene in an insect species, PCR primers may be designed based on the sequence as found in the insects from which the HAT gene has been cloned. The primers are designed to amplify a DNA segment of sufficient length for use in the present disclosure. DNA (either genomic DNA or cDNA) is prepared from the insect species, and the PCR primers are used to amplify the DNA segment. Amplification conditions are selected so that amplification will occur even if the primers do not exactly match the target sequence. Alternately, the gene (or a portion thereof) may be cloned from a gDNA or cDNA library prepared from the insect pest species, using the HAT gene or another known insect gene as a probe. Techniques for performing PCR and cloning from libraries are known. Further details of the process by which DNA segments from target insect pest species may be isolated based on the sequence of the HAT genes previously cloned from *Acyrtosiphon pisum* or other insect species are provided in the Examples. One of ordinary skill in the art will recognize that a variety of techniques may be used to isolate gene segments from insect pest species that correspond to genes previously isolated from other species.

Further examples of HAT-inhibitors include also substances that specifically bind to the already expressed HAT (for example, an antibody, an antibody fragment, a peptide or a low molecular weight compound (small molecule)). The substance that specifically binds to the expressed HAT may be obtained or prepared using binding assay targeted at HAT. An antibody that specifically binds to HAT may be prepared using, for example, an immunological method, a phage display method, or a ribosome display method.

Advantageous embodiments for HAT-inhibitors that binds to a protein product of a gene coding HAT are small chemical molecule targeted the protein product of a gene coding HAT and peptides or polypeptides targeted the protein product of a gene coding HAT

The terms "polypeptide", "peptide", or "protein" are used interchangeably herein to designate a linear series of amino acid residues connected one to the other by peptide bonds between the alpha-amino and carboxyl groups of adjacent residues. The amino acid residues are preferably in the natural "L" isomeric form. However, residues in the "D" isomeric form can be substituted for any L-amino acid residue, as long as the desired functional property is retained by the polypeptide. In addition, the amino acids, in addition to the 20 "standard" amino acids, include modified and unusual amino acids.

In an advantageous embodiment, the HAT inhibitor is an antibody or an antibody fragment selected from the group consisting of a monoclonal antibody, polyclonal antibody, Fab, scFv, single domain, or a fragment thereof, bis scFv, F(ab')₂, F(ab)₃, minibody, diabody, triplebody, tetrabody and tandab, wherein the antibody or antibody fragment binds specifically to HAT

In summary, the present disclosure pertains to the following items:
Item 1: A pest control method comprising incorporating an inhibitor against a histone acetyltransferase (HAT) into the body of an agricultural target pest expressing a HAT, wherein the target pest is an insect belonging to the order Hemiptera and the uptake by the target pest of said inhibitor reduces at least the reproduction and/or survival and/or inhibits the development of said target pest.
Item 2: The pest control method according to item 1, wherein the target pest belonging to the suborder Sternorrhyncha, in particular the target pest belonging to the genera of aphids and in particular the aphid is *Acyrthosiphon pisum.*
Item 3: The pest control method according to any one of item 1 to item 2, wherein the HAT is coded by a nucleic acid comprising the sequence of SEQ ID NO: 1, or homologs thereof, wherein said homologs have a sequence identity of at least 80%, in particular of at least 85%, in particular of at least 90%, in particular of at least 95, 96, 97, 98 or 99% to SEQ ID NO: 1 and encodes a functional HAT in the target pest.
Item 4: The pest control method according to any one of item 1 to item 3, wherein the inhibitor is a compound selected from the group consisting of the following (a) to (d):
   (a) a RNAi inducing compound targeted a nucleic acid coding a HAT or parts thereof;
   (b) a nucleic acid construct intracellularly producing a RNAi inducing compound targeted a nucleic acid coding a HAT or parts thereof;
   (c) an antisense nucleic acid targeted at the transcript product of a gene coding a HAT of the target pest; and
   (d) a ribozyme targeted at the transcript product of a gene coding a HAT of the target pest.
Item 5: The pest control method according to any one of item 1 to item 4, wherein the RNAi inducing compound is a compound selected from the group consisting of short interfering nucleic acids, siNA, short interfering RNA (siRNA), microRNA (miRNA), short hairpin RNAs (shRNA) and precursors thereof which are processed in the cell to the actual RNAi inducing compound, in particular wherein the RNAi inducing compound is double-stranded RNA (dsRNA).
Item 6: The pest control method according to any one of item 1 to item 5, wherein the dsRNA is coded by the sequence set forth in SEQ ID NO: 2, or homologs thereof, wherein said homologs have a sequence identity of at least 80 %, in particular of at least 85%, in particular of at least 90%, in particular of at least 95, 96, 97, 98 or 99% to SEQ ID NO: 2 and the dsRNA reduces at least the reproduction and/or survival and/or inhibits the development of said target pest.
Item 7: The pest control method according to any one of item 1 to item 6, comprising making a plant, which is to be attacked by the target pest, possess an agent containing the inhibitor by application, spraying, or atomization in advance, and incorporating the inhibitor into the body of the target pest by ingestion and/or topical application.
Item 8: The pest control method according to any one of item 1 to item 7 comprising incorporating the HAT inhibitor into the body of the target pest by ingestion of a transgenic plant containing a gene encoding the inhibitor.
Item 9: An isolated polynucleotide, which
   (i) is defined as operably linked to a heterologous promoter; or
   (ii) is defined as comprised on a plant transformation vector;
   wherein the polynucleotide is selected from the group consisting of:
   a) a polynucleotide comprising a nucleic acid sequence of SEQ ID NO. 2;
   b) a polynucleotide that hybridizes to a nucleic acid sequence of SEQ ID NO. 2 under stringent conditions;
   c) a polynucleotide of at least 70, at least 80, at least 85, at least 90 percent sequence identity, to the nucleic acid sequence of SEQ ID NO. 2;
   d) a fragment of at least 16 contiguous nucleotides, in particular of at least 21 contiguous nucleotides of the nucleic acid sequence of SEQ ID NO. 1; and
   e) a complement of the sequence of (a), (b), (c) or (d),
   wherein the uptake by a Hemiptera pest of a double stranded ribonucleotide sequence comprising at least one strand that is complementary to said polynucleotide or said fragment reduce at least the reproduction and/or survival of said pest and/or inhibits the development of said pest, in particular wherein the Hemiptera pest belonging to the suborder Sternorrhyncha, in particular belonging to the family of aphids like *Acyrthosiphon pisum.*
Item 10: The isolated polynucleotide according to item 9, wherein the promotor is active in the phloem of a crop plant, in particular wherein the promotor is a CaMV 35S or a SUC2 promotor.19.
Item 11: The isolated polynucleotide according to item 9, wherein the vector is a binary vector.
Item 12: A double stranded ribonucleotide sequence produced from the expression of a polynucleotide according to any one of item 9 to 11, wherein the incorporation of said ribonucleotide sequence in a Hemiptera pest reduces at least the survival and/or reproduction and/or inhibits the development of said pest, in particular wherein the Hemiptera pest belonging to the suborder Sternorrhyncha, in particular belonging to the family of aphids like *Acyrthosiphon pisum.*
Item 13: 4. An isolated double stranded ribonucleotide (dsRNA) sequence having a survival and/or reproduction and/or development inhibitory effect on a Hemiptera pest and consisting of an nucleic acid sequence between 250 to 600 nucleic acids in length, in particular between 300 and 550 nucleic acids in length, in particular between 300 and 520 nucleic acids in length, wherein the dsRNA sequence comprises a nucleic acid sequence derived from SEQ ID NO. 1 or homologs thereof, wherein said homologs have a sequence identity of at least 80 %, in particular of at least 85%, in particular of at least 90%, in particular of at least 95, 96, 97, 98 or 99% to SEQ ID NO: 1, wherein the derived dsRNA is coded by a continuous part of SEQ ID NO. 1 or homologs thereof, wherein said homologs have a sequence identity of at least 80%, in particular of at least 85%, in particular of at least 90%, in particular of at least 95, 96, 97, 98 or 99% to SEQ ID NO: 1 and having a nucleic acid sequence between 300 to 520 nucleic acids in length and wherein the Hemiptera pest belonging to the suborder Sternorrhyncha, in particular belonging to the family of aphids like *Acyrthosiphon pisum.*
Item 14: The double stranded ribonucleotide sequence according to item 12 or item 13, wherein the ribonucleotide sequence is coded by a nucleic acid sequence of SEQ ID NO:2 or homologs thereof, wherein said homologs have a sequence identity of at least 80 %, in particular of at least 85%, in particular of at least 90%, in particular of at least 95, 96, 97, 98 or 99% to SEQ ID NO: 2 and the ribonucleotide sequence reduces the survival and/or reproduction and/or inhibits the development of said target pest.
Item 15: A cell transformed, transduced or transfected with a polynucleotide according to any one of item 9 to 11, in particular wherein the cell is defined as
   (i) a prokaryotic cell; or
   (ii) a eukaryotic cell; or
   (iii) a plant cell
Item 16: A plant transformed, transduced or transfected with a polynucleotide according to any one of item 9 to 11, or a seed thereof comprising the polynucleotide.
Item 17: The plant according to item 16, wherein said polynucleotide is expressed in a cell of the plant as a double stranded ribonucleotide sequence and ingestion of a target pest inhibitory amount of said double stranded ribonucleotide sequence and/or of a RNAi inducing compound derived from said double stranded ribonucleotide sequence in a diet inhibits the target pest from further feeding on said diet, preferably
   (i) the target pest is an insect belonging to the order Hemiptera, in particular belonging to the suborder Sternorrhyncha, in particular to a pest belonging to the family of aphids, in particular *Acyrthosiphon pisum;* or
   (ii) ingestion of the target pest inhibitory amount of the double stranded ribonucleotide sequence or fragments thereof stunts the survival and/or reproduction of the pest.
Item 18: A commodity product produced from a plant according to any one of item 16 to 17, wherein said commodity product comprises a detectable amount of a polynucleotide according to any one of items 9 to 10 or a ribonucleotide expressed therefrom.
Item 19: A method for controlling Hemiptera pest infestation comprising providing in the diet of a Hemiptera pest an agent comprising a first polynucleotide sequence that functions upon ingestion by the pest to inhibit a biological function within said pest, wherein said polynucleotide sequence exhibits from about 95 to about 100 percent nucleotide sequence identity along at least from about 16 to about 25 contiguous nucleotides to a HAT coding sequence derived from said pest and is hybridized to a second polynucleotide sequence that is complementary to said first polynucleotide sequence, and wherein said HAT coding sequence derived from said pest is SEQ ID NO:1, or complements thereof, wherein in particular said Hemiptera pest is an insect belonging to the suborder Sternorrhyncha, which preferably is selected from the group consisting of the family of aphids, in particular said Hemiptera pest is the aphid *Acyrthosiphon pisum.*
Item 20: A method for controlling a Hemiptera pest infestation comprising providing in the diet of a Hemiptera pest a plant cell expressing a polynucleotide sequence according to any one of claims 16 to 20, wherein the polynucleotide is expressed to produce a double stranded ribonucleic acid, wherein said double stranded ribonucleotide acid and/or a RNAi inducing compound derived from said double stranded ribonucleotide acid functions upon ingestion by the pest to inhibit the expression of a HAT encoding target sequence within said pest and results in decreased at least survival and/or reproduction on said diet relative to a diet lacking the plant cell, wherein in particular the Hemiptera pest is an insect belonging to the suborder Sternorrhyncha, which preferably is selected from the group consisting of the genera of aphids; or said Hemiptera pest is the aphid *Acyrthosiphon pisum*; or the polynucleotide functions upon ingestion by the pest to suppress a HAT gene, in particular p300/CBP.
Item 21: A method for improving the yield of a crop produced from a crop plant subjected to insect pest infestation, said method comprising the steps of,
   a) introducing a polynucleotide according to any one of items 9 to 10 into said crop plant,
   b) cultivating the crop plant to allow the expression of said polynucleotide, wherein expression of the polynucleotide inhibits s insects pests and loss of yield due to pest infestation.
Item 22: A transgenic plant comprising a gene coding an inhibitor against HAT of a target pest, in particular p300/CBP of an aphid.
Item 23: The transgenic plant according to item 22, wherein the gene is a polynucleotide according to any one of items 9 to 10.
Item 24: Use of a polynucleotide according to any one of items 9 to 10 as an insect control agent.
Item 25: A method of inhibiting the growth of a target pest expressing histone acetyltransferase (HAT), whereby the method comprises contacting said target pest with an inhibitor against said HAT, wherein said inhibitor inhibits the HAT expression and/or binds to a protein product of a gene coding HAT, wherein in particular the HAT is p300 and/or its paralog CREB-binding protein (p300/CBP).
Item 26: The method according to item 25, wherein the target pest belonging to the suborder Sternorrhyncha, in particular belonging to the family of aphids like *Acyrthosiphon pisum.*
Item 27: The method according to any one of items 25 to 26, wherein the HAT is coded by an mRNA comprising SEQ ID NO: 1, or homologs thereof, preferably said homologs have a sequence identity of at least 60%, in particular of at least 70%, in particular of at least 80%, in particular of at least 85%, in particular of at least 90%, in particular of at least 95%, in particular of at least 96, 97, 98 or 99% to SEQ ID NO: 1 and encodes a functional HAT in the target pest.
Item 28: The method according to any one of items 25 to 27, wherein the inhibitor is a compound selected from the group consisting of the following (a) to (f):
   (a) an RNAi inducing compound targeted a nucleic acid coding HAT or parts thereof;
   (b) a nucleic acid construct intracellularly producing an RNAi inducing compound targeted a nucleic acid coding HAT or parts thereof;
   (c) an antisense nucleic acid targeted at the transcript product of a gene coding HAT or parts thereof;
   (d) a ribozyme targeted at the transcript product of a gene coding HAT or parts thereof;
   (e) a small chemical molecule targeted the protein product of a gene coding HAT;
   (f) a peptide or polypeptide targeted the protein product of a gene coding HAT.
Item 29: The method according to item 28, wherein the RNAi inducing compound is a compound selected from the group consisting of short interfering nucleic acids, siNA, short interfering RNA (siRNA), microRNA (miRNA), short hairpin RNAs (shRNA) and precursors thereof which are processed in the cell to the actual RNAi inducing compound, in particular wherein the precursor is double-stranded RNA (dsRNA).
Item 30: The method according to item 29, wherein the dsRNA is derived from SEQ ID NO:1, or homologs thereof, preferably said homologs have a sequence identity of at least 60%, in particular of at least 70%, in particular of at least 80%, in particular of at least 85%, in particular of at least 90%, in particular of at least 95%, in particular of at least 96, 97, 98 or 99% to SEQ ID NO:1, and wherein the dsRNA reduces at least the reproduction and/or survival of said target pest.
Item 31: The method according to any one of items 28 to 30, wherein the dsRNA is coded by a cDNA sequence comprising or consistsing of SEQ ID NO:2, or homologs thereof, preferably said homologs have a sequence identity of at least 60%, in particular of at least 70%, in particular of at least 80%, in particular of at least 85%, in particular of at least 90%, in particular of at least 95%, in particular of at least 96, 97, 98 or 99% to SEQ ID NO:2 and wherein the dsRNA reduces at least the reproduction and/or survival of said target pest.
Item 32: The method according to item 25, wherein the inhibitor is an antibody-like a polyclonal or monoclonal antibody, or an antibody fragment like an antibody fragment selected from the group consisting of Fab, scFv, single domain, or a fragment thereof, bis scFv, F(ab')₂, F(ab')₃, minibody, diabody, triabody, tetrabody and tandab.
Item 33: The method according to one of the previous items, wherein the incorporating of said inhibitor reduces at least survival and/or reproduction and/or inhibits the development of said pest, in particular wherein the incorporating of said inhibitor reduces the survival and reproduction and inhibits the development of said Hemiptera pest, wherein said Hemiptera pest is the aphid *Acyrthosiphon pisum.*

The following methods and examples are offered for illustrative purposes only, and are not intended to limit the scope of the present disclosure in any way.

### Methods and Examples

In the following examples, materials and methods of the present disclosure are provided including the determination of the effect of HAT silencing on pest reproduction, survival and/or development. It should be understood that these examples are for illustrative purpose only and are not to be construed as limiting this disclosure in any manner. All publications, patents, and patent applications cited herein are hereby incorporated by reference in their entirety for all purposes.

### Example 1: RNA extraction, cloning, and sequencing of the gene encoding p300/CBP

We extracted total RNA from pools of 10 aphids (*A. pisum* clone LL01) using the NucleoSpin RNA Kit (Macherey-Nagel, Germany) according to the manufacturer's protocol. The first strand cDNA was synthesized from RNA (100 ng) using the RevertAid First Strand cDNA synthesis kit and the oligo dT primers provided with it (Thermo Fisher Scientific, Germany). We sequenced nine overlapping fragments covering the open reading frame (ORF) together with the 5' untranslated region (UTR) of the *A. pisum* p300/CBP mRNA (Table 3). Sequencing primers were designed using Primer3 v4.1.0 (http://primer3.ut.ee/). As a template for primer design, we used the *A*. *pisum* sequence from the NCBI database (XM_003242184). The p300/CBP fragments were amplified by PCR using the following parameters: an initial denaturation step at 95°C for 3 min, then 34 cycles of denaturation at 95°C for 30 s, annealing at 58°C for 30 s, and elongation at 72°C for 60 s, followed by a final elongation step at 72°C for 5 min. The reaction volume of 25 µl comprised 4 µl cDNA (25 ng/µl), 10 µM of each primer (1 µl), 12.5 µl of GoTaq Green 2x Master Mix (Promega, Germany) and 6.5 µl nuclease-free water. The PCR products were visualized by 1% agarose gel electrophoresis using SYBR Safe (Invitrogen, Germany) and eluted using the NucleoSpin Gel and PCR clean-up kit (Macherey-Nagel, Germany). Subsequently, the purified amplicons were transferred to vector pGEM-T Easy (Promega, Germany) and introduced into RapidTrans TAM1 competent Escherichia coli cells (Active Motif, USA). The transformed cells were spread on MacConkey agar (Carl Roth, Germany) and incubated at 37°C overnight. The colonies were then screened for the PCR insert using the standard T7 primer set. Positive colonies were cultivated at 37°C overnight in 5 ml lysogeny broth supplemented with 100 µg/ml ampicillin in a shaking incubator (200 rpm). Plasmids were extracted using the NucleoSpin PlasmidEasyPure kit (Macherey-Nagel, Germany) and the insert was sequenced for verification using standard primer SP6 on a 3730xl DNA analyzer (Macrogen Europe, Netherlands). Sequence fragments were aligned using Geneious v10.2.4 (https://www.geneious.com).

**Table 3. Primers used to sequence the A. pisum p300/CBP gene.**

| **Primer name** | **Sequence** | **Fragment size (bp)** | **Position on NCBI sequence XM_003242184.3** |
|---|---|---|---|
| **seq_AP_p300_fwd_1** | 5'CTG ACC TTG GAC CGT GAG TT3' | 807 | 43-849 |
| **seq_AP_p300_rev_1** | 5'CAA ATT GAG AAA CAA AAT AGT TAA ACA3' | | |
| **seq_AP_p300_fwd_2** | 5'AAT CAG CCT CCG ACT TAG CA3' | 856 | 742-1597 |
| **seq_AP_p300_rev_2** | 5'CTG GTT GCG AAT TTG TTG TG3' | | |
| **seq_AP_p300_fwd_3** | 5'AGC AAC AAC AAA ACG GAC CT3' | 887 | 1527-2413 |
| **seq_AP_p300_rev_3** | 5'TAC CTG GGG TTG TGA ATG TG3' | | |
| **seq_AP_p300_fwd_4** | 5'CTC AAA TGC CAC ATC CTC CT3' | 801 | 2328-3128 |
| **seq_AP_p300_rev_4** | 5'TTG GAA AAT GTA CCC TGT CTG A3' | | |
| **seq_AP_p300_fwd_5** | 5'TCA ATT CAA CCC AAT CAG CA3' | 769 | 3032-3800 |
| **seq_AP_p300_rev_5** | 5'CCA TTT TCT TTG AAC TTG AAC CA3' | | |
| **seq_AP_p300_fwd_6** | 5'AAC ACC GTC GGC ATC TGT GA3' | 1002 | 3727-4729 |
| **seq_AP_p300_rev_6** | 5'AGC AAC TTC ACC AGC GCC TG3' | | |
| **seq_AP_p300_fwd_7** | 5'ATT GGC TAT AAC AAA ATT AGG TAC G3' | 839 | 4642-5480 |
| **seq_AP_p300_rev_7** | 5'TGC TCT TTC TCT GGC TGG AC3' | | |
| **seq_AP_p300_fwd_8** | 5'GAA GTT GAA ATT GGC CCT GA3' | 865 | 5393-6257 |
| **seq_AP_p300_rev_8** | 5'GCT GTG CTT GTT GCA GTC TT3' | | |
| **seq_AP_p300_fwd_9** | 5'TGT CCA GTG TTG TTC TGT CCA3' | 811 | 6179-6992 |
| **seq_AP_p300_rev_9** | 5'GGA ACA TTT GCC TTT GTC GT3' | | |

### Example 2: Synthesis of dsRNA

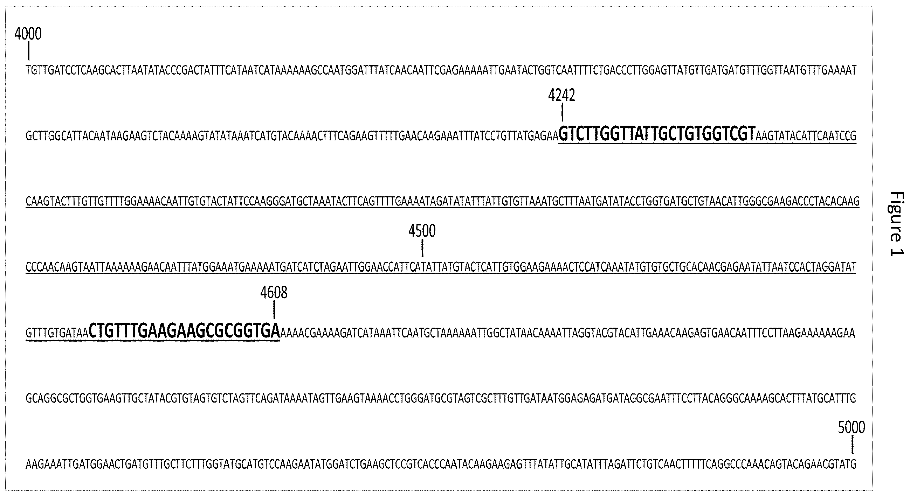

The *A. pisum* p300/CBP mRNA sequence was used to design gene-specific RNAi primers (with Primer3 v4.1.0) which were then prepared externally (Sigma-Aldrich, Germany). Each primer included a 5' T7 promoter sequence. The dsRNA construct was designed to be 367 bp in length (GC content = 40-60%) covering parts of ORF (Figure 1, Table 4). This construct was checked for off-targets by screening against the entire pea aphid genome, ensuring there are no overlaps >19 bp with other *A*. *pisum* genes. The PCR amplicon generated using the RNAi primers and cDNA template was cloned and sequenced as described above. The verified plasmid vector was used as a PCR template for the RNAi primers, and the amplicon was excised from the gel and purified using the NucleoSpin Gel and PCR Clean-up kit (Macherey-Nagel, Germany). Subsequently, the purified PCR product was used within the Ambion MEGAscript T7 kit (Applied Biosystems, USA) to synthesize dsRNA. The dsRNA was purified by isopropanol precipitation and washed with ethanol. The pellet was resuspended in 30-50 µl nuclease-free water and stored -20°C.

**Table 4. RNAi primers (5' T7 promoter sequence underlined) for the A. pisum p300/CBP gene.**

| **Primer name** | **Sequence** | **dsRNA length** |
|---|---|---|
| **RNAi_AP_p300_fwd** | 5'TAA TAC GAC TCA CTA TAG GGA GAG TCT TGG TTA TTG CTG TGG TCG T3' | 367 |
| **RNAi_AP_p300_rev** | 5'TAA TAC GAC TCA CTA TAG GGA GAT CAC CGC GCT TCT TCA AAC AGT3' | |

### Example 3: Aphid rearing, RNAi injection and dissections

*A. pisum* parthenogenetic clone LL01 was reared on 2-3-week-old bean plants (*Vicia faba* var. *minor*) in a KBWF 720 climate cabinet (Binder GmbH, Germany) with a 16-h photoperiod and a day/night temperature of 24/18°C (Luna-Ramirez et al., 2017, Will et al., 2017). Prior to aphid infestation, bean plants were cultivated at a constant temperature of 20°C with a 14-h photoperiod. Age-synchronized, 5-day-old aphids were used for the injection assays (Sapountzis et al., 2014). In the RNAi experiments, aphids were injected using glass capillaries held on a M3301 micromanipulator (World Precision Instruments, USA). The aphids were injected laterally, between the mesothorax and metathorax, with 25 nl of the p300/CBP dsRNA (50, 250, 1000 and 3000 ng/µl) or GFP dsRNA as a control (3000 ng/µl). We injected a total of 200 aphids per treatment, comprising five biological replicates of 40 aphids each. After injection, aphids were individually transferred to Petri dishes with *V. faba* leaves on 1% agarose gel. The Petri dishes and leaves were replaced every 5 days to ensure optimal conditions. The following parameters were monitored: aphid survival, age of first reproduction, total number of offspring, average number of premature (dead) offspring, number of premature offspring per day, and number of viviparous (normal) offspring per day (Skaljac et al., 2018). Premature aphid nymphs are defined as individuals that died a few hours after eclosion, with antennae and legs remaining in the folded position (Will et al., 2017).

We also measured the body weight (0, 3 and 8 days post-injection), body size (3 and 8 days post-injection) and body color (3 and 8 days post-injection) of 40 individuals treated with the highest concentration of p300/CBP or GFP dsRNA (3000 ng/µl). To record the size and color of the aphids, images were acquired under an MZ 16 FA stereomicroscope (Leica, Germany) and measured using ImageJ v1.52o. In addition, ovaries were dissected from aphids injected with the highest concentration of p300/CBP or GFP dsRNA (3000 ng/µl) 10 days post-injection. The ovaries were stored in PBS-T (phosphate-buffered saline with 0.1% Tween-20) and images of the dissected specimens were acquired as described above. We examined the total number of embryos in the ovaries but also the number of late stage embryos (stage 18 or older, defined by the occurrence of visible eyes) and early stage embryos (stage 17 or younger; no visible eyes) (Miura et al., 2003).

Finally, the survival of aphids was also examined in the F1 offspring generation in order to evaluate transgenerational silencing effects. The neonate F1 nymphs (0-24 h old, 40 nymphs per treatment or control) were collected and individually transferred to Petri dishes with *V. faba* leaves 6 days after the injection of their mothers.

### Example 4: Data analysis

The statistical data were analyzed using IBM SPSS Statistics v25 (Armonk, USA). The threshold for statistical significance was set to p < 0.05 for all the tests, except two-way analysis of variance (ANOVA) where the threshold was p < 0.001. The significance of survival, visualized by Kaplan-Meier survival analysis, and the start of reproduction, visualized as bars, were calculated using the log-rank test. The total numbers of viviparous and premature offspring were analyzed using a Kruskal-Wallis test with Bonferroni post hoc correction. The number of offspring per day was analyzed by two-way ANOVA, whereas body size, body color, and body weight were evaluated using Student's t-test.

### Example 5: Sequence of A. pisum p300/CBP mRNA and similarities with other aphid pests

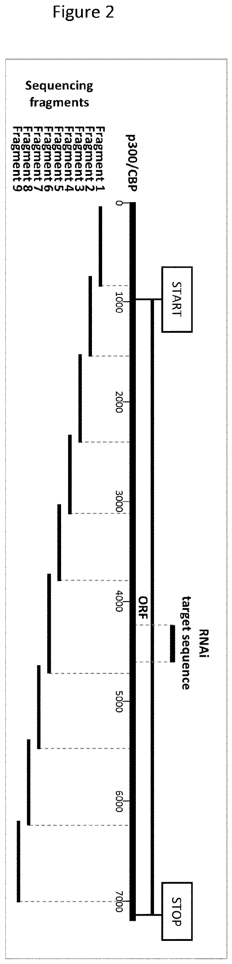

To confirm the p300/CBP mRNA sequence from the *A. pisum* LL01 clone, we used the corresponding sequence available in the NCBI database (XM_003242184) as the template. The predicted *A. pisum* p300-like mRNA sequence (XM_003242184) is 7183 bp long, whereas we generated a p300/CBP assembly 6947 bp in length. This assembly was produced by sequencing nine overlapping fragments of 800-1000 bp each (Figure 2, Table 3). The p300/CBP mRNA sequence we isolated includes the 5' UTR (928 bp), ATG start codon and ORF (6019 bp). Based on the predicted sequence, our p300/CBP sequence does not contain the last 134 bp of the ORF, which includes the stop codon and 3' UTR (Figure 2, Table 3). Apart from a few single nucleotide polymorphisms (SNPs), we confirmed the predicted p300/CBP mRNA in *A. pisum.* The sequences of the fragments and the assembly are shown below.

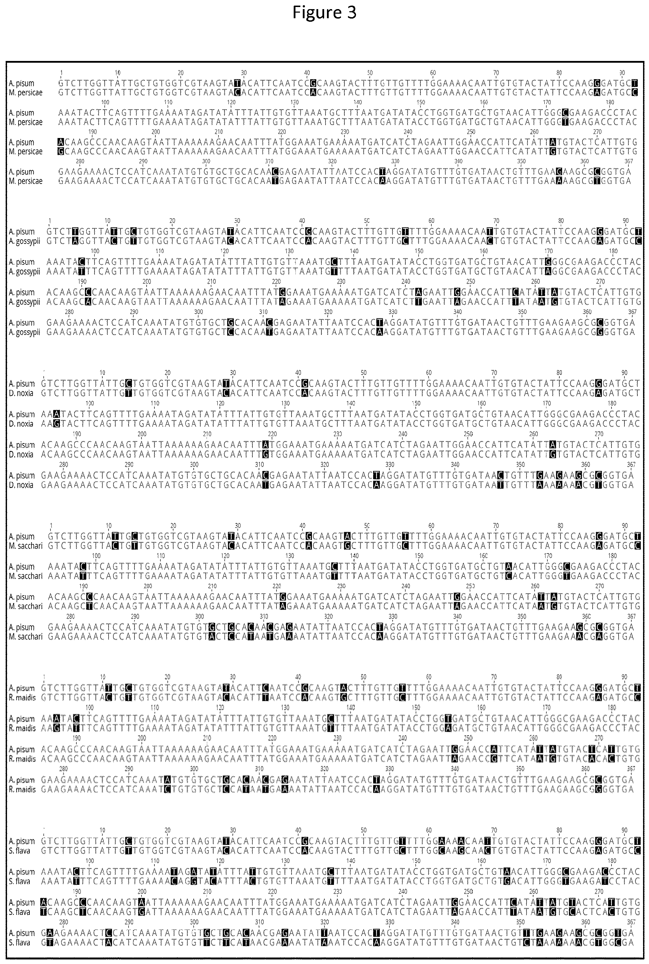

To evaluate whether the p300/CBP dsRNA construct generated in this study might silence the corresponding gene in other aphid species, we used the BLAST search tool as well as alignments integrated into Geneious v10.2.4. We observed 88-97% identity at the nucleotide level between the *A. pisum* p300/CBP gene and other aphid pest species: *Myzus persicae, Aphis gossypii, Diuraphis noxia, Melanaphis sacchari, Rhopalosiphum maidis* and *Sipha flava* (Table 5, Figure 3). We found three contiguous matches of 22 nt or longer in *S. flava,* whereas a maximum of seven matches (26 nt or longer) was found in *M. sacchari* (Table 5). There is a high probability that the p300/CBP dsRNA construct designed in this study will target the other aphid species listed above, although this remains to be experimentally confirmed.

**Table 5. Contiguous nucleotides (>21-mers) of the A. pisum RNAi construct (367 bp) matching p300/CBP mRNA in other aphid species.**

| **Aphid species** | **Sequence identity between *A. pisum* p300/CBP dsRNA and orthologs in other species** | **Number and size of contiguous nucleotides (>21-mers) of *A*. *pisum* RNAi construct (367 bp) matching p300/CBP mRNA of other aphid species** |
|---|---|---|
| *Aphis gossypii* | 93% | 38 nt, 32 nt, 29 nt, 41 nt, 33 nt |
| *Diuraphis noxia* | 96% | 44 nt, 123 nt, 43 nt, 47 nt |
| *Myzus persicae* | 97% | 28 nt, 44 nt, 80 nt, 77 nt, 47 nt, 27 nt |
| *Melanaphis sacchari* | 93% | 29 nt, 38 nt, 26 nt, 29 nt, 26 nt, 38 nt, 30 nt |
| *Rhopalosiphum maidis* | 92% | 29nt, 38nt, 92 nt, 23 nt, 33 nt |
| *Sipha flava* | 88% | 26 nt, 47 nt, 22 nt |

### Example 6: Effects of RNAi-mediated knockdown of p300/CBP on life-history traits of aphids

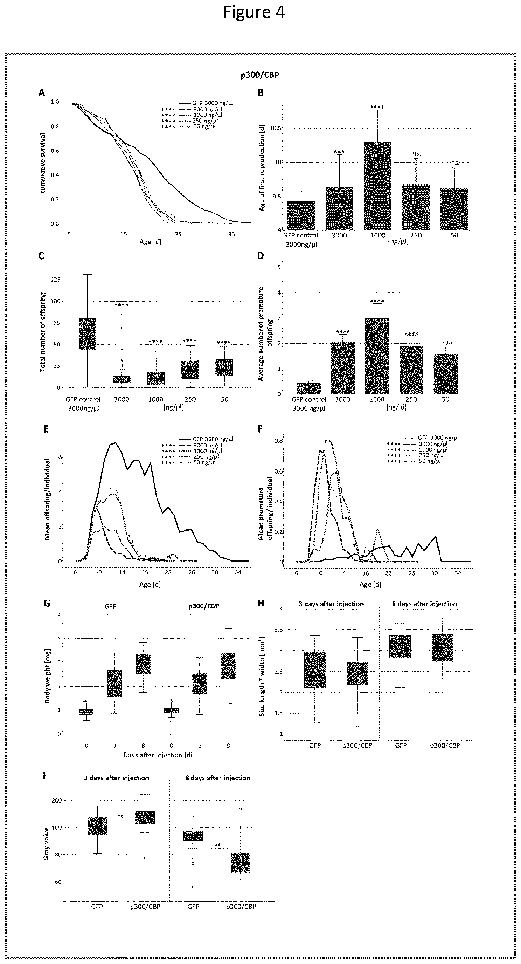

Aphid survival did not differ significantly between the p300/CBP dsRNA and GFP dsRNA control group during the first few days (∼5 days) post-injection. However, the overall lifespan of aphids injected with p300/CBP dsRNA was severely affected (Tables 6 and 7, Figure 4A). The silencing of p300/CBP showed the strongest impact 15 days post-injection, when only ∼10% of aphids survived. After 20 days, there were very few survivors. In the GFP control group, ∼50% of the aphids remained alive 15 days post-injection, and 20% remained alive after 20 days (Table 6, Figure 4A).

The suppression of p300/CBP with large amounts of dsRNA (3000 and 1000 ng/µl) induced a mild but significant delay to the start of reproduction (Table 8, Figure 4B). The total number of offspring was strongly reduced in all four p300/CBP treatment groups compared to the GFP control (reductions of 82%, 81%, 65% and 63% for treatments with 3000, 1000, 250 and 50 ng/µl p300/CBP dsRNA, respectively; Table 9, Figure 4C). Further analysis revealed that the number of offspring per day was also significantly reduced in the p300/CBP dsRNA groups in a concentration-dependent manner (Table 9, Figure 4C,E). Remarkably, the reproductive phase of aphids injected with p300/CBP dsRNA was much shorter (7-10 days) compared to the control group (up to ∼25 days) (Table 10, Figure 4E). The suppression of p300/CBP induced a significant increase in the number of premature nymphs throughout the reproductive phase (Tables 11 and 12, Figure 4D,F). The appearance of premature offspring indicated that p300/CBP plays a key role in aphid embryogenesis and/or eclosion.

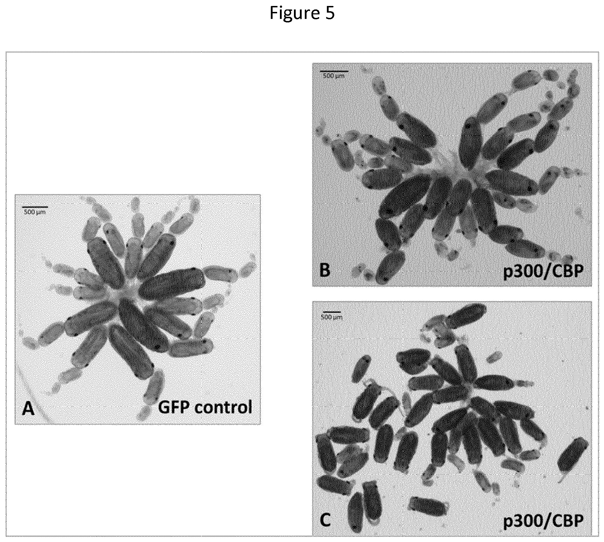

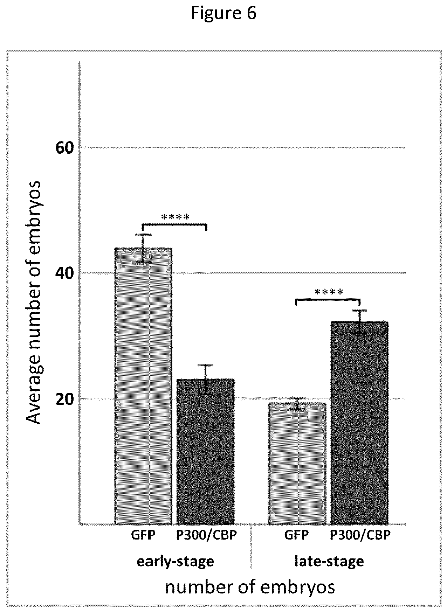

The RNAi-mediated knockdown of p300/CBP did not induce significant changes in aphid weight or size at any time point (Tables 13 and 14, Figure 4G,H). However, the lower expression of p300/CBP caused the injected aphids to become significantly darker in color 8 days post-injection, even though there were no significant differences 3 days post-injection (Table 15, Figure 4I).
In order to better understand the impact of p300/CBP silencing on *A. pisum* reproduction, we dissected ovaries from individuals in the treatment and control groups. Interestingly, ovaries dissected from aphids injected with p300/CBP dsRNA contained a greater number of late-stage embryos 10 days post-injection, and the tissue structure of the ovaries was very fragile and susceptible to rupture (Figure 5B,C). In contrast, ovaries dissected from aphids injected with GFP dsRNA had a normal tissue structure and contained embryos spanning all developmental stages (Figure 5A). Ovaries of p300/CBP dsRNA treated aphids contain significantly less early-stage embryos (p < 0.0001) and significantly more late-stage embryos (p < 0.0001) than the GFP control group (Figure 6). The total amount of embryos did not differ between the two treatment groups (data not shown).

**Table 6. Percentage survival of injected aphids 5, 10, 15 and 20 days after the injection of p300/CBP dsRNA or the control GFP dsRNA.**

| **dsRNA treatment** | **Percentage survival after** | | | |
|---|---|---|---|---|
| | **5 days** | **10 days** | **15 days** | **20 days** |
| **GFP 3000 ng/µl** | 79 | 65 | 45 | 20 |
| **p300/CBP 3000 ng/µl** | 79 | 51 | 13 | 1 |
| **p300/CBP 1000 ng/µl** | 85 | 57 | 8 | 0 |
| **p300/CBP 250 ng/µl** | 88 | 60 | 11 | 0 |
| **p300/CBP 50 ng/µl** | 85 | 61 | 12 | 3 |

**Table 7. Statistical analysis of RNAi data for survival compared to the GFP control.**

| **Treatment** | **Mean survival [d]** | **Standard error [d]** | **Chi-square** | **Significance** |
|---|---|---|---|---|
| **Control (GFP)** | 13.88 | 0.44 | | |
| **p300/CBP 3000 ng/µl** | 10.23 | 0.34 | 63.95 | p<0.0000 |
| **p300/CBP 1000 ng/µl** | 10.26 | 0.39 | 49.03 | p<0.0000 |
| **p300/CBP 250 ng/µl** | 10.93 | 0.40 | 38.21 | p<0.0000 |
| **p300/CBP 50 ng/µl** | 11.08 | 0.47 | 30.65 | p<0.0000 |

**Table 8. Statistical analysis of RNAi data for start of reproduction compared to the GFP control.**

| **treatment** | **Mean start [d]** | **Standard error [d]** | **Chi-square** | **significances** |
|---|---|---|---|---|
| **Control (GFP)** | 4.93 | 0.07 | | |
| **p300/CBP 3000 ng/µl** | 5.13 | 0.25 | 9 | p=0.003 |
| **p300/CBP 1000 ng/µl** | 5.79 | 0.24 | 15.16 | p<0.0000 |
| **p300/CBP 250 ng/µl** | 5.18 | 0.19 | 2.02 | ns |
| **p300/CBP 50 ng/µl** | 5.18 | 0.15 | 1.6 | ns |

**Table 9. Statistical analysis of RNAi data for an average number of offspring compared to the GFP control.**

| **Treatment** | **Mean number** | **Standard error** | **Kruskal-Wallis test value** | **Significances** |
|---|---|---|---|---|
| **Control (GFP)** | 62.38 | 1.84 | | |
| **p300/CBP 3000 ng/µl** | 11.11 | 0.81 | 317.24 | p<0.000 |
| **p300/CBP 1000 ng/µl** | 11.84 | 1.17 | 306.36 | p<0.000 |
| **p300/CBP 250 ng/µl** | 21.38 | 1.42 | 209.96 | p<0.000 |
| **p300/CBP 50 ng/µl** | 23.02 | 1.31 | 184.17 | p<0.000 |

**Table 10. Reproductive parameters evaluated during the RNAi experiments including viviparous offspring determined by two-way ANOVA.**

| **Treatment** | | **Statistical parameters** | **Significances** |
|---|---|---|---|
| **p300/CBP 3000 ng/µl** | Aphid line (control vs p300/CBP) | df=1 mean square=463.702 F=98.235 | p<0.0000 |
| | Age of reproduction | df=28 mean square=349.784 F=74.102 | p<0.0000 |
| | Aphid line x Age of reproduction | df=27 mean square=262.781 F=55.67 | p<0.0000 |
| **p300/CBP 1000 ng/µl** | Aphid line (control vs p300/CBP) | df=1 mean square=2462.32 F=596.994 | p<0.0000 |
| | Age of reproduction | df=30 mean square=204.834 F=49.662 | p<0.0000 |
| | Aphid line x Age of reproduction | df=16 mean square=203.719 F=49.392 | p<0.0000 |
| **p300/CBP 250 ng/µl** | Aphid line (control vs p300/CBP) | df=1 mean square=1829.640 F=373.094 | p<0.0000 |
| | Age of reproduction | df=30 mean square=289.921 F=59.120 | p<0.0000 |
| | Aphid line x Age of reproduction | df= 17mean square=167.724 F=34.202 | p<0.0000 |
| **p300/CBP 50 ng/µl** | Aphid line (control vs p300/CBP) | df=1 mean square=1173.761 F=242.996 | p<0.0000 |
| | Age of reproduction | df=30 mean square=297.476 F=61.584 | p<0.0000 |
| | Aphid line x Age of reproduction | df=20 mean square=132.424 F=27.415 | p<0.0000 |

**Table 11. Statistical analysis of RNAi data for an average number of premature offspring compared to the GFP control.**

| **Treatment** | **Mean number** | **Standard error** | **Kruskal-Wallis test value** | **Significances** |
|---|---|---|---|---|
| **Control (GFP)** | 0.43 | 0.05 | | |
| **p300/CBP 3000 ng/µl** | 2.06 | 0.14 | -185.09 | p<0.000 |
| **p300/CBP 1000 ng/µl** | 2.98 | 0.17 | -242.40 | p<0.000 |
| **p300/CBP 250 ng/µl** | 1.89 | 0.24 | -185.41 | p<0.000 |
| **p300/CBP 50 ng/µl** | 1.57 | 0.24 | -145.93 | p<0.000 |

**Table 12. Reproductive parameters evaluated during the RNAi experiments including premature offspring determined by two-way ANOVA.**

| **Treatment** | | **Statistical parameters** | **Significances** |
|---|---|---|---|
| **p300/CBP 3000 ng/µl** | Aphid line (control vs p300/CBP) | df=1 mean square=0.836 F=5.994 | P=0.014 |
| | Age of reproduction | df=28 mean square=2.472 F=17.722 | p<0.0000 |
| | Aphid line x Age of reproduction | df=21 mean square=3.745 F=26.848 | p<0.0000 |
| **p300/CBP 1000 ng/µl** | Aphid line (control vs p300/CBP) | df=1 mean square=8.954 F=41.950 | p<0.0000 |
| | Age of reproduction | df=30 mean square=1.702 F=7.973 | p<0.0000 |
| | Aphid line x Age of reproduction | df=16 mean square=3.119F=14.614 | p<0.0000 |
| **p300/CBP 250 ng/µl** | Aphid line (control vs p300/CBP) | df=1 mean square=3.658 F=27.031 | p<0.0000 |
| | Age of reproduction | df=30 mean square=0.823 F=6.083 | p<0.0000 |
| | Aphid line x Age of reproduction | df=17 mean square=1.237 F=9.144 | p<0.0000 |
| **p300/CBP 50 ng/µl** | Aphid line (control vs p300/CBP) | df=1 mean square=0.842 F=6.774 | p<0.009 |
| | Age of reproduction | df=30 mean square=0.643 F=5.171 | p<0.0000 |
| | Aphid line x Age of reproduction | df=20 mean square=0.924 F=7.435 | p<0.0000 |

**Table 13. Statistical analysis of RNAi data for body weight [mg] compared to the GFP control (dsRNA concentration = 3000 ng/µl).**

| **Time after injection [d]** | **Body weight p300/CBP [mg]** | | **Body weight GFP control [mg]** | | **Significances** |
|---|---|---|---|---|---|
| | Mean | Standard error | Mean | Standard error | |
| **0** | 1 | 0.02 | 0.92 | 0.02 | ns |
| **3** | 2.10 | 0.07 | 2.07 | 0.09 | ns |
| **8** | 2.86 | 0.09 | 2.89 | 0.07 | ns |

| | | | | | |
|---|---|---|---|---|---|
| ns = not significant | | | | | |

**Table 14. Statistical analysis of RNAi data for body size (length*width)[mm²] compared to the GFP control (dsRNA concentration = 3000 ng/µl).**

| **Time after injection [d]** | **Body size p300/CBP [mm²]** | | **Body size GFP control [mm²]** | | **Significances** |
|---|---|---|---|---|---|
| | mean | Std. error | mean | Std. error | |
| **3** | 2.43 | 0.08 | 2.45 | 0.1 | ns |
| **8** | 3.07 | 0.07 | 3.07 | 0.06 | ns |

| | | | | | |
|---|---|---|---|---|---|
| ns = not significant | | | | | |

**Table 15. Statistical analysis of RNAi data for body color (grayscale) compared to the GFP control (dsRNA concentration = 3000 ng/µl).**

| **Time after injection [d]** | **Body color p300/CBP - grayscale** | | **Body color GFP control - grayscale** | | **Significances** |
|---|---|---|---|---|---|
| | Mean | Standard error | Mean | Standard error | |
| **3** | 107.22 | 1.33 | 101.15 | 1.56 | ns |
| **8** | 75.59 | 2.4 | 95.41 | 0.95 | p<0.01 |

| | | | | | |
|---|---|---|---|---|---|
| ns = not significant | | | | | |

### Example 7: Transgenerational effect of RNAi-mediated knockdown of p300/CBP in the F1 generation

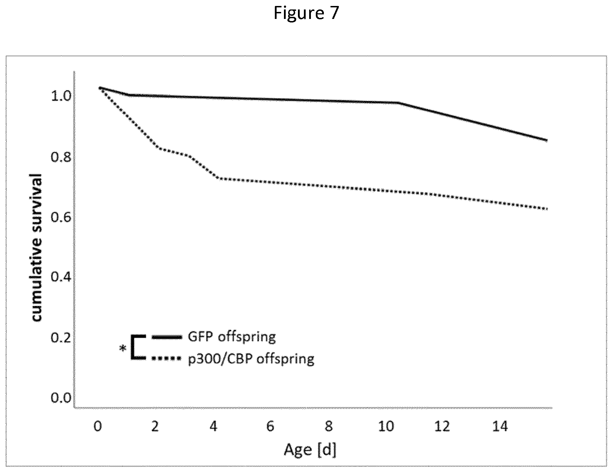

We monitored the survival of the viable offspring (F1 generation) of the aphids treated with p300/CBP dsRNA to investigate the potential for transgenerational effects. We found that the survival rate of the offspring of mothers injected with p300/CBP dsRNA was significantly lower than peers from the control group, whose mothers were injected with GFP dsRNA (Figure 7).

### References

All publications, patents, and patent applications cited in the present application are hereby incorporated by reference in their entirety for all purposes.
ALI, I., CONRAD, R. J., VERDIN, E. & OTT, M. 2018. Lysine Acetylation Goes Global: From Epigenetics to Metabolism and Therapeutics. Chem Rev, 118, 1216-1252.
BANNISTER, A. J. & KOUZARIDES, T. 2011. Regulation of chromatin by histone modifications. Cell Res, 21, 381-95.
BARZMAN, M., BARBERI, P., BIRCH, A. N. E., BOONEKAMP, P., DACHBRODT-SAAYDEH, S., GRAF, B., HOMMEL, B., JENSEN, J. E., KISS, J., KUDSK, P., LAMICHHANE, J. R., MESSEAN, A., MOONEN, A.-C., RATNADASS, A., RICCI, P., SARAH, J.-L. & SATTIN, M. 2015. Eight principles of integrated pest management. Agronomy for Sustainable Development, 35, 1199-1215.
BASSETT, S. & BARNETT, M. 2014. The role of dietary histone deacetylases (HDACs) inhibitors in health and disease. Nutrients, 6, 4273-4301.
BAUM, J. A., BOGAERT, T., CLINTON, W., HECK, G. R., FELDMANN, P., ILAGAN, O., JOHNSON, S., PLAETINCK, G., MUNYIKWA, T., PLEAU, M., VAUGHN, T. & ROBERTS, J. 2007. Control of hemipteran insect pests through RNA interference. Nature Biotechnology, 25, 1322.
BLACKMAN, R. & EASTOP, V. 2017. Aphids on the World's Plants: An online identification and information guide. Online version: http://www.aphidsonworldsplants. info*.*
BLACKMAN, R. L. & EASTOP, V. 2016. Aphids on the World's Plants. An Online Identification and Information Guide. URL: http://www.aphidsonworldsplants. info (last accessed 4 Sep. 2016*).*
BORDOLI, L., NETSCH, M., LÜTHI, U., LUTZ, W. & ECKNER, R. 2001. Plant orthologs of p300/CBP: conservation of a core domain in metazoan p300/CBP acetyltransferase-related proteins. Nucleic acids research, 29, 589-597.
CAGLIARI, D., DOS SANTOS, E. A., DIAS, N., SMAGGHE, G. & ZOTTI, M. 2018. Non-transformative strategies for RNAi in crop protection.
COLEMAN, A., WOUTERS, R., MUGFORD, S. & HOGENHOUT, S. 2014. Persistence and transgenerational effect of plant-mediated RNAi in aphids. Journal of experimental botany, 66, 541-548.
CRUMP, N. T., HAZZALIN, C. A., BOWERS, E. M., ALANI, R. M., COLE, P. A. & MAHADEVAN, L. C. 2011. Dynamic acetylation of all lysine-4 trimethylated histone H3 is evolutionarily conserved and mediated by p300/CBP. Proceedings of the National Academy of Sciences, 108, 7814-7819.
DAMJANOVSKI, S., SACHS, L. M. & SHI, Y. 2004. Multiple stage-dependent roles for histone deacetylases during amphibian embryogenesis: implications for the involvement of extracellular matrix remodeling. International Journal of Developmental Biology, 44, 769-776.
DANCY, B. M. & COLE, P. A. 2015. Protein Lysine Acetylation by p300/CBP. Chemical Reviews, 115, 2419-2452.
GONG, Y.-H., YU, X.-R., SHANG, Q.-L., SHI, X.-Y. & GAO, X.-W. 2014. Oral Delivery Mediated RNA Interference of a Carboxylesterase Gene Results in Reduced Resistance to Organophosphorus Insecticides in the Cotton Aphid, Aphis gossypii Glover. PLOS ONE, 9, e102823.
GOODMAN, R. H. & SMOLIK, S. 2000. CBP/p300 in cell growth, transformation, and development. Genes & development, 14, 1553-1577.
HEIDEBRECHT JR, R. W. 2017. Delivery strategies: RNA interference in agriculture and human health. Pest Management Science, 73, 686-691.
JAUBERT-POSSAMAI, S., LE TRIONNAIRE, G., BONHOMME, J., CHRISTOPHIDES, G. K., RISPE, C. & TAGU, D. 2007. Gene knockdown by RNAi in the pea aphid Acyrthosiphon pisum. BMC biotechnology, 7, 63.
JOGA, M. R., ZOTTI, M. J., SMAGGHE, G. & CHRISTIAENS, O. 2016. RNAi Efficiency, Systemic Properties, and Novel Delivery Methods for Pest Insect Control: What We Know So Far. Frontiers in Physiology, 7*.*
MAO, J. & ZENG, F. 2012. Feeding-Based RNA Intereference of a Gap Gene Is Lethal to the Pea Aphid, Acyrthosiphon pisum. PLOS ONE, 7, e48718.
MAO, J. & ZENG, F. 2014. Plant-mediated RNAi of a gap gene-enhanced tobacco tolerance against the Myzus persicae. Transgenic research, 23, 145-152.
MIURA, T., BRAENDLE, C., SHINGLETON, A., SISK, G., KAMBHAMPATI, S., & STERN, D.L. 2003. A comparison of parthenogenetic and sexual embryogenesis of the pea aphid Acyrthosiphon pisum (Hemiptera: Aphidoidea). Journal of Experimental Zoology Part B: Molecular and Developmental Evolution 295, 59-81.
MUKHERJEE, K. & BAUDACH, A. F. 2016. Epigenetic control of polyphenism in aphids. CRC Press.
MUKHERJEE, K., FISCHER, R. & VILCINSKAS, A. 2012. Histone acetylation mediates epigenetic regulation of transcriptional reprogramming in insects during metamorphosis, wounding and infection. Frontiers in zoology, 9, 25.
MUTTI, N. S., PARK, Y., REESE, J. C. & REECK, G. R. 2006. RNAi Knockdown of a Salivary Transcript Leading to Lethality in the Pea Aphid, <i>Acyrthosiphon pisum</i>. Journal of Insect Science, 6, 1-7, 7.
SAN MIGUEL, K. & SCOTT, J. G. 2016. The next generation of insecticides: dsRNA is stable as a foliar-applied insecticide. Pest Manag Sci, 72, 801-9.
SKALJAC, M., KIRFEL, P., GROTMANN, J. & VILCINSKAS, A. 2018. Fitness costs of infection with Serratia symbiotica are associated with greater susceptibility to insecticides in the pea aphid Acyrthosiphon pisum. Pest management science, 74, 1829-1836.
SKALJAC, M., VOGEL, H., WIELSCH, N., MIHAJLOVIC, S. & VILCINSKAS, A. 2019. Transmission of a protease-secreting bacterial symbiont among pea aphids via host plants. Frontiers in physiology, 10, 438.
SPARKS, T. C. & NAUEN, R. 2015. IRAC: Mode of action classification and insecticide resistance management. Pestic Biochem Physiol, 121, 122-8.
VILCINSKAS, A. 2016a. Biology and ecology of aphids, CRC Press.
VILCINSKAS, A. 2016b. The role of epigenetics in host-parasite coevolution: lessons from the model host insects Galleria mellonella and Tribolium castaneum. Zoology, 119, 273-280.
WILL, T. & VILCINSKAS, A. 2013. Aphid-proof plants: Biotechnology-based approaches for aphid control. Yellow Biotechnology II. Springer.
YI, S. V. 2017. Insights into Epigenome Evolution from Animal and Plant Methylomes. Genome Biol Evol, 9, 3189-3201.
ZHANG, J., KHAN, S. A., HECKEL, D. G. & BOCK, R. 2017. Next-Generation Insect-Resistant Plants: RNAi-Mediated Crop Protection. Trends in Biotechnology, 35, 871-882.
ZHOU, J., SHUM, K.-T., BURNETT, J. & ROSSI, J. 2013. Nanoparticle-based delivery of RNAi therapeutics: progress and challenges. Pharmaceuticals, 6, 85-107.
ZOTTI, M., DOS SANTOS, E. A., CAGLIARI, D., CHRISTIAENS, O., TANING, C. N. T. & SMAGGHE, G. 2018. RNA interference technology in crop protection against arthropod pests, pathogens and nematodes. Pest Management Science, 74, 1239-1250.
ZOTTI, M. J. & SMAGGHE, G. 2015. RNAi Technology for Insect Management and Protection of Beneficial Insects from Diseases: Lessons, Challenges and Risk Assessments. Neotrop Entomol, 44, 197-213.
ZUCCONI, B., MAKOFSKE, J., MEYERS, D., HWANG, Y., WU, M., KURODA, M. & COLE, P. A. 2019. Combination Targeting of the Bromodomain and Acetyltransferase Active Site of p300/CBP. Biochemistry*.*

## Claims

1. A pest control method comprising incorporating an inhibitor against a histone acetyltransferase (HAT) into the body of an agricultural target pest expressing a HAT, wherein the target pest is an insect belonging to the order Hemiptera and incorporating of said inhibitor reduces at least the reproduction and/or survival of said target pest.

2. The pest control method according to claim 1, wherein the target pest belonging to the suborder Sternorrhyncha, in particular belonging to the family of aphids like *Acyrthosiphon pisum.*

3. The pest control method according to any one of claims 1 to 2, wherein the HAT is p300 and/or its paralog CREB-binding protein.

4. The pest control method according to any one of claims 1 to 3, wherein the HAT is coded by a cDNA comprising the sequence of SEQ ID NO: 1, or homologs thereof, wherein said homologs have a sequence identity of at least 80%, in particular of at least 85%, in particular of at least 90% to SEQ ID NO: 1 and encodes a functional HAT in the target pest.

5. The pest control method according to any one of claims 1 to 4, wherein the inhibitor is a compound selected from the group consisting of the following (a) to (d):
(a) a RNAi inducing compound targeted a nucleic acid coding a HAT or parts thereof;
(b) a nucleic acid construct intracellularly producing a RNAi inducing compound targeted a nucleic acid coding a HAT or parts thereof;
(c) an antisense nucleic acid targeted at the transcript product of a gene coding a HAT of the target pest; and
(d) a ribozyme targeted at the transcript product of a gene coding a HAT of the target pest.

6. The pest control method according to claim 5, wherein the RNAi inducing compound is a compound selected from the group consisting of short interfering nucleic acids (siNA), short interfering RNA (siRNA), microRNA (miRNA), short hairpin RNAs (shRNA) and precursors thereof, in particular double-stranded RNA (dsRNA), which are processed in the cell to the actual RNAi inducing compound.

7. The pest control method according to claim 6, wherein the dsRNA is coded by SEQ ID NO: 2, or homologs thereof, wherein said homologs have a sequence identity of at least 80 %, in particular of at least 85%, in particular of at least 90% to SEQ ID NO: 2 and the dsRNA reduces at least the reproduction and/or survival of said target pest.

8. The pest control method according to any one of claims 1 to 7, wherein the inhibitor is incorporated into the body of the target pest by delivering the inhibitor via ingestion, application, spraying and/or atomization of the inhibitor on the target pest.

9. The pest control method according to any one of claims 1 to 8, comprising incorporating the inhibitor into the body of the target pest by ingestion of a transgenic plant containing a gene encoding the inhibitor.

10. An isolated polynucleotide, which
(i) is defined as operably linked to a heterologous promoter; or
(ii) is defined as comprised on a plant transformation vector;
wherein the polynucleotide is selected from the group consisting of:
a) a polynucleotide comprising a nucleic acid sequence of SEQ ID NO. 2;
b) a polynucleotide that hybridizes to a nucleic acid sequence of SEQ ID NO. 2 under stringent conditions;
c) a polynucleotide of at least 70, at least 80, at least 85, at least 90 percent sequence identity, to the nucleic acid sequence of SEQ ID NO. 2;
d) a polynucleotide that is derived from SEQ ID NO. 1;
e) a fragment of at least 16 contiguous nucleotides of a nucleic acid sequence of SEQ ID NO. 1; and
f) a complement of the sequence of (a), (b), (c), (d) or (e),
wherein incorporating of a double stranded ribonucleotide sequence comprising at least one strand that is complementary to said polynucleotide or said fragment into the body of a Hemiptera pest reduces at least the reproduction and/or survival of said pest.

11. A plant transformed, transduced or transfected with a polynucleotide according to claim 10 or a seed thereof comprising the polynucleotide.

12. The plant according to claim 11, wherein said polynucleotide is expressed in a cell of the plant as a double stranded ribonucleotide sequence and ingestion of a target pest inhibitory amount of said double stranded ribonucleotide sequence and/or of a RNAi inducing compound derived from said double stranded ribonucleotide sequence in a diet inhibits the target pest from further feeding on said diet, preferably
(i) the target pest is an insect belonging to the order Hemiptera, in particular belonging to the suborder Sternorrhyncha, in particular to a pest belonging to the family of aphids, in particular *Acyrthosiphon pisum; or*
(ii) uptake of the target pest inhibitory amount of the double stranded ribonucleotide sequence or fragments thereof at least reduce survival and/or reproduction of the pest.

13. A method for controlling a Hemiptera pest infestation comprising providing in the diet of a Hemiptera pest a polypeptide according to claim 10, wherein the polynucleotide is expressed to produce a double stranded ribonucleic acid, wherein said double stranded ribonucleotide acid and/or a RNAi inducing compound derived from said double stranded ribonucleotide acid functions upon uptake by the pest to inhibit the expression of a HAT encoding target sequence within said pest and results at least in a reduced survival and/or reproduction on said diet relative to a diet lacking the plant cell.

14. The method of claim 13 wherein
a) the pest exhibits reduced growth following ingestion of the cell or its content; or
b) said Hemiptera pest is an insect belonging to the suborder Sternorrhyncha, which preferably
is selected from the group consisting of the family of aphids; or
d) said Hemiptera pest is the aphid *Acyrthosiphon pisum; or*
e) the polynucleotide functions upon uptake by the pest to suppress a gene that performs a function essential for insect survival, said function is survival, development and/or reproduction of said insect.

15. An isolated double stranded ribonucleotide (dsRNA) sequence having a survival and/or reproduction and/or development inhibitory effect on a Hemiptera pest and consisting of an nucleic acid sequence between 250 to 600 nucleic acids in length, in particular between 300 and 550 nucleic acids in length, in particular between 300 and 520 nucleic acids in length, wherein the dsRNA sequence comprises an nucleic acid sequence derived from SEQ ID NO. 1 or homologs thereof, wherein said homologs have a sequence identity of at least 80 %, in particular of at least 85%, in particular of at least 90% to SEQ ID NO: 1, and wherein the derived dsRNA is coded by an continuous part of SEQ ID NO. 1 or said homologs thereof, and having a nucleic acid sequence between 300 to 520 nucleic acids in length and wherein the Hemiptera pest belonging to the suborder Sternorrhyncha, in particular belonging to the family of aphids like *Acyrthosiphon pisum.*


SEQUENCE LISTING

<110>  Fraunhofer-Gesellschaft zur Förderung der angewandten
       Forschung e.V.

<120>  METHODS OF MULTI-SPECIES INSECT PEST CONTROL

<130>  FRA-PA35-EP

<160>  32

<170>  PatentIn version 3.5

<210>  1
<211>  6947
<212>  DNA
<213>  Acyrthosiphon pisum

<400>  1
ctgaccttgg accgtgagtt ccccggacgc cggtcacact ggtggcaata cgtcgataaa      60

gaaccaaaat tcttgataaa attgtcagcg aaaattttgt tttttatgcg tttcggtata     120

aattttttac aaaaaatagc gtaaaaccat ccgtcggacg aatttcagta attccggacg     180

tttgacgcgt ttgccggccg ttttcgcata aacggtgaca gcacagcgca actatcgata     240

gttgagcgct ccaaacaatc gttgacccga ctgccgatgg ctgcccccgt tccccgtatg     300

tctatcacgt cccgtgagtg ctttatttac atctagtttt gttgtcgaaa aatatatttc     360

cagtgtccga attcgctgtt aatccgtcgc gtttaccgca tcgaaccgtc gcagatcgtc     420

ggcgtaccgc gcaacgtgtc caggaacgct ttcagacggt ccaccgaagt gcccaggaac     480

ggcccttatc agccgcaatc atcggctcgg atcacgtcaa tttcggacac ggtcggttct     540

gcttatggct gcccatgtta cacacgaaac tcatcttatg acttgaattc accgcccagc     600

ttcaaatatt aattattgtt gaaccattta tcagtcacaa agaattttaa catgcaagaa     660

tcgagctgaa aatagagacc tacaaatgaa atcttcaata atcagcctcc gacttagcat     720

aacatggccc atattactgt atggtgatat cacatactgc ttaattatgt gacttaacta     780

tgtttaacta ttttgtttct caatttgagt ttttaagaat ttgacattgt aaaataggtt     840

taggtcattg tgcaattttt aatatgtgat tattgatact agtaactttt tactaggtct     900

tttctgtttt taaattctta aactaaatat gtcagaccta ttggtggata gccctccgaa     960

caagaggcca aagttggaag atccatttca agggtcttct gactcatctg gtgtctattc    1020

tagcaatgag atgtttgatc tcgagaatga tctccctgat gagcttatta gcactacgtc    1080

ttggaaccag caacctgata tcaagccaaa tcttggtcct caattaccat tgcaaaatgg    1140

tatgattgtt cagcaaaata atcctcaaca acttcaacaa cctaatattc aacaacgatt    1200

agtagtcagt caacatcagt tagctcacca ttttatgggt actaaacatc tcgtgtcagc    1260

gactggaatg tctatgaata aaccaattaa tccaaacatg ccatcaccaa atgttttagt    1320

tagtaaaact ggagaaccga tggttgtaaa tatgggaaat gcaggcatta attccaatca 1380

attacaaaat agtgttagtg gaataaatgt agcaaatgta aatattatga tgggaaacaa 1440

tttggttagt aattcaatgc acaatcctca tcatggcata ccccagcaac aacaaaacgg 1500

acctatgatg ttacggaaca tagccatgca tcatccacaa caaattcgca accagactcc 1560

tcatcaactt catagtttgc caattaattc tcataggtta caagctccaa tgggtgcaat 1620

gagtccagtg aataatttca atgcatacaa ccaaaataat caacaaatta atgttgtcca 1680

acaacaacca attggtattg tacgacaacc agtaccgaga tttaatgcca acactaatgg 1740

aatgttagta gatactccgg ttcctcctca gtctcaagca aatgtacgtc ttggaccaca 1800

gcaagttaat atggtcaatc aaaatgtagt tgtaccacct caaggagttc cctctgaccg 1860

tagtcaagca aatccagaaa aacgaaaaat gataacccaa cagcttgtgc tattattaca 1920

tgcacatcaa tgtcagagac gagataatca agctaatggc gaagtgagac aatgtcgttt 1980

gcaacattgt aatacaatga aaggagtctt agctcatatg accaattgct tagctggtcg 2040

aaattgtaat gtagctcact gttcgtcatc tagacaaata atatcacatt ggaaacaatg 2100

tactagacca gattgtccta tttgccaacc attaaaagca tcaaataatc gcaatgcttt 2160

agcaaataac actactcaaa atactactgg aatgaacgat atgaattgtt atagcactgt 2220

aaataatcaa gtgaaccaag tacagggagt ccctaacaat gttggtatac gaacggcttt 2280

gcaatctcaa atgccacatc ctcctaataa caatgtgctg acacctaatc aaaatacacg 2340

cgtattagtc ccacattcac aaccccaggt aacaactaca atgagtgcag atcccagtac 2400

aagttctgta aatgaaatgc tgcagtctgc ttcgaacaca actgttccag ttccatctag 2460

cattcaacaa tctgcgacaa atattcctaa ttctttcata atgacaacta gtactgatac 2520

aatgactact cagatgaaca atcaagcaca accgagtcaa tcaataccag gaaatcacat 2580

tgaaagtaag gaatggcatg agtctattac acctgaacta agaaatcatt tagtgcataa 2640

attagttcaa gcaattttcc caacatatga tcctaaagca atgcttgata aacgtatgaa 2700

taatttagtg gcttatgcta gaaaagtaga aggtgatatg tacaatgttg cgaactctag 2760

gtcagaatat tatcataggt tagcccaaac attttataaa atacaaaaag agttggaaga 2820

aaaaatgcaa aagcgaaaag aacaacaaca aatgcaacag cagcaattgg gaactcagca 2880

accgactaat aatatcaatg cacaatgctt cccaactgcg tctacacaag gattgcctac 2940

tcagcagcaa ccatcacaac cacagcagca acaaccttta catcatactt caattcaacc 3000

caatcagcaa aatttgccac aacaacaagt agtaacaaca tctggtctaa tgaaaacaca 3060

aatgtcagac agggtacatt ttccaatcac ttcacctgta cctggaccaa gtccaaatcc 3120

aaatttaccc ccaactactt ttggtttgac ccagccacct gtcccaagtc caatcaacac 3180

tagtcagttc ccatctaact cacctatgat gtcaaacata tctcaagctt cgttcgctca 3240

acctcaattt ccaaatcaat caatgaatca aatgccacaa caaatacatt tacaacaaca 3300

agttcaacag caacaacaag tgcaacagca acaacaacaa gtacaacagc aagttcaaca 3360

acaacaacac ttgaacaatc aaatgttaca accaaataga cttaatcaaa tgaatatgga 3420

aaaaatggtt aatggaccaa gtttgatagc gaataatact gtgccatcaa ctacaagtgt 3480

accatcaatg atgcccaata gtatgggtaa aggctatcct agatcagaac catcatcaat 3540

tagtcattcg agtcagatga atgctattgc ttctgcttta gcacaagatg actctcccaa 3600

ggatgttaaa ggggaagtaa aagatgagta tgatgatagt aatgatggaa aaggtgctta 3660

tggaaaaggt aaaatgaatt ctgaaacacc gtcggcatct gtgaaatctg aaattaaaca 3720

agaacgcgac gataatggtt caagttcaaa gaaaatggaa gttaaaatta aagatgaacc 3780

tatgagtcct aaaccaaaca tcactacaga tattaaacca tttgagccaa ctacatcgga 3840

cagtaaaaca gccataaaga aatgtgtatt taggcctgat gaacttagac aagccttaat 3900

gcctactctt gaaaaattat acaaacaaga acctgaatca gatcctttta aacagcctgt 3960

tgatcctcaa gcacttaata tacccgacta tttcataatc ataaaaaagc caatggattt 4020

atcaacaatt cgagaaaaat tgaatactgg tcaattttct gacccttgga gttatgttga 4080

tgatgtttgg ttaatgtttg aaaatgcttg gcattacaat aagaagtcta caaaagtata 4140

taaatcttgt acaaaacttt cagaagtttt tgaacaagaa atttatcctg ttatgagaag 4200

tcttggttat tgctgtggtc gtaagtatac attcaatccg caagtacttt gttgttttgg 4260

aaaacaattg tgtactattc caagggatgc taaatacttc agttttgaaa atagatatat 4320

ttattgtgtt aaatgcttta atgatatacc tggtgatgct gtaacattgg gcgaagaccc 4380

tacacaagcc caacaagtaa ttaaaaaaga acaatttatg gaaatgaaaa atgatcatct 4440

agaattggaa ccattcatat tatgtactca ttgtggaaga aaactccatc aaatatgtgt 4500

gctgcacaac gagaatatta atccactagg atatgtttgt gataactgtt tgaagaagcg 4560

cggtgaaaaa cgaaaagatc ataaattcaa tgctaaaaaa ttggctataa caaaattagg 4620

tacgtacatt gaaacaagag tgaacaattt ccttaagaaa aaagaagcag gcgctggtga 4680

agttgctata cgtgtagtgt ccagttcaga taaaatagtt gaagtaaaac ctgggatgcg 4740

tagtcgcttt gttgataatg gagagatgat aggcgaattt ccttacaggg caaaagcact 4800

ttatgcattt gaagaaattg atggaactga tgtttgcttc tttggtatgc atgtccaaga 4860

atatggatct gaagctccgt cacccaatac aagaagagtt tatattgcat atttagattc 4920

tgtcaacttt ttcaggccca aacagtacag aacgtatgtt tatcatgaaa tattattggg 4980

ttaccttgat tatgtaaaac aattaggata tactatggct cacatttggg catgccctcc 5040

atctgaaggt gacgattaca tatttcattg tcatccacct gaacaaaaaa taccaaaacc 5100

aaaacgatta caagaatggt ataagaaaat gctagataag ggtataattg aacgcataat 5160

tctggactat aaagatattt taaagcaagc tattgaagat aaattaagtt cagcagcaga 5220

attaccttac tttgaaggcg atttttggcc aaatgcttta gaagatagca ttaaagaact 5280

tgaccaagaa gaagaacaga agcgtaaact agccgaggct gaagaatctg ttgaaatgtg 5340

tgtaagtgat gaagttgaaa ttggccctga cggaaagaaa aaaggtatgc aaaagtccaa 5400

aaaatcaaac aaatctaagt ccagccagag aaagagcagt tcaaagaaaa ctatgttacc 5460

tcaaactagc agtgatcttt ctgctaaaat ttttgacaat atggataaac ataaagaagt 5520

gttttttgta attagattgc atagtgtaca gtcggctgca gcgttaggag caatacaaga 5580

tccagatcct ttaatcagct gtgatttaat ggacggtaga gatgcatttt tgaccatggc 5640

tcgagaaaaa cactatgaat tttcatcact taggagagca aagttttctt caatggcaat 5700

gttgtatgaa ctccataatc aaggacaaga taaatttgtt tacacatgta atagttgtaa 5760

atcacatgtg gaaaccagat accactgcac tgtttgcgat gattttgatt tatgtattac 5820

ctgttacgat aaagaaaaac atccacatac catggagaaa ttgggtcttg atttggatga 5880

cggatcatct cctggtgacc aaaaacaagc agatcctcaa gaagctagaa aattatctat 5940

ccgccggtgt atattatcac ttgtacatgc ttgccaatgt agggatgcaa attgtcgctt 6000

ggcaagttgt caacgtatga aaagggtggt acaacatgtt aaactatgta aagtcaaatc 6060

taatggcaac tgtccgattt gtaaacaata tattgcactt tgctttcatc atgctaagta 6120

ttgtactgaa gctaagtgtc cagtgttgtt ctgtccaaat atgaaacaca agatcaaaca 6180

gcaacaactt caacaaagac tgcaacaagc acagctatta agaagacgta tggctattat 6240

gaatactggt actttaggga cggaaaatac cactcctgtt gtagcaacac aacatatgtc 6300

taatcaaact aaaatgatac ctcataaagg gccagttatg gtcccaggga aacaaacacc 6360

gcctgccaat gttttacaag tagttaagca agtacaagaa gaagcggcac gacaacatga 6420

taacgtggga tatggaaaag gtggtcctca acaacagatg caacgaccac atatgcagat 6480

cgctccccct gttccacaac aagtcaatca atgggttact gaacctaatt acaatcaaat 6540

gatgatgcag cagcaacaac agcaacaaca acaacaacaa caacaacaac aacaacaaca 6600

acaacaacag cagcagcaac agcagcagca acaacagcag cagcaacaac aagttcaaca 6660

gcagcagcaa agacaaatga acattcaacc tcaacaacaa atgatactca ataacccaca 6720

aaatccaatg cttcaacagc aacaagttat gacctccaga cctcaaggtt accaacaaaa 6780

ccctaatcaa caagtaccta accaatcttt acaacagtta ctaaatgcac tgcgcaccaa 6840

cactgacagt ccacctgaac agcaacaacg cctaagagag attctgagga gcaacccaca 6900

gctgttggcc caattcatta ggaaccaacg acaaaggcaa atgttcc 6947

<210>  2
<211>  367
<212>  DNA
<213>  Acyrthosiphon pisum

<400>  2
gtcttggtta ttgctgtggt cgtaagtata cattcaatcc gcaagtactt tgttgttttg      60

gaaaacaatt gtgtactatt ccaagggatg ctaaatactt cagttttgaa aatagatata     120

tttattgtgt taaatgcttt aatgatatac ctggtgatgc tgtaacattg ggcgaagacc     180

ctacacaagc ccaacaagta attaaaaaag aacaatttat ggaaatgaaa aatgatcatc     240

tagaattgga accattcata ttatgtactc attgtggaag aaaactccat caaatatgtg     300

tgctgcacaa cgagaatatt aatccactag gatatgtttg tgataactgt ttgaagaagc     360

gcggtga                                                               367


<210>  3
<211>  1001
<212>  DNA
<213>  Acyrthosiphon pisum

<400>  3
tgttgatcct caagcactta atatacccga ctatttcata atcataaaaa agccaatgga      60

tttatcaaca attcgagaaa aattgaatac tggtcaattt tctgaccctt ggagttatgt     120

tgatgatgtt tggttaatgt ttgaaaatgc ttggcattac aataagaagt ctacaaaagt     180

atataaatca tgtacaaaac tttcagaagt ttttgaacaa gaaatttatc ctgttatgag     240

aagtcttggt tattgctgtg gtcgtaagta tacattcaat ccgcaagtac tttgttgttt     300

tggaaaacaa ttgtgtacta ttccaaggga tgctaaatac ttcagttttg aaaatagata     360

tatttattgt gttaaatgct ttaatgatat acctggtgat gctgtaacat tgggcgaaga     420

ccctacacaa gcccaacaag taattaaaaa agaacaattt atggaaatga aaaatgatca     480

tctagaattg gaaccattca tattatgtac tcattgtgga agaaaactcc atcaaatatg     540

tgtgctgcac aacgagaata ttaatccact aggatatgtt tgtgataact gtttgaagaa     600

gcgcggtgaa aaacgaaaag atcataaatt caatgctaaa aaattggcta taacaaaatt     660

aggtacgtac attgaaacaa gagtgaacaa tttccttaag aaaaaagaag caggcgctgg     720

tgaagttgct atacgtgtag tgtctagttc agataaaata gttgaagtaa aacctgggat     780

gcgtagtcgc tttgttgata atggagagat gataggcgaa tttccttaca gggcaaaagc     840

actttatgca tttgaagaaa ttgatggaac tgatgtttgc ttctttggta tgcatgtcca     900

agaatatgga tctgaagctc cgtcacccaa tacaagaaga gtttatattg catatttaga     960

ttctgtcaac tttttcaggc ccaaacagta cagaacgtat g                        1001


<210>  4

<211>  807
<212>  DNA
<213>  Acyrthosiphon pisum

<400>  4
ctgaccttgg accgtgagtt ccccggacgc cggtcacact ggtggcaata cgtcgataaa      60

gaaccaaaat tcttgataaa attgtcagcg aaaattttgt tttttatgcg tttcggtata      120

aattttttac aaaaaatagc gtaaaaccat ccgtcggacg aatttcagta attccggacg      180

tttgacgcgt ttgccggccg ttttcgcata aacggtgaca gcacagcgca actatcgata      240

gttgagcgct ccaaacaatc gttgacccga ctgccgatgg ctgcccccgt tccccgtatg      300

tctatcacgt cccgtgagtg ctttatttac atctagtttt gttgtcgaaa aatatatttc      360

cagtgtccga attcgctgtt aatccgtcgc gtttaccgca tcgaaccgtc gcagatcgtc      420

ggcgtaccgc gcaacgtgtc caggaacgct ttcagacggt ccaccgaagt gcccaggaac      480

ggcccttatc agccgcaatc atcggctcgg atcacgtcaa tttcggacac ggtcggttct      540

gcttatggct gcccatgtta cacacgaaac tcatcttatg acttgaattc accgcccagc      600

ttcaaatatt aattattgtt gaaccattta tcagtcacaa agaattttaa catgcaagaa      660

tcgagctgaa aatagagacc tacaaatgaa atcttcaata atcagcctcc gacttagcat      720

aacatggccc atattactgt atggtgatat cacatactgc ttaattatgt gacttaacta      780

tgtttaacta ttttgtttct caatttg                                          807

<210>  5
<211>  856
<212>  DNA
<213>  Acyrthosiphon pisum

<400>  5
aatcagcctc cgacttagca taacatggcc catattactg tatagtgata tcacatactg      60

cttaattatg tgacttaact atgtttaact attttgtttc tcaatttgag tttttaagaa      120

tttgacattg taaaataggt ttaggtcatt gtgcaatttt taatatgtga ttattgatac      180

tagtaacttt ttactaggtc ttttctgttt ttaaattctt aaactaaata tgtcagacct      240

attggtggat agccctccga acaagaggcc aaagttggaa gatccatttc aagggtcttc      300

tgactcatct ggtgtctatt ctagcaatga gatgtttgat ctcgagaatg atctccctga      360

tgagcttatt agcactacgt cttggaacca gcaacctgat atcaagccaa atcttggtcc      420

tcaattacca ttgcaaaatg gtatgattgt tcagcaaaat aatcctcaac aacttcaaca      480

acctaatatt caacaacgat tagtagtcag tcaacatcag ttagctcacc attttatggg      540

tactaaacat ctcgtgtcag cgactggaat gtctatgaat aaaccaatta atccaaacat      600

gccatcacca aatgttttag ttagtaaaac tggagaaccg atggttgtaa atatgggaaa      660

tgcaggcatt aattccaatc aattacaaaa tagtgttagt ggaataaatg tagcaaatgt      720

aaatattatg atgggaaaca atttggttag taattcaatg cacaatcctc atcatggcat 780

accccagcaa caacaaaacg gacctatgat gttacggaac atagccatgc atcatccaca 840

acaaattcgc aaccag 856

<210> 6
<211> 887
<212> DNA
<213> Acyrthosiphon pisum

<400> 6
agcaacaaca aaacggacct atgatgttac ggaacatagc catgcatcat ccacaacaaa 60

ttcgcaacca gactcctcat caacttcata gtttgccaat taattctcat aggttacaag 120

ctccaatggg tgcaatgagt ccagtgaata atttcaatgc atacaaccaa aataatcaac 180

aaattaatgt tgtccaacaa caaccaattg gtattgtacg acaaccagta ccgagattta 240

atgccaacac taatggaatg ttagtagata ctccggttcc tcctcagtct caagcaaatg 300

tacgtcttgg accacagcaa gttaatatgg tcaatcaaaa tgtagttgta ccacctcaag 360

gagttccctc tgaccgtagt caagcaaatc cagaaaaacg aaaaatgata acccaacagc 420

ttgtgctatt attacatgca catcaatgtc agagacgaga taatcaagct aatggcgaag 480

tgagacaatg tcgtttgcaa cattgtaata caatgaaagg agtcttagct catatgacca 540

attgcttagc tggtcgaaat tgtaatgtag ctcactgttc gtcatctaga caaataatat 600

cacattggaa acaatgtact agaccagatt gtcctatttg ccaaccatta aaagcatcaa 660

ataatcgcaa tgctttagca aataacacta ctcaaaatac tactggaatg aacgatatga 720

attgttatag cactgtaaat aatcaagtga accaagtaca gggagtccct aacaatgttg 780

gtatacgaac ggctttgcaa tctcaaatgc cacatcctcc taataacaat gtgctgacac 840

ctaatcaaaa tacacgcgta ttagtcccac attcacaacc ccaggta 887

<210> 7
<211> 801
<212> DNA
<213> Acyrthosiphon pisum

<400> 7
ctcaaatgcc acatcctcct aataacaatg tgctgacacc taatcaaaat acacgcgtat 60

tagtcccaca ttcacaaccc caggtaacaa ctacaatgag tgcagatccc agtacaagtt 120

ctgtaaatga aatgctgcag tctgcttcga acacaactgt tccagttcca tctagcattc 180

aacaatctgc gacaaatatt cctaattctt tcataatgac aactagtact gatacaatga 240

ctactcagat gaacaatcaa gcacaaccga gtcaatcaat accaggaaat cacattgaaa 300

gtaaggaatg gcatgagtct attacacctg aactaagaaa tcatttagtg cataaattag 360

ttcaagcaat tttcccaaca tatgatccta aagcaatgct tgataaacgt atgaataatt 420

tagtggctta tgctagaaaa gtagaaggtg atatgtacaa tgttgcgaac tctaggtcag 480

aatattatca taggttagcc caaacatttt ataaaataca aaaagagttg gaagaaaaaa 540

tgcaaaagcg aaaagaacaa caacaaatgc aacagcagca attgggaact cagcaaccga 600

ctaataatat caatgcacaa tgcttcccaa ctgcgtctac acaaggattg cctactcagc 660

agcaaccatc acaaccacag cagcaacaac ctttacatca tacttcaatt caacccaatc 720

agcaaaattt gccacaacaa caagtagtaa caacatctgg tctaatgaaa acacaaatgt 780

cagacagggt acattttcca a 801

<210> 8
<211> 769
<212> DNA
<213> Acyrthosiphon pisum

<400> 8
tcaattcaac ccaatcagca aaatttgcca caacaacaag tagtaacaac atctggtcta 60

atgaaaacac aaatgtcaga cagggtacat tttccaatca cttcacctgt acctggacca 120

agtccaaatc caaatttacc cccaactact tttggtttga cccagccacc tgtcccaagt 180

ccaatcaaca ctagtcagtt cccatctaac tcacctatga tgtcaaacat atctcaagct 240

tcgttcgctc aacctcaatt tccaaatcaa tcaatgaatc aaatgccaca acaaatacat 300

ttacaacaac aagttcaaca gcaacaacaa gtgcaacagc aacaacaaca agtacaacag 360

caagttcaac aacaacaaca cttgaacaat caaatgttac aaccaaatag acttaatcaa 420

atgaatatgg aaaaaatggt taatggacca agtttgatag cgaataatac tgtgccatca 480

actacaagtg taccatcaat gatgcccaat agtatgggta aaggctatcc tagatcagaa 540

ccatcatcaa ttagtcattc gagtcagatg aatgctattg cttctgcttt agcacaagat 600

gactctccca aggatgttaa aggggaagta aaagatgagt atgatgatag taatgatgga 660

aaaggtgctt atggaaaagg taaaatgaat tctgaaacac cgtcggcatc tgtgaaatct 720

gaaattaaac aagaacgcga cgataatggt tcaagttcaa agaaaatgg 769

<210> 9
<211> 1002
<212> DNA
<213> Acyrthosiphon pisum

<400> 9
aacaccgtcg gcatctgtga aatctgaaat taaacaagaa cgcgacgata atggttcaag 60

ttcaaagaaa atggaagtta aaattaaaga tgaacctatg agtcctaaac caaacatcac 120

tacagatatt aaaccatttg agccaactac atcggacagt aaaacagcca taaagaaatg 180

tgtatttagg cctgatgaac ttagacaagc cttaatgcct actcttgaaa aattatacaa 240

acaagaacct gaatcagatc cttttaaaca gcctgttgat cctcaagcac ttaatatacc 300

cgactatttc ataatcataa aaaagccaat ggatttatca acaattcgag aaaaattgaa 360

tactggtcaa ttttctgacc cttggagtta tgttgatgat gtttggttaa tgtttgaaaa 420

tgcttggcat tacaataaga agtctacaaa agtatataaa tcttgtacaa aactttcaga 480

agtttttgaa caagaaattt atcctgttat gagaagtctt ggttattgct gtggtcgtaa 540

gtatacattc aatccgcaag tactttgttg ttttggaaaa caattgtgta ctattccaag 600

ggatgctaaa tacttcagtt ttgaaaatag atatatttat tgtgttaaat gctttaatga 660

tatacctggt gatgctgtaa cattgggcga agaccctaca caagcccaac aagtaattaa 720

aaaagaacaa tttatggaaa tgaaaaatga tcatctagaa ttggaaccat tcatattatg 780

tactcattgt ggaagaaaac tccatcaaat atgtgtgctg cacaacgaga atattaatcc 840

actaggatat gtttgtgata actgtttgaa gaagcgcggt gaaaaacgaa aagatcataa 900

attcaatgct aaaaaattgg ctataacaaa attaggtacg tacattgaaa caagagtgaa 960

caatttcctt aagaaaaaga agcaggcgct ggtgaagttg ct 1002

<210> 10
<211> 839
<212> DNA
<213> Acyrthosiphon pisum

<400> 10
attggctata acaaaattag gtacgtacat tgaaacaaga gtgaacaatt tccttaagaa 60

aaaagaagca ggcgctggtg aagttgctat acgtgtagtg tccagttcag ataaaatagt 120

tgaagtaaaa cctgggatgc gtagtcgctt tgttgataat ggagagatga taggcgaatt 180

tccttacagg gcaaaagcac tttatgcatt tgaagaaatt gatggaactg atgtttgctt 240

ctttggtatg catgtccaag aatatggatc tgaagctccg tcacccaata caagaagagt 300

ttatattgca tatttagatt ctgtcaactt tttcaggccc aaacagtaca gaacgtatgt 360

ttatcatgaa atattattgg gttaccttga ttatgtaaaa caattaggat atactatggc 420

tcacatttgg gcatgccctc catctgaagg tgacgattac atatttcatt gtcatccacc 480

tgaacaaaaa ataccaaaac caaaacgatt acaagaatgg tataagaaaa tgctagataa 540

gggtataatt gaacgcataa ttctggacta taaagatatt ttaaagcaag ctattgaaga 600

taaattaagt tcagcagcag aattacctta ctttgaaggc gatttttggc caaatgcttt 660

agaagatagc attaaagaac ttgaccaaga agaagaacag aagcgtaaac tagccgaggc 720

tgaagaatct gttgaaatgt gtgtaagtga tgaagttgaa attggccctg acggaaagaa 780

aaaaggtatg caaaagtcca aaaaatcaaa caaatctaag tccagccaga gaaagagca 839

<210>  11
<211>  865
<212>  DNA
<213>  Acyrthosiphon pisum

<400>  11
gaagttgaaa ttggccctga cggaaagaaa aaaggtatgc aaaagtccaa aaaatcaaac      60

aaatctaagt ccagccagag aaagagcagt tcaaagaaaa ctatgttacc tcaaactagc     120

agtgatcttt ctgctaaaat ttttgacaat atggataaac ataaagaagt gttttttgta     180

attagattgc atagtgtaca gtcggctgca gcgttaggag caatacaaga tccagatcct     240

ttaatcagct gtgatttaat ggacggtaga gatgcatttt tgaccatggc tcgagaaaaa     300

cactatgaat tttcatcact taggagagca aagttttctt caatggcaat gttgtatgaa     360

ctccataatc aaggacaaga taaatttgtt tacacatgta atagttgtaa atcacatgtg     420

gaaaccagat accactgcac tgtttgcgat gattttgatt tatgtattac ctgttacgat     480

aaagaaaaac atccacatac catggagaaa ttgggtcttg atttggatga cggatcatct     540

cctggtgacc aaaaacaagc agatcctcaa gaagctagaa aattatctat ccgccggtgt     600

atattatcac ttgtacatgc ttgccaatgt agggatgcaa attgtcgctt ggcaagttgt     660

caacgtatga aaagggtggt acaacatgtt aaactatgta aagtcaaatc taatggcaac     720

tgtccgattt gtaaacaata tattgcactt tgctttcatc atgctaagta ttgtactgaa     780

gctaagtgtc cagtgttgtt ctgtccaaat atgaaacaca agatcaaaca gcaacaactt     840

caacaaagac tgcaacaagc acagc                                           865

<210>  12
<211>  811
<212>  DNA
<213>  Acyrthosiphon pisum

<400>  12
tgtccagtgt tgttctgtcc aaatatgaaa cacaagatca aacagcaaca acttcaacaa      60

agactgcaac aagcacagct attaagaaga cgtatggcta ttatgaatac tggtacttta     120

gggacggaaa ataccactcc tgttgtagca acacaacata tgtctaatca aactaaaatg     180

atacctcata aagggccagt tatggtccca gggaaacaaa caccgcctgc caatgtttta     240

caagtagtta agcaagtaca agaagaagcg gcacgacaac atgataacgt gggatatgga     300

aaaggtggtc ctcaacaaca gatgcaacga ccacatatgc agatcgctcc ccctgttcca     360

caacaagtca atcaatgggt tactgaacct aattacaatc aaatgatgat gcagcagcaa     420

caacagcaac aacaacaaca acaacaacaa caacaacaac aacaacaaca acagcagcag     480

caacagcagc agcaacaaca gcagcagcaa caacaagttc aacagcagca gcaaagacaa     540

atgaacattc aacctcaaca acaaatgata ctcaataacc cacaaaatcc aatgcttcaa     600

cagcaacaag ttatgacctc cagacctcaa ggttaccaac aaaaccctaa tcaacaagta     660

cctaaccaat ctttacaaca gttactaaat gcactgcgca ccaacactga cagtccacct     720

gaacagcaac aacgcctaag agagattctg aggagcaacc cacagctgtt ggcccaattc     780

attaggaacc aacgacaaag gcaaatgttc c                                    811


<210>  13
<211>  46
<212>  DNA
<213>  Artificial Sequence

<220>
<223>  RNAi_AP_p300_fwd

<400>  13
taatacgact cactataggg agagtcttgg ttattgctgt ggtcgt                    46


<210>  14
<211>  45
<212>  DNA
<213>  Artificial Sequence

<220>
<223>  RNAi_AP_p300_rev

<400>  14
taatacgact cactataggg agatcaccgc gcttcttcaa acagt                     45


<210>  15
<211>  20
<212>  DNA
<213>  Artificial Sequence

<220>
<223>  seq_AP_p300_fwd_1

<400>  15
ctgaccttgg accgtgagtt                                                 20


<210>  16
<211>  27
<212>  DNA
<213>  Artificial Sequence

<220>
<223>  seq_AP_p300_rev_1

<400>  16
caaattgaga aacaaaatag ttaaaca                                         27


<210>  17
<211>  20
<212>  DNA
<213>  Artificial Sequence

<220>

<223>  seq_AP_p300_fwd_2

<400>  17
aatcagcctc cgacttagca                                                   20


<210>  18
<211>  20
<212>  DNA
<213>  Artificial Sequence

<220>
<223>  seq_AP_p300_rev_2

<400>  18
ctggttgcga atttgttgtg                                                   20


<210>  19
<211>  20
<212>  DNA
<213>  Artificial Sequence

<220>
<223>  seq_AP_p300_fwd_3

<400>  19
agcaacaaca aaacggacct                                                   20


<210>  20
<211>  20
<212>  DNA
<213>  Artificial Sequence

<220>
<223>  seq_AP_p300_rev_3

<400>  20
tacctggggt tgtgaatgtg                                                   20


<210>  21
<211>  20
<212>  DNA
<213>  Artificial Sequence

<220>
<223>  seq_AP_p300_fwd_4

<400>  21
ctcaaatgcc acatcctcct                                                   20


<210>  22
<211>  22
<212>  DNA
<213>  Artificial Sequence

<220>
<223>  seq_AP_p300_rev_4

<400>  22

ttggaaaatg taccctgtct ga                                                22

<210>  23
<211>  20
<212>  DNA
<213>  Artificial Sequence

<220>
<223>  seq_AP_p300_fwd_5

<400>  23
tcaattcaac ccaatcagca                                                    20

<210>  24
<211>  23
<212>  DNA
<213>  Artificial Sequence

<220>
<223>  seq_AP_p300_rev_5

<400>  24
ccatttcctt tgaacttgaa cca                                                23

<210>  25
<211>  20
<212>  DNA
<213>  Artificial Sequence

<220>
<223>  seq_AP_p300_fwd_6

<400>  25
aacaccgtcg gcatctgtga                                                    20

<210>  26
<211>  20
<212>  DNA
<213>  Artificial Sequence

<220>
<223>  seq_AP_p300_rev_6

<400>  26
agcaacttca ccagcgcctg                                                    20

<210>  27
<211>  25
<212>  DNA
<213>  Artificial Sequence

<220>
<223>  seq_AP_p300_fwd_7

<400>  27
attggctata acaaaattag gtacg                                              25

<210>  28
<211>  20
<212>  DNA
<213>  Artificial Sequence

<220>
<223>  seq_AP_p300_rev_7

<400>  28
tgctctttct ctggctggac                                                    20


<210>  29
<211>  20
<212>  DNA
<213>  Artificial Sequence

<220>
<223>  seq_AP_p300_fwd_8

<400>  29
gaagttgaaa ttggccctga                                                    20


<210>  30
<211>  20
<212>  DNA
<213>  Artificial Sequence

<220>
<223>  seq_AP_p300_rev_8

<400>  30
gctgtgcttg ttgcagtctt                                                    20


<210>  31
<211>  21
<212>  DNA
<213>  Artificial Sequence

<220>
<223>  seq_AP_p300_fwd_9

<400>  31
tgtccagtgt tgttctgtcc a                                                  21


<210>  32
<211>  20
<212>  DNA
<213>  Artificial Sequence

<220>
<223>  seq_AP_p300_rev_9

<400>  32
ggaacatttg cctttgtcgt                                                    20